# EUROPÄISCHE PATENTSCHRIFT

(11) **EP 2 260 022 B1**
(45) Veröffentlichungstag und Bekanntmachung des Hinweises auf die Patenterteilung: **04.01.2017**
(21) Anmeldenummer: 09723844.8
(22) Anmeldetag: 25.03.2009
(51) Int. Cl.: C07D 209/20, C07D 413/06, C07D 417/06, C07D 403/06, A61K 31/405, A61K 31/422, A61K 31/4245, A61P 25/00

(54) **SUBSTITUIERTE CYCLOHEXYLDIAMINE**
SUBSTITUTED CYCLOHEXYLDIAMINES
CYCLOHEXYLDIAMINES SUBSTITUÉES

(30) Priorität: 27.03.2008 EP 08005759
(43) Veröffentlichungstag der Anmeldung: 15.12.2010
(73) Patentinhaber: Grünenthal GmbH, 52078 Aachen (DE)
(72) Erfinder: ZEMOLKA, Saskia, 52066 Aachen (DE); NOLTE, Bert, 53902 Bad Münstereifel (DE); LINZ, Klaus, 53359 Rheinbach (DE); SAUNDERS, Derek, John, 52072 Aachen (DE); SCHRÖDER, Wolfgang, 52074 Aachen (DE); ENGLBERGER, Werner, 52223 Stolberg (DE); THEIL, Fritz, 12247 Berlin (DE); SCHICK, Hans, 13086 Berlin (DE); KAUFMANN, Jens, D-13507 Berlin (DE); GEBAUER, Julian, 13353 Berlin (DE); SONNENSCHEIN, Helmut, 10245 Berlin (DE)
(74) Vertreter: Bülle, Jan
(86) Internationale Anmeldenummer: PCT/EP2009/002188
(87) Internationale Veröffentlichungsnummer: WO 2009/118174

(56) Entgegenhaltungen:
- WO-A-02/090317

## Beschreibung

Die vorliegende Erfindung betrifft substituierte Cyclohexyldiamine, welche eine Affinität an den µ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2002/090317, WO2002/90330, WO2003/008370, WO2003/008371, WO2003/080557, WO2004/043899, WO2004/043900, WO2004/043902, WO2004/043909, WO02004/043949, WO02004/043967, WO2005/063769, WO2005/066183, WO2005/110970, WO2005/110971, WO2005/110973, WO2005/110974, WO2005/110975, WO2005/110976, WO2005/110977, WO2006/018184, WO02006/108565, WO2007/079927, WO2007/079928, WO02007/079930, WO2007/079931, WO2007/124903, WO2008/009415 und WO2008/009416.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner weisen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte Cyclohexyldiamine hergestellt werden können, welche eine Affinität an den µ-Opioid-Rezeptor und den ORL1-Rezeptor aufweisen.

Die Erfindung betrifft Verbindungen der allgemeinen Formel (1), 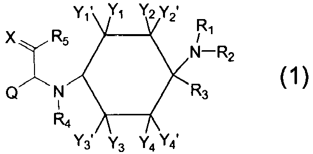 worin
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂,-NHC(=O)NHR₀, -NHC(=O)N(R₀)₂; vorzugsweise jeweils unabhängig voneinander ausgewählt sind aus der Gruppe bestehend aus -H, -F, -Cl, -CN und -C₁₋₈-Aliphat; oder Y₁ und Y₁', oder Y₂ und Y₂', oder Y₃ und Y₃', oder Y₄ und Y₄' gemeinsam für =O stehen;
Q für -R₀ steht; bevorzugt für -C₁₋₈-Aliphat-Aryl oder -C₁₋₈-Aliphat-Heteroaryl; besonders bevorzugt für -CH₂-Indolyl;
X für =O oder =N-R₆ steht;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat,-C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für-CH₂CH₂OCH₂CH₂-, -(CH₂)₃₋₆- oder -CH₂CH₂NR'CH₂CH₂- mit R' = -H, -R₀ oder -C(=O)R₀ stehen;
R₃ für -R₀ steht;
R₄ für -H, -C(=O)R₀ oder -R₀;
R₅ für -NH₂, -NHR₀, -N(R₀)₂ steht;
R₆ und R₇ jeweils unabhängig voneinander für -H, -F, -Cl, -Br, -I, -CN, -NO₂, -CHO, -R₀,-C(=O)H, -C(=O)R₀, -C(=O)OH, -C(=O)OR₀, -C(=O)NH₂, -C(=O)NHR₀, -C(=O)N(R₀)₂, -OH,-OR₀, -OC(=O)H, -OC(=O)R₀, -OC(=O)OR₀, -OC(=O)NHR₀, -OC(=O)N(R₀)₂, -SH, -SR₀,-SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀,-NHC(=O)OR₀, -NHC(=O)NH₂, -NHC(=O)NHR₀ oder -NHC(=O)N(R₀)₂ stehen;
oder R₅ und R₆ gemeinsam einen fünf- oder sechsgliedrigen Ring bilden, dessen übrige Ringatome jeweils unabhängig voneinander C, N, S.oder O sind, wobei der Ring aromatisch oder nicht aromatisch, unsubstituiert oder ein- oder mehrfach substituiert ist durch Substituenten unabhängig voneinander ausgewählt aus der Gruppe bestehend aus -F, -Cl, -Br, -I,-CN, -NO₂, -CHO, =O, -R₀, -C(=O)R₀, -C(=O)H, -C(=O)OH, -C(=O)OR₀, -C(=O)NH₂, -C(=O)-NHR₀, -C(=O)N(R₀)₂, -OH, -OR₀, -OC(=O)H, -OC(=O)R₀, -OC(=O)OR₀, -OC(=O)NHR₀,-OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀ und -NH-C(=O)N(R₀)₂;
   wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀,-C(=O)N(R₀)₂, -OH, -OR₀, -OC(=O)H, -OC(=O)R₀, -OC(=O)OR₀, -OC(=O)NHR₀,-OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -Si(R₀)₃, -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein-oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₂O-, -OR₀, -OC(=O)H, -OC(=O)R₀, -OC(=O)OR₀, -OC(=O)NHR₀,-OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂,-Si(R₀)₃, -PO(OR₀)₂ verstanden wird; wobei ggf. vorhandene N-Ringatome jeweils oxidiert sein können (N-Oxid);
in Form eines einzelnen Stereoisomers oder deren Mischung, der freien Verbindungen und/oder ihrer physiologisch verträglichen Salze und/oder Solvate.

Bei der Zusammenfassung verschiedener Reste, beispielsweise Y₁, Y₁', Y₂, Y₂', Y₃, Y₃', Y₄ und Y₄' sowie der Zusammenfassung von Resten an deren Substituenten, wie z. B. -OR₀,-OC(=O)R₀, -OC(=O)NHR₀, kann ein Substituent, z.B. R₀, für zwei oder mehrere Reste, beispielsweise -OR₀, -OC(=O)R₀, -OC(=O)NHR₀, innerhalb einer Substanz unterschiedliche Bedeutungen annehmen.

Die erfindungsgemäßen Verbindungen zeigen gute Bindung an den ORL1-Rezeptor und den µ-Opioid-Rezeptor.

In einer bevorzugten Ausführungsform weisen die erfindungsgemäßen Verbindungen ein Verhältnis ORL1/µ-Affinität von wenigstens 0,1 auf. Das ORL1/µ-Verhältnis ist definiert als 1/[K_{i(ORL1)}/K_{i(µ)}]. Besonders bevorzugt beträgt das ORL1/µ-Verhältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1/µ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/µ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen Kᵢ-Wert am µ-Opioid-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ-Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und µ-Opioid-Rezeptor, bei denen das durch 1/[K_{i(ORL1)}/K_{i(µ)}] definierte Verhältnis von ORL1 zu µ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan-oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -NH₂, -NH-C₁₋₆-Aliphat, -NH-C₃₋₈-Cycloaliphat, -NH-C₁₋₆-Aliphat-OH, -N(C₁₋₆-Aliphat)₂, -N(C₃₋₈-Cycloaliphat)₂, -N(C₁₋₆-Aliphat-OH)₂, -NO₂, -NH-C₁₋₆-Aliphat-C₃₋₈-Cycloaliphat, -NH-C₁₋₆-Aliphat-Aryl, -NH-C₁₋₆-Aliphat-Heteroaryl, -NH-Aryl, -NH-Heteroaryl, -SH, -S-C₁₋₆-Aliphat, -S-C₃₋₈-Cycloaliphat, -S-C₁₋₆-Aliphat-C₃₋₈-Cycloaliphat, -S-C₁₋₆-Aliphat-Aryl, -S-C₁₋₆-Aliphat-Heteroaryl, -S-Aryl, -S-Heteroaryl, -OH, -O-C₁₋₆-Aliphat, -O-C₃₋₈-Cycloaliphat, -O-C₁₋₆-Aliphat-OH, -O-C₁₋6-Aliphat-C₃₋₈-Cycloaliphat, -O-C₁₋₆-Aliphat-Aryl, -O-C₁₋₆-Aliphat-Heretoaryl, -O-Aryl, -O-Heteroaryl, -O-C(=O)C₁₋₆-Aliphat, -O-C(=O)C₃₋₈-Cycloaliphat, -O-C(=O)C₁₋₆-Aliphat-OH, -O-C(=O)C₁₋₆-Aliphat-C₃₋₈-Cycloaliphat, -O-C(=O)C₁₋₆-Aliphat-Aryl, -O-C(=O)C₁₋₆-Aliphat-Heretoaryl, -O-C(=O)Aryl, -O-C(=O)Heteroaryl, -C₁₋₆-Aliphat, -C₃₋₈-Cycloaliphat, -C₁₋₆-Aliphat-C₃₋₈-Cycloaliphat, -C₁₋₆-Aliphat-Aryl, -C₁₋₆-Aliphat-Heteroaryl, -Aryl, -Heteroaryl, -C(=O)C₁₋₆-Aliphat,-C(=O)C₃₋₈-Cycloaliphat, -C(=O)C₁₋₆-Aliphat-C₃₋₈-Cycloaliphat, -C(=O)C₁₋₆-Aliphat-Aryl,-C(=O)C₁₋₆-Aliphat-Heteroaryl, -C(=O)Aryl, -C(=O)Heteroaryl, -CO₂H, -CO₂-C₁₋₆-Aliphat, -CO₂-C₃₋₈-Cycloaliphat, -CO₂-C₁₋₆-Aliphat-C₃₋₈-Cycloaliphat, -CO₂-C₁₋₆-Aliphat-Aryl, -CO₂-C₁₋₆-Aliphat-Heteroaryl, -CO₂-Aryl, -CO₂-Heteroaryl; oder Y₁ und Y₁', oder Y₂ und Y₂', oder Y₃ und Y₃', oder Y₄ und Y₄' stehen gemeinsam für =O. Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl,-Br, -I, -CN, -NH₂ und -OH.

In einer bevorzugten Ausführungsform ist einer der Reste Y₁, Y₁', Y₂, Y₂', Y₃, Y₃', Y₄ und Y₄' ungleich -H und die übrigen Reste stehen für -H.

Besonders bevorzugt stehen Y₁, Y₁', Y₂, Y₂', Y₃, Y₃', Y₄ und Y₄' jeweils für -H.

Bevorzugt steht Q für -C₁₋₈-Aliphat, -Aryl, -Heteroaryl, -C₁₋₈-Aliphat-Aryl oder -C₁₋₈-Aliphat-Heteroaryl; bevorzugter für -C₁₋₈-Aliphat-Aryl oder -C₁₋₈-Aliphat-Heteroaryl. Dabei können-Aliphat, -Aryl und -Heteroaryl jeweils unsubstituiert oder ein- oder mehrfach substituiert sein, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F, -Cl, -Br, -NO₂, -CN, - Heteroaryl, -C₁₋₈-Aliphat-Aryl und -C₁₋₈-Aliphat-Heteroaryl.

In einer bevorzugten Ausführungsform ist Q ausgewählt aus der Gruppe bestehend aus ggf. jeweils über -C₁₋₈-Aliphat verbrücktes -Phenyl, -Pyrrolyl, -Furyl, -Thienyl, -Pyridyl, -Indolyl, - Benzofuryl und -Benzothienyl, wobei diese jeweils unsubstituiert oder ein- oder mehrfach substituiert sein können, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F,-Cl, -Br, -NO₂, -CN, -Heteroaryl, -C₁₋₈-Aliphat-Aryl und -C₁₋₈-Aliphat-Heteroaryl (z.B. -Ethyl-4-Pyridyl).

R₀ steht bevorzugt jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂-Cycloaliphat, -Aryl oder -Heteroaryl jeweils über eine zweibindige Brücke -C₁₋₈-Aliphat- gebunden sind. Bevorzugte Beispiele für -C₁₋₈-Aliphat-Aryl sind -CH₂-C₆H₅, -CH₂CH₂-C₆H₅, und -CH=CH-C₆H₅.

Bevorzugt stehen R₁ und R₂, unabhängig voneinander für -H; -C₁₋₆-Aliphat; -C₃₋₈-Cycloaliphat, -C₁₋₆-Aliphat-Aryl, -C₁₋₆-Aliphat-C₃₋₈-Cycloaliphat oder -C₁₋₆-Aliphat-Heteroaryl; oder die Reste R₁ und R₂ bilden zusammen einen Ring und bedeuten -CH₂CH₂OCH₂CH₂-, -CH₂CH₂NR'CH₂CH₂- oder -(CH₂)₃₋₆-. Bevorzugter stehen R₁ und R₂ unabhängig voneinander für -H; -C₁₋₅-Aliphat; oder die Reste R₁ und R₂ bilden zusammen einen Ring und bedeuten -CH₂CH₂OCH₂CH₂-, -CH₂CH₂NR'-CH₂CH₂- oder -(CH₂)₃₋₆-, wobei R' bevorzugt -H oder -C₁₋₅-Aliphat bedeutet. Besonders bevorzugt sind Verbindungen, worin R₁ und R₂ unabhängig voneinander für -CH₃ oder -H stehen, wobei R₁ und R₂ nicht gleichzeitig -H bedeuten; oder R₁ und R₂ bilden einen Ring und bedeuten -(CH₂)₃₋₄-. Ganz besonders bevorzugt sind Verbindungen, worin R₁ und R₂ für -CH₃ oder worin R₁ für -H und R₂ für -CH₃ stehen.

Besonders bevorzugt bilden R₁ und R₂ zusammen mit dem Stickstoffatom, an welches sie gebunden sind, eine der folgenden funktionellen Gruppen:

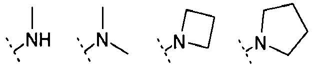

Bevorzugt steht R₃ für -C₁₋₈-Aliphat, -C₃₋₈-Cycloaliphat, -Aryl, -Heteroaryl; oder für jeweils über eine -C₁₋₃-Aliphat-Gruppe gebundenes -Aryl, -Heteroaryl oder -C₃₋₈-Cycloaliphat.

Besonders bevorzugt steht R₃ für -Ethyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl, -Benzothiophenyl, -Furyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -C₁₋₃-Aliphat-Gruppe gebundenes -C₅₋₆-Cycloaliphat, -Phenyl, -Naphthyl, -Anthracenyl, -Thiophenyl, -Benzothiophenyl, -Pyridyl, -Furyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert oder ein- oder mehrfach substituiert.

Bevorzugter steht R₃ für -Propyl, -Butyl, -Pentyl, -Hexyl, -Phenyl, -Furyl, -Thiophenyl, -Naphthyl, -Benzyl, -Benzofuranyl, -Indolyl, -Indanyl, -Benzodioxanyl, -Benzodioxolanyl, -Pyridyl, -Pyrimidyl, -Pyrazinyl, -Triazolyl oder -Benzothiophenyl, jeweils unsubstituiert oder ein- oder mehrfach substituiert; über eine gesättigte, unverzweigte -C₁₋₃-Aliphat-Gruppe gebundenes -Phenyl, -Furyl oder -Thiophenyl, jeweils unsubstituiert oder ein- oder mehrfach substituiert.

Noch bevorzugter steht R₃ für -Propyl, -Butyl, -Pentyl, -Hexyl, -Phenyl, -Phenethyl, -Thiophenyl, -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder -Phenethyl.

Ganz besonders bevorzugt steht R₃ für -Butyl, -Ethyl, -3-Methoxypropyl, -Benzothiophenyl, -Phenyl, -3-Methylphenyl, -3-Fluorphenyl, -Benzo[1,3]-dioxolyl, -Benzyl, -1-Methyl-1,2,4-triazolyl, -Thienyl oder -Phenethyl.

Am bevorzugtesten steht R₃ für -Phenyl, -Benzyl oder -Phenethyl, jeweils unsubstituiert oder am Ring ein- oder mehrfach substituiert; -C₁₋₅-Aliphat, -C₄₋₆-Cycloaliphat, -Pyridyl, -Thienyl, -Thiazolyl, -Imidazolyl, -1,2,4 Triazolyl oder -Benzimidazolyl, unsubstituiert oder ein- oder mehrfach substituiert.

Insbesondere bevorzugt steht R₃ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -OCH₃ oder -OC₂H₅, wobei vorzugsweise -Thienyl, -Pyridyl, -Thiazolyl, -Imidazolyl, -1,2,4 Triazolyl und -Benzimidazolyl unsubstituiert sind.

Insbesondere bevorzugt steht R₃ für -Phenyl, unsubstituiert oder einfach substituiert mit -F, -Cl, -CN, -CH₃; -Thienyl; -Ethyl, -n-Propyl oder -n-Butyl, unsubstituiert oder ein- oder mehrfach substituiert mit -OCH₃, -OH oder -OC₂H₅, insbesondere mit -OCH₃.

Bevorzugt steht R₄ für -H, -C₁₋₅-Aliphat, -C₃₋₈-Cycloaliphat, -Aryl, -Heteroaryl, -C₁₋₆-Aliphat-Aryl, -C₁₋₆-Aliphat-C₃₋₈-Cycloaliphat, -C₁₋₆-Aliphat-Heteroaryl, -C(=O)Aryl, -C(=O)Heteroaryl, -C(=O)C₁₋₆-Aliphat, -C(=O)C₁₋₆-Aliphat-Aryl, -C(=O)C₁₋₆-Aliphat-Heteroaryl, -C(=O)C₃₋₈-Cycloaliphat-Aryl, -C(=O)C₃₋₈-Cycloaliphat-Heteroaryl, bevorzugter für -H oder -C₁₋₅-Aliphat, insbesondere für -H oder -CH₃.

X steht für =O oder =N-R₆. Steht X für =O, so weist die erfindungsgemäße Verbindung der allgemeinen Formel (1) die allgemeine Formel (1-a) auf. Steht X für =NR₆, so weist die erfindungsgemäße Verbindung der allgemeinen Formel (1) die allgemeine Formel (1-b) auf. Steht X nicht erfindungsgemäß für =CR₆R₇, so weist die erfindungsgemäße Verbindung der allgemeinen Formel (1) die allgemeine Formel (1-c) auf:

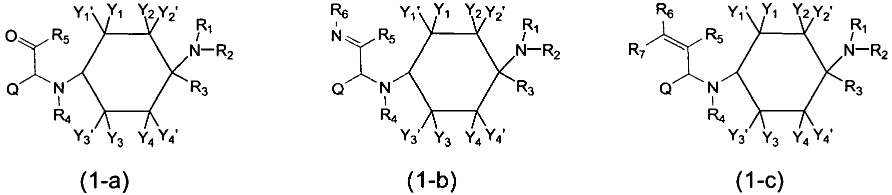

Bevorzugte Ausführungsformen der Verbindungen der allgemeinen Formel (1-b) und (1-c) sind nachfolgend abgebildet (E-2 und E-4 nicht erfindungsgemäß): 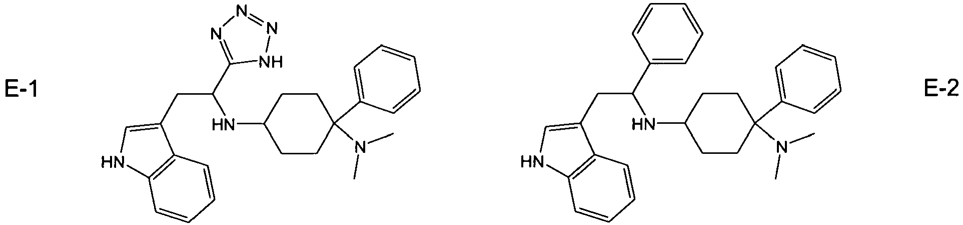 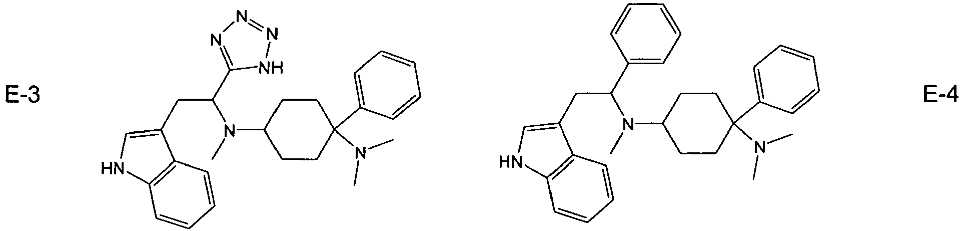
R₅ steht bevorzugt für -NH_{2,} -NH-C₁₋₈-Aliphat, -NH-C₃₋₁₂-Cycloaliphat, -NH-Aryl, -NH-Heteroaryl, -NH-C₁₋₈-Aliphat-C₃₋₁₂-Cycloaliphat, -NH-C₁₋₈-Aliphat-Aryl, -NH-C₁₋₈-Aliphat-Heteroaryl, -N(C₁₋₈-Aliphat)₂, -N(C₃₋₁₂-Cycloaliphat)₂, -N(Aryl)₂, -N(Heteroaryl)₂, -N(C₁₋₈-Aliphat-C₃₋₁₂-Cycloaliphat)₂, -N(C₁₋₈-Aliphat-Aryl)₂, -N(C₁₋₈-Aliphat-Heteroaryl)₂,
R₆ und R₇ stehen bevorzugt jeweils unabhängig voneinander für -H, -F, -Cl, -Br, -I, -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C(=O)H, -C(=O)-C₁₋₈-Aliphat, -C(=O)-C₃₋₁₂-Cycloaliphat, -C(=O)-Aryl, -C(=O)-Heteroaryl, -C(=O)-C₁₋₈-Aliphat-C₃₋₁₂-Cycloaliphat, -C(=O)-C₁₋₈-Aliphat-Aryl, -C(=O)-C₁₋₈-Aliphat-Heteroaryl, -C(=O)O-C₁₋₈-Aliphat, -C(=O)O-C₃₋₁₂-Cycloaliphat, -C(=O)O-Aryl, -C(=O)O-Heteroaryl, -C(=O)O-C₁₋₈-Aliphat-C₃₋₁₂-Cycloaliphat, -C(=O)O-C₁₋₈-Aliphat-Aryl, -C(=O)O-C₁₋₈-Aliphat-Heteroarl, -CN, -C(=O)NH₂, -C(=O)-NH-C₁₋₈-Aliphat, -C(=O)NH-C₃₋₁₂-Cycloaliphat, -C(=O)NH-Aryl, -C(=O)NH-Heteroaryl, -C(=O)-NH-C₁₋₈-Aliphat-C₃₋₁₂-Cycloaliphat, -C(=O)NH-C₁₋₈-Aliphat-Aryl, -C(=O)NH-C₁₋₈-Aliphat-Heteroaryl, -C(=O)-N(C₁₋₈-Aliphat)₂, -C(=O)N(C₃₋₁₂-Cycloaliphat)₂, -C(=O)N(Aryl)₂, -C(=O)N-(Heteroaryl)₂, -C(=O)N(C₁₋₈-Aliphat-C₃₋₁₂-Cycloaliphat)₂, -C(=O)N(C₁₋₈-Aliphat-Aryl)₂, -C(=O)-N(C₁₋₈-Aliphat-Heteroaryl)₂, -OH, -OC₁₋₈-Aliphat, -OC₃₋₁₂-Cycloaliphat, -OAryl, -OHeteroaryl, -OC₁₋₈-Aliphat-C₃₋₁₂-Cycloaliphat, -OC₁₋₈-Aliphat-Aryl, -OC₁₋₈-Aliphat-Heteroaryl, -OC(=O)H, -OC(=O)-C₁₋₈-Aliphat, -OC(=O)-C₃₋₁₂-Cycloaliphat, -OC(=O)-Aryl, -OC(=O)-Heteroaryl, -OC(=O)-C₁₋₈-Aliphat-C₃₋₁₂-Cycloaliphat, -OC(=O)-C₁₋₈-Aliphat-Aryl, -OC(=O)-C₁₋₈-Aliphat-Heteroaryl, -OC(=O)O-C₁₋₈-Aliphat, -OC(=O)O-C₃₋₁₂-Cycloaliphat, -OC(=O)O-Aryl, -OC(=O)-O-Heteroaryl, -OC(=O)O-C₁₋₈-Aliphat-C₃₋₁₂-Cycloaliphat, -OC(=O)O-C₁₋₈-Aliphat-Aryl, -OC(=O)-O-C₁₋₈-Aliphat-Heteroaryl, -OC(=O)NH-C₁₋₈-Aliphat, -OC(=O)NH-C₃₋₁₂-Cycloaliphat,-OC(=O)NH-Aryl, -OC(=O)NH-Heteroaryl, -OC(=O)NH-C₁₋₈-Aliphat-C₃₋₁₂-Cycloaliphat,-OC(=O)NH-C₁₋₈-Aliphat-Aryl, -OC(=O)NH-C₁₋₈-Aliphat-Heteroaryl, -OC(=O)N(C₁₋₈-Aliphat)₂,-OC(=O)N(C₃₋₁₂-Cycloaliphat)₂, -OC(=O)N(Aryl)₂, -OC(=O)-N(Heteroaryl)₂, -OC(=O)N(C₁₋₈-Aliphat-C₃₋₁₂-Cycloaliphat)₂, -OC(=O)N(C₁₋₈-Aliphat-Aryl)₂, -OC(=O)N(C₁₋₈-Aliphat-Hetero-aryl)₂, -NH₂, -NO₂, -NH-C₁₋₈-Aliphat, -NH-C₃₋₁₂-Cycloaliphat, -NH-Aryl, -NH-Heteroaryl, -NH-C₁₋₈-Aliphat-C₃₋₁₂-Cycloaliphat, -NH-C₁₋₈-Aliphat-Aryl, -NH-C₁₋₈-Aliphat-Heteroaryl, -N(C₁₋₈-Aliphat)₂, -N(C₃₋₁₂-Cycloaliphat)₂, -N(Aryl)₂, -N(Heteroaryl)₂, -N(C₁₋₈-Aliphat-C₃₋₁₂-Cycloaliphat)₂, -N(C₁₋₈-Aliphat-Aryl)₂, -N(C₁₋₈-Aliphat-Heteroaryl)₂, -NHC(=O)-C₁₋₈-Aliphat,-NHC(=O)-C₃₋₁₂-Cycloaliphat, -NHC(=O)-Aryl, -NHC(=O)-Heteroaryl, -NHC(=O)-C₁₋₈-Aliphat-C₃₋₁₂-Cycloaliphat, -NHC(=O)-C₁₋₈-Aliphat-Aryl, -NHC(=O)-C₁₋₈-Aliphat-Heteroaryl,-NHC(=O)O-C₁₋₈-Aliphat, -NHC(=O)O-C₃₋₁₂-Cycloaliphat, -NHC(=O)O-Aryl, -NHC(=O)O-Heteroaryl, -NHC(=O)O-C₁₋₈-Aliphat-C₃₋₁₂-Cycloaliphat, -NHC(=O)O-C₁₋₈-Aliphat-Aryl,-NHC(=O)O-C₁₋₈-Aliphat-Heteroaryl, -NHC(=O)NH-C₁₋₈-Aliphat, -NHC(=O)NH-C₃₋₂-Cycloaliphat, -NHC(=O)NH-Aryl, -NHC(=O)-NH-Heteroaryl, -NHC(=O)NH-C₁₋₈-Aliphat-C₃₋₁₂-Cycloaliphat, -NHC(=O)NH-C₁₋₈-Aliphat-Aryl, -NHC(=O)NH-C₁₋₈-Aliphat-Heteroaryl, -NH-C(=O)N(C₁₋₈-Aliphat)₂, -NHC(=O)N(C₃₋₁₂-Cycloaliphat)₂, -NHC(=O)N(Aryl)₂, -NHC(=O)-N(Heteroaryl)₂, -NHC(=O)N(C₁₋₈-Aliphat-C₃₋₁₂-Cycloaliphat)₂, -NHC(=O)N(C₁₋₈-Aliphat-Aryl)₂,-NHC(=O)N(C₁₋₈-Aliphat-Heteroaryl)₂, -SH, -SC₁₋₈-Aliphat, -SC₃₋₁₂-Cycloaliphat, -SAryl,-SHeteroaryl, -SC₁₋₈-Aliphat-C₃₋₁₂-Cycloaliphat, -SC₁₋₈-Aliphat-Aryl, -SC₁₋₈-Aliphat-Heteroaryl,-S(=O)₁₋₂C₁₋₈-Aliphat, -S(=O)₁₋₂C₃₋₁₂-Cycloaliphat, -S(=O)₁₋₂-Aryl, -S(=O)₁₋₂Heteroaryl, -S(=O)₁₋₂C₁₋₈-Aliphat-C₃₋₁₂-Cycloaliphat, -S(=O)₁₋₂C₁₋₈-Aliphat-Aryl, -S(=O)₁₋₂C₁₋₈-Aliphat-Heteroaryl,-S(=O)₁₋₂OH, -S(=O)₁₋₂OC₁₋₈-Aliphat, -S(=O)₁₋₂OC₃₋₁₂-Cycloaliphat, -S(=O)₁₋₂OAryl, -S(=O)₁-₂OHeteroaryl, -S(=O)₁₋₂OC₁₋₈-Aliphat-C₃₋₁₂-Cycloaliphat, -S(=O)₁₋₂OC₁₋₈-Aliphat-Aryl, -S(=O)₁₋₂OC₁₋₈-Aliphat-Heteroaryl, -S(=O)₁₋₂NH₂, -S(=O)₁₋₂NHC₁₋₈-Aliphat, -S(=O)₁₋₂NHC₃₋₂-Cycloaliphat, -S(=O)₁₋₂NHAryl, -S(=O)₁₋₂NH-Heteroaryl, -S(=O)₁₋₂NHC₁₋₈-Aliphat-C₃₋₁₂-Cycloaliphat, -S(=O)₁₋₂NHC₁₋₈-Aliphat-Aryl, -S(=O)₁₋₂NHC₁₋₈-Aliphat-Heteroaryl, -S(=O)₁₋₂N(C₁₋₈-Aliphat)₂, -S(=O)₁₋₂N(C₃₋₁₂-Cycloaliphat)₂, -S(=O)₁₋₂N(Aryl)₂, -S(=O)₁₋₂N(Heteroaryl)₂,-S(=O)₁₋₂N(C₁₋₈-Aliphat-C₃₋₁₂-Cycloaliphat)₂, -S(=O)₁₋₂N(C₁₋₈-Aliphat-Aryl)₂ oder -S(=O)₁₋₂N(C₁₋₈-Aliphat-Heteroaryl)₂;
oder R₅ und R₆ bilden gemeinsam einen fünf- oder sechsgliedrigen Ring, dessen übrige Ringatome (d.h. die 3 bzw. 4 Ringatome neben X und dem C-Atom, an welches X gebunden ist) jeweils unabhängig voneinander C, N, S oder O sind, wobei der Ring aromatisch oder nicht aromatisch, unsubstituiert oder ein- oder mehrfach substituiert ist. In dieser Ausführungsform stellt R₅ demnach ein ggf. substituiertes Ringatom dar, ausgewählt aus der Gruppe bestehend aus C, N, S oder O. Bildet R₅ ein Ringatom -O- oder -S-, so ist dieses nicht weiter substituiert. Bildet R₅ ein Ringatom -C-, so ist dieses zweifach substituiert, wobei die Substituenten u.a. -H sein können (-CH₂-). Bildet R₅ ein Ringatom =C-, so ist dieses einfach substituiert, wobei der Substituent u.a. -H sein kann (=CH-). Bildet R₅ ein Ringatom - N-, so ist dieses einfach substituiert, wobei der Substituent u.a. -H sein kann (-NH-). Bildet R₅ ein Ringatom =N-, so ist dieses nicht weiter substituiert. Ist der von R₅ und R₆ gem,einsam gebildete Ring substituiert mit einem oder mehreren Substituenten, welche ungleich -H sind, so sind die Substituenten bevorzugt unabhängig voneinander ausgewählt aus der Gruppe bestehend aus -F, -Cl, -Br, -I, -CN, -NO₂, -CHO, =O, -R₀, -OH, -OR₀, -NH₂, -NHR₀ und-N(R₀)₂; besonders bevorzugt =O und -CH₃.

Steht X für =O, so steht R₅ bevorzugt für -NH₂, -NH-C₁₋₈-Aliphat, -NH-C₃₋₁₂-Cycloaliphat, -NH-Aryl, -NH-Heteroaryl, -NH-C₁₋₈-Aliphat-C₃₋₁₂-Cycloaliphat, -NH-C₁₋₈-Aliphat-Aryl, -NH-C₁₋₈-Aliphat-Heteroaryl, -N(C₁₋₈-Aliphat)₂, -N(C₃₋₁₂-Cycloaliphat)₂, -N(Aryl)₂, -N(Heteroaryl)₂, -N(C₁₋₈-Aliphat-C₃₋₁₂-Cycloaliphat)₂, -N(C₁₋₈-Aliphat-Aryl)₂, oder-N(C₁₋₈-Aliphat-Heteroaryl)₂; besonders bevorzugt für -NH₂, -NHC₁₋₈-Aliphat oder -N(C₁₋₈-Aliphat)₂.

Steht X für =NR₆, so bildet R₅ bevorzugt zusammen mit R₆ einen fünf- oder sechsgliedrigen Ring, dessen übrige Ringatome (d.h. die 3 bzw. 4 Ringatome neben X und dem C-Atom, an welches X gebunden ist) jeweils unabhängig voneinander C, N, S oder O sind, wobei der Ring aromatisch oder nicht aromatisch, unsubstituiert oder ein- oder mehrfach substituiert ist. Bevorzugt steht die funktionelle Gruppe -CR₅(=X) für einen der folgenden Reste, welche unsubstituiert oder ein- oder mehrfach substituiert sein können:

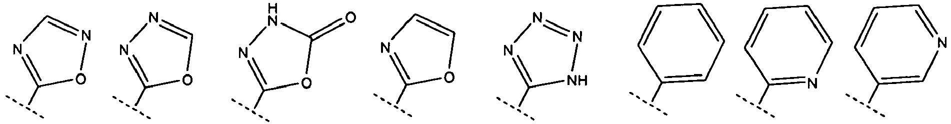

R₇ steht bevorzugt für -H, -F, -Cl, -Br, -CH₃, -OH, -OCH₃, -CN oder -NO₂.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen-.

Bevorzugt ist Aliphat jeweils ein verzweigter oder unverzweigter, gesättigter oder ein ein-oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -CO₂, -CHO, =O, -R₀, -C(=O)R₀, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂,-NHC(=O)NHR₀, -NH-C(=O)N(R₀)₂, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C=C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂-CH₂CH₃, -CH(CH₃)CH₂CH₃, -CH₂CH(CH₃)₂, -C(CH₃)₃, -CH₂CH₂CH₂-CH₂CH₃ und -CH₂CH₂-CH₂CH₂CH₂CH₃; aber auch -CH=CH₂, -C=CH, -CH₂CH=CH₂, -CH=CHCH₃, -CH₂C≡CH,-C≡CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-,-CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡CCH₂-. Bevorzugte substituierte einbindige Aliphaten umfassen -CH₂F, -CHF₂, -CF₃, -CH₂CF₃, -CF₂CF₃,-CH₂OH, -CH₂CH₂OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂- und -CH₂CHOHCH₂-. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH,-OR₀, -OC(=O)H, -OC(=O)R₀, -OC(=O)OR₀, -OC(=O)NHR₀, -OC(=O)-N(R₀)₂, -SH, -SR₀,-SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀,-NHC(=O)OR₀, -NHC(=O)NH₂, -NHC(=O)NHR₀, -NH-C(=O)N(R₀)₂, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I,-OH, -OC₁₋₆-Alkyl, -OC(=O)C₁₋₆Alkyl, -SH, -NH₂, -NHC₁₋₆-Alkyl, -N(C₁₋₆-Alkyl)₂, -C(=O)OC₁₋₆-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN,-CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂-OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder - OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen konden-siert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀,-C(=O)-NH₂, -C(=O)NHR₀, -C(=O)N(R₀)₂, -OH, -O(CH₂)₁₋₂O-, -OR₀, -OC(=O)H, -OC(=O)R₀,-OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂,-NHC(=O)NHR₀, -NH-C(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀,-OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀,-N(R₀)₂, -N⁺(R₀)₃, -N+(R₀)₂O⁻, -NH-C(=O)R₀, -NHC(=O)OR₀, -NHC(=O)NH₂, -NHC(=O)NHR₀,-NH-C(=O)N(R₀)₂, -Si(R₀)₃, -PO(OR₀)₂.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, -CO₂H, -NH₂, -NO₂, -NHR₀, -N(R₀)₂,-N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀,-C(=O)N(R₀)₂, -S(=O)₁₋₂R₀, -S(=O)₂NH₂, -SO₃H, =O oder -R₀. Bevorzugte Substituenten sind-F, -Cl, -Br, -I, -OH, -OC₁₋₆-Alkyl, -O-C(=O)-C₁₋₆-Alkyl, -SH, -NH₂, -NHC₁-₆-Alkyl, -N(C₁₋₆-Alkyl)₂, -C(=O)OC₁₋₆-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, -CH₃,-C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen kann es sich je nach Substitution in Bezug auf den Cyclohexanring um Isomere handeln, bei denen das Substitutionsmuster in 1,4-Position (1-Position: >C(NR₁R)R₃; 4-Position: >CHNCHQC(=X)R₅) auch mit syn/anti bezeichnet werden kann. "Syn/anti-Isomere" sind eine Untergruppe der Stereoisomere (Konfigurationsisomere).

In einer bevorzugten Ausführungsform beträgt der Diastereomerenüberschuss des syn-Isomers wenigstens 50%de, bevorzugter wenigstens 75%de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des anti-Isomers wenigstens 50%de, bevorzugter wenigstens 75%de, noch bevorzugter wenigstens 90%de, am bevorzugtesten wenigstens 95%de und insbesondere wenigstens 99%de. Beide Diastereomere unterscheiden sich in ihrer Polarität, so dass nachfolgend das unpolare Diastereomer vom polaren Diastereomer unterschieden wird. Beide Diastereomere liegen (im Falle zweier Stereozentren) als Enantiomerenpaare vor (RR+SS bzw. RS+SR).

Geeignete Methoden zur Trennung der Isomere (Diastereomere) sind dem Fachmann bekannt. Als Beispiele können Säulenchromatographie, präparative HPLC und Kristallisationsverfahren genannt werden. Die Polarität ist beispielsweise für die Reihenfolge verantwortlich, in der beide Diastereomere bei der Dünnschichtchromatographie eluiert werden (keine *reversed-phase* Bedingungen).

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Nachfolgend werden jeweils bevorzugte Ausführungsformen der erfindungsgemäßen Verbindungen erläutert. Sofern nicht ausdrücklich spezifiziert gelten alle jeweils zuvor erläuterten Definitionen der Substituenten (d.h. z.B. von R₀ bis R₇, Y₁ bis Y₄', Q, X etc.) und deren jeweilige bevorzugte Ausführungsformen entsprechend und werden daher nicht wiederholt.

Bevorzugte Ausführungsformen der erfindungsgemäßen Verbindungen der allgemeinen Formel (1) haben die allgemeine Formel (1.1) oder (1.2): 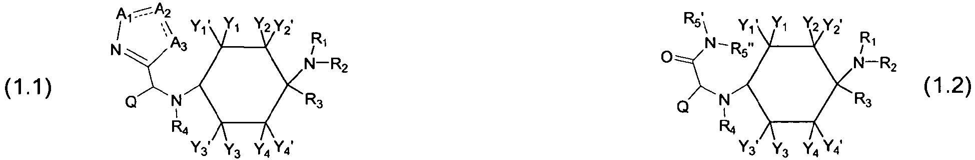 worin, soweit vorhanden,
A₁ für -N=, -NH-, -NR₈- oder -CR₈= steht;
A₂ für =N-, -C(=O)- oder =CR₉- steht;
A₃ für -O-, -NH- oder -NR₁₀- steht; und
R₅', R₅", R₈, R₉ und R₁₀ jeweils unabhängig voneinander für -H, =O oder -C₁₋₈-Aliphat stehen.

Bevorzugt stehen R₅' und R₅" jeweils unabhängig voneinander für -H oder -C₁₋₈-Aliphat.

Zum Zwecke der Beschreibung steht "-̅-̅-̅-̅-̅" für eine Doppelbindung oder für eine Einfachbindung. Ein Fachmann erkennt, dass üblicherweise die Bindung zwischen A₁ und A₂ nicht eine Doppelbindung sein kann, wenn bereits die Bindung zwischen A₂ und A₃ eine Doppelbindung ist, und umgekehrt. Ein Fachmann erkennt weiterhin, dass ggf. eine bestimmte Anzahl von Wasserstoffatomen als Substituenten vorhanden ist.

Weitere bevorzugte Ausführungsformen der erfindungsgemäßen Verbindungen der allgemeinen Formel (1) haben die allgemeine Formel (2), (3), (4), (5) oder (6): 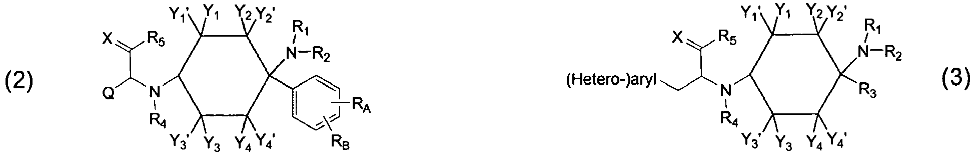 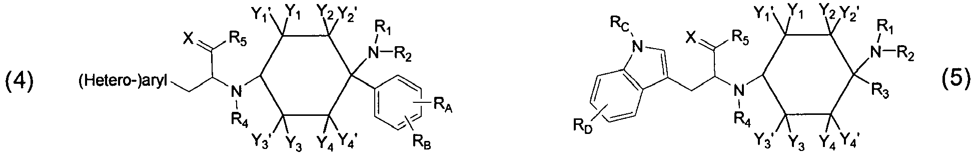 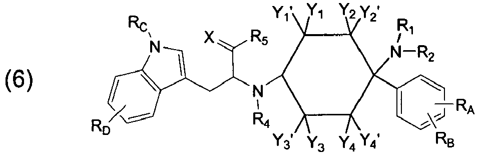 worin, soweit vorhanden,
R_{A}, R_{B}, R_{C} und R_{D} jeweils unabhängig voneinander ausgewählt sind aus der Gruppe bestehend aus -H, -C₁₋₈-Aliphat, -OH, -OC₁₋₈-Aliphat, -CF₃, -F, -Cl, -Br, -NO₂, -CN,-Heteroaryl, -C₁₋₈-Aliphat-Aryl und -C₁₋₈-Aliphat-Heteroaryl; und
(Hetero-)aryl für Heteroaryl oder Aryl steht.

Dabei können -Aryl und -Heteroaryl jeweils unsubstituiert oder ein- oder mehrfach substituiert sein, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F, -Cl, -Br,-NO₂, -CN, -Heteroaryl, -C₁₋₈-Aliphat-Aryl und -C₁₋₈-Aliphat-Heteroaryl (z.B. -Ethyl-4-Pyridyl).

In einer bevorzugten Ausführungsform ist (Hetero-)aryl ausgewählt aus der Gruppe bestehend aus Phenyl, Benzyl, Pyrrolyl, Furyl, Thienyl, Pyridyl, Indolyl, Benzofuryl und Benzothienyl, wobei diese jeweils unsubstituiert oder ein- oder mehrfach substituiert sein können, vorzugsweise mit Substituenten, welche unabhängig voneinander ausgewählt sind aus der Gruppe bestehend aus -C₁₋₈-Aliphat, -OH, -OC₁₋₈-Aliphat, -CF₃, -F, -Cl, -Br, -NO₂,-CN, -Aryl, -Heteroaryl, -C₁₋₈-Aliphat-Aryl und -C₁₋₈-Aliphat-Heteroaryl (z.B. -Ethyl-4-Pyridyl). Bevorzugte Ausführungsformen der Verbindungen der allgemeinen Formel (2) haben die allgemeine Formel (2.1), (2.2), (2.3) oder (2.4) ((2.4. nicht erfindungsgemäß): 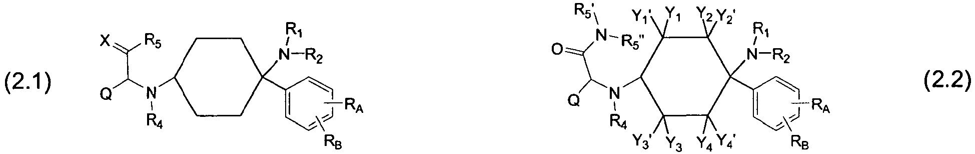 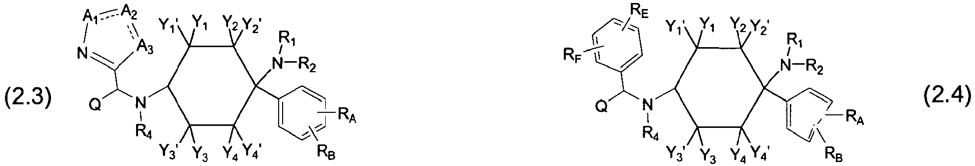 worin, soweit vorhanden,
R_{E} und R_{F} jeweils unabhängig voneinander ausgewählt sind aus der Gruppe bestehend aus-H, -C₁₋₈-Aliphat, -OH, -OC₁₋₈-Aliphat, -CF₃, -F, -Cl, -Br, -NO₂, -CN, -Heteroaryl, -C₁₋₈-Aliphat-Aryl und -C₁₋₈-Aliphat-Heteroaryl.

Bevorzugte Ausführungsformen der Verbindungen der allgemeinen Formel (3) haben die allgemeine Formel (3.1), (3.2), (3.3) oder (3.4) ((3.4) nicht erfindungsgemäß):

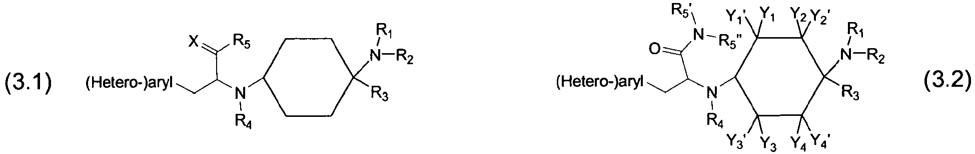

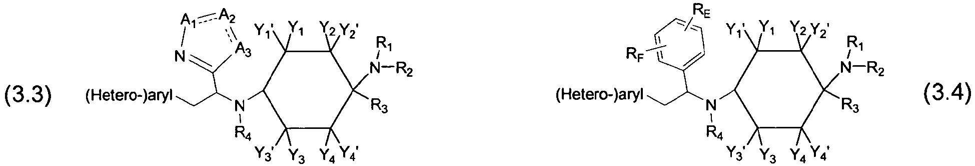

Bevorzugte Ausführungsformen der Verbindungen der allgemeinen Formel (4) haben die allgemeine Formel (4.1), (4.2), (4.3) oder (4.4) ((4.4) nicht erfindungsgemäß):

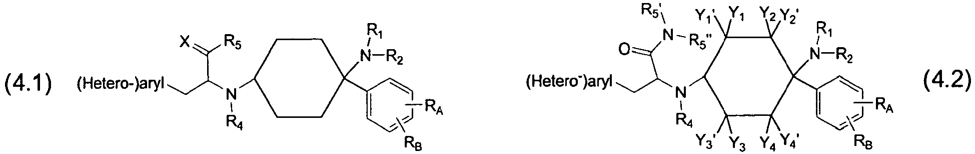

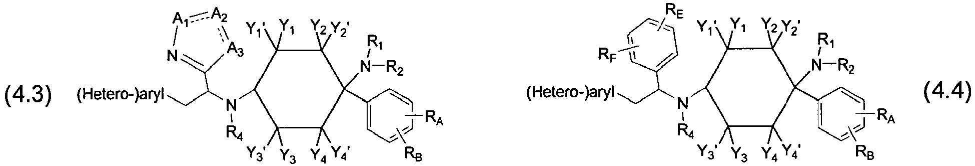

Bevorzugte Ausführungsformen der Verbindungen der allgemeinen Formel (5) haben die allgemeine Formel (5.1), (5.2), (5.3) oder (5.4) ((5.4) nicht erfindungsgemäß):

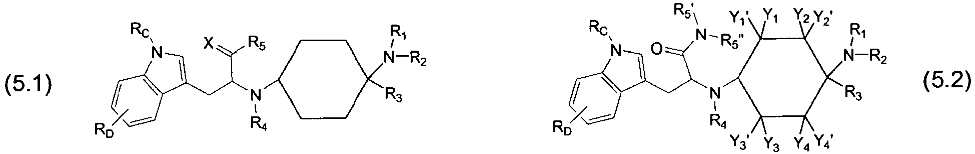

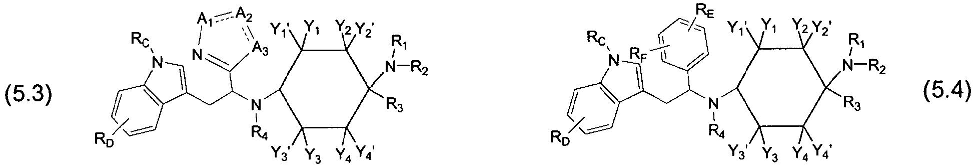

Bevorzugte Ausführungsformen der Verbindungen der allgemeinen Formel (6) haben die allgemeine Formel (6.1), (6.2), (6.3) oder (6.4) ((6.4) nicht erfindungsgemäß):

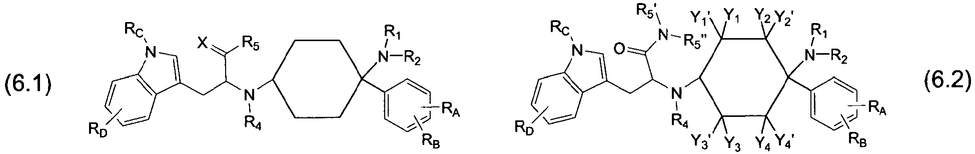

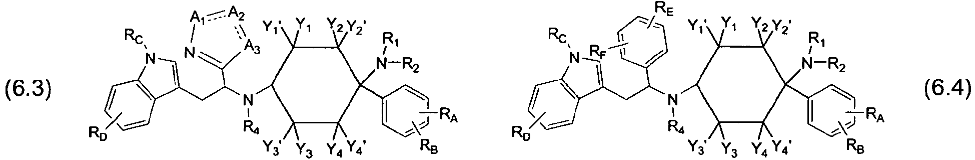

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁₋₈-Aliphat (Substituent der 1. Generation), so kann -C₁₋₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis R₇ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis R₇ jeweils nicht substituiert sein.

Ganz besonders bevorzugt sind Verbindungen aus der Gruppe:
- (S)-2-(4-(Dimethylamino)-4-phenylcyclohexylamino)-3-(1H-indol-3-yl)propanamid;
- (±)-2-(4-(Dimethylamino)-4-phenylcyclohexylamino)-3-(1H-indol-3-yl)propanamid;
- (S)-2-(4-(Dimethylamino)-4-phenylcyclohexylamino)-3-(1H-indol-3-yl)-N,N-dimethylpropanamid;
- (S)-2-(4-(Dimethylamino)-4-phenylcyclohexylamino)-3-(1H-indol-3-yl)-N-methylpropanamid;
- 5-((S)-1-(4-(Dimethylamino)-4-phenylcyclohexylamino)-2-(1H-indol-3-yl)ethyl)-1,3,4-oxadiazol-2(3H)-on;
- N4-((S)-2-(1H-indol-3-yl)-1-(3-methyl-1,2,4-oxadiazol-5-yl)ethyl)-N1,N1-dimethyl-1-phenylcyclohexan-1,4-diamin;
- 5-((R)-1-(4-(Dimethylamino)-4-phenylcyclohexylamino)-2-(1H-indol-3-yl)ethyl)-1,3,4-oxadiazol-2(3H)-on;
- N4-((R)-2-(1H-indol-3-yl)-1-(3-methyl-1,2,4-oxadiazol-5-yl)ethyl)-N1,N1-dimethyl-1-phenylcyclohexan-1,4-diamin;
- N4-((S)-2-(1H-indol-3-yl)-1-(5-methyloxazol-2-yl)ethyl)-N1,N1-dimethyl-1-phenylcyclohexan-1,4-diamin;
- N4-(2-(1H-indol-3-yl)-1-(1H-tetrazol-5-yl)ethyl)-N1,N1-dimethyl-1-phenylcyclohexan-1,4-diamin;
- N4-(2-(1H-Indol-3-yl)-1-(1H-tetrazol-5-yl)ethyl)-N1,N1,N4-trimethyl-1-phenylcyclohexan-1,4-diamin;
- N4-(2-(1H-indol-3-yl)-1-(3-methyl-1,2,4-oxadiazol-5-yl)ethyl)-N1,N1,N4-trimethyl-1-phenylcyclohexan-1,4-diamin;
- N4-(2-(1H-indol-3-yl)-1-(5-methyloxazol-2-yl)ethyl)-N1,N1,N4-trimethyl-1-phenylcyclohexan-1,4-diamin;
- 5-(1-((4-(dimethylamino)-4-phenylcyclohexyl)(methyl)amino)-2-(1H-indol-3-yl)ethyl)-1,3,4-oxadiazol-2(3H)-one;
- 2-((4-(dimethylamino)-4-phenylcyclohexyl)(methyl)amino)-3-(1H-indol-3-yl)-N,N-dimethylpropanamide;
- N4-(2-(1H-indol-3-yl)-1-(3-methyl-1,2,4-oxadiazol-5-yl)ethyl)-1-(3-fluorophenyl)-N1,N1-dimethylcyclohexan-1,4-diamin;
- N4-(2-(1H-indol-3-yl)-1-(5-methyloxazol-2-yl)ethyl)-1-(3-fluorophenyl)-N1,N1-dimethylcyclohexan-1,4-diamin;
- N-(2-(1H-indol-3-yl)-1-(5-methyloxazol-2-yl)ethyl)-N-(4-(dimethylamino)-4-(3-fluorophenyl)cyclohexyl)zimtsäureamid; und
- N-(2-(1 H-indol-3-yl)-1-(3-methyl-1,2,4-oxadiazol-5-yl)ethyl)-N-(4-(dimethylamino)-4-(3-fluorophenyl)cyclohexyl)zimtsäureamid;
- (R)-N4-(2-(1H-Indol-3-yl)-1-(5-methyloxazol-2-yl)ethyl)-N1,N1-dimethyl-1-phenylcyclohexan-1,4-diamin
- (S)-N4-(2-(1H-Indol-3-yl)-1-(4-methylthiazol-2-yl)ethyl)-N1,N1-dimethyl-1-penylcyclo-hexan-1,4-diamin
- (R)-N4-(2-(1H-Indol-3-yl)-1-(4-methylthiazol-2-yl)ethyl)-N1,N1-dimethyl-1-penylcyclo-hexan-1,4-diamin
- N-(2-(1 H-Indol-3-yl)-1-(1H-tetrazol-5-yl)ethyl)-N-(4-dimethylamino-4-phenylcyclohexyl)-2,2,2-trifluoracetamid
- N4-(2-(1H-Indol-3-yl)-1-(1H-tetrazol-5-yl)ethyl)-N1,N1-dimethyl-1-phenylcyclohexan-1,4-diamin
oder deren physiologisch verträgliche Salze und/oder Solvate.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Verbindungen weisen eine Affinität zum µ-Opioid- bzw. zum ORL1-Rezeptor auf und eignen sich daher für die Arzneimittelentwicklung.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nichtäquimolare oder äquimolare Mischung der Diastereomere und/oder Enantiomere vorliegt. Ein weiterer Gegenstand der Erfindung betrifft ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Offenbarung betrifft ein Verfahren zur Herstellung der erfindungsgemäßen Verbindungen wie in der folgenden Beschreibung und Beispielen ausgeführt.

### Synthese von Verbindungen des Typs 1

### a) Methode 1

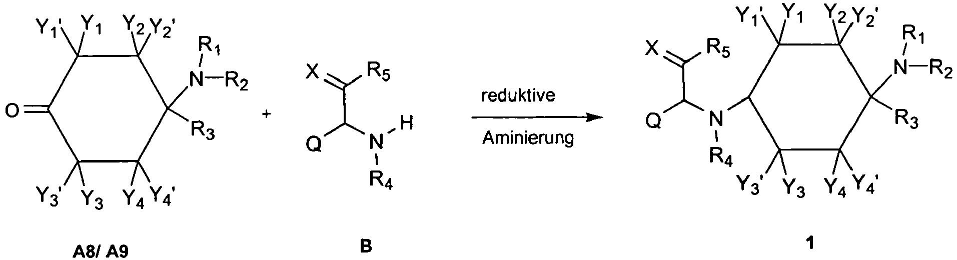

Ketone der allgemeinen Formel A8/ A9 können durch eine reduktive Aminierung mit Aminen der allgemeinen Struktur B in wenigstens einem organischen Lösungsmittel, vorzugsweise aus der Gruppe bestehend aus Diethylether, Tetrahydrofuran, Methanol, Ethanol, Dichlorethan, Dichlormethan und Toluol, unter Zusatz wenigstens eines Reduktionsmittels, vorzugsweise aus der Gruppe bestehend aus Boran-Pyridin Komplex, Natriumborhydrid, Natriumtriacetoxyborhydrid, Natriumcyanoborhydrid und Triethylsilan ggf. in Gegenwart wenigstens einer Säure, vorzugsweise ausgewählt aus der Gruppe bestehend aus Ameisensäure, Essigsäure, Salzsäure und Trifluoressigsäure bei Temperaturen von vorzugsweise -70°C bis 150°C ggf. unter Mikrowelleneinstrahlung zu Verbindungen der allgemeinen Formel 1 umgesetzt werden. Gegebenenfalls wird bei Verbindungen der allgemeinen Formel 1 mit R₄= H anschließend acyliert, alkyliert oder sulfoniert oder bei Verbindungen R₄=mit einer Schutzgruppe geschütztem H, mindestens einmal eine Schutzgruppe abgespalten und ggf. acyliert, alkyliert oder sulfoniert.

### b) Methode 2

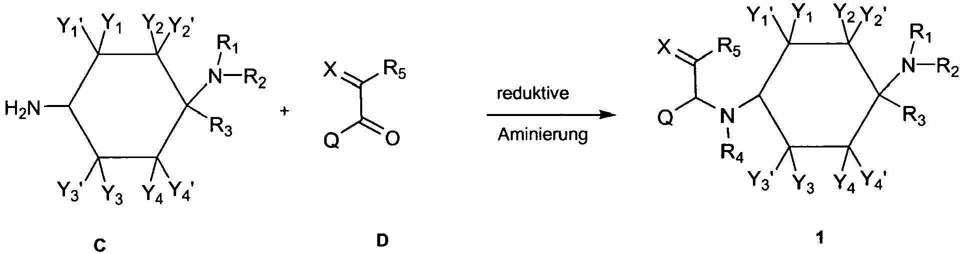

Alternativ dazu können Amine der allgemeinen Struktur C mit Ketonen der allgemeinen Struktur D im Sinne einer reduktiven Aminierung (s.o.) zu Verbindungen der allgemeinen Formel 1 umgesetzt werden.

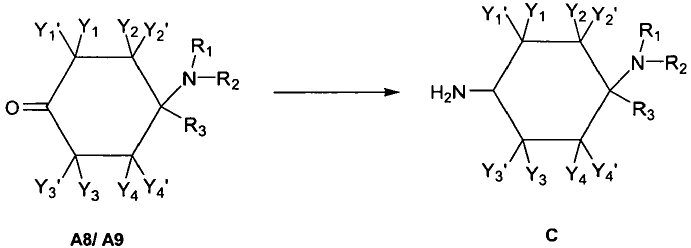

Amine der allgemeinen Struktur C können aus Ketonen der allgemeinen Struktur A8/A9 nach dem Fachmann bekannten Methoden, beispielsweise nach Überführung in die korrespondierenden Oxime und anschließender Reduktion, gewonnen werden.

### Synthese der Ketonbaustein A8/A9

### a) Derivatisierung in der 2,3,5 und/oder 6-Position der Cyclohexandionketale

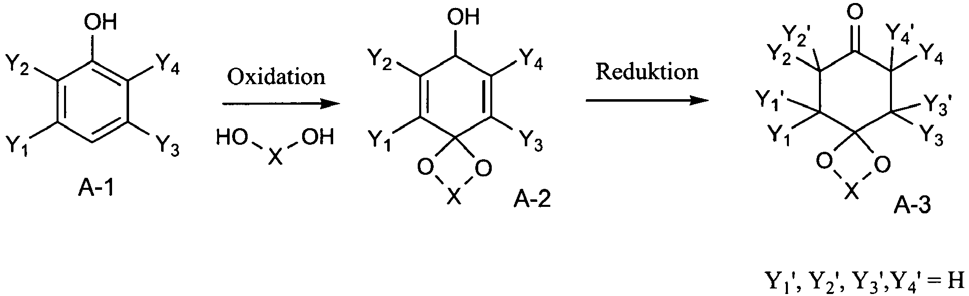

Substituierte Cyclohexandionketale des Typs A-3 lassen sich durch dem Fachmann bekannte Methoden aus den bekannten Edukten A-1 synthetisieren. In der Literatur wird die Oxidation von Phenolen A-1 mittels hypervalenten lodreagenzien zu den intermediären Cyclohexadienonketalen A-2 beschrieben (Rose et al.Can. J. Chem., 74, 1996, 1836.). Verbindungen der Formel A-3 können dann aus den entsprechenden Ketalen A-2, nach dem Fachmann bekannten Methoden durch Reduktion unter Wasserstoffatmosphäre und in Anwesenheit von Metallkatalysatoren z.B. auf Rhodiumbasis erhalten werden.

### b) Derivatisierung in 2-Position der Cyclohexandionketale

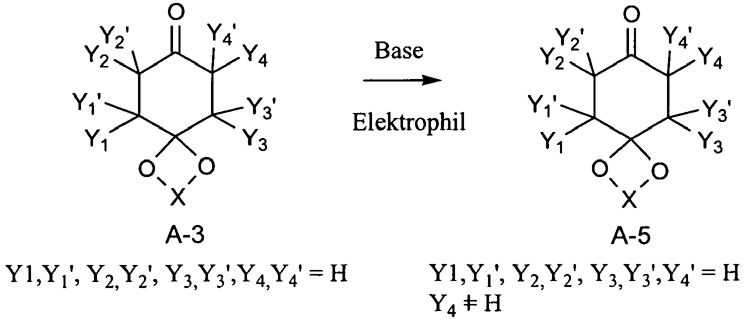

α-substituierte Cyclohexandionketale der allgemeinen Formel A-5 können durch Umsatz der unsubstituierten Ketale A-3 mit einer Base, beispielsweise Lithiumdiisopropylamid (LDA), Lithiumhexamethyldisilazid (LHMDS), Kaliumhexamethyldisilazid (KHMDS), Natriumhydrid (NaH), Kaliumhydrid (KH), Natriummethanolat (NaOMe), Kaliumtertbutoxylat (K^{t}OBu), Aminbasen wie z.B. Diethylamin (HNEt₂), Diisopropylethylamin (Hünig's Base), Piperidin, Pyrrolidin, Prolin, und mit den entsprechenden Elektrophilen z. B. vom Typ Y₄-X (mit X= z. B. Br, I, OTos, OTf etc. und Y₄ = z.B. Alkyl, Benzyl) in organischen Lösungsmitteln oder Lösungsmittelgemischen, beispielsweise Dichlormethan (DCM), Dichlorethan (DCE), Diethylether (Et₂O), Tehtrahydrofuran (THF), Dimethoxyethan (DME), Methanol (MeOH), Ethanol (EtOH), Dimethylformamid (DMF), Dimethylsulfoxid (DMSO) bei Temperaturen zwischen -78°C und 150°C umgesetzt werden. Desweiteren kann das generierte Anion mit entsprechenden Michael-Akzeptorsystemen, umgesetzt werden. Die Einführung von Heteroatomen kann durch Umsetzung mit Dischwefelverbindungen (Y₄ = S-alkyl oder S-aryl), entsprechenen elektrophilen Fluorierungsreagenzien wie z.B. Selectfluor™ (Y₄ = F), entsprechenen elektrophilen Aminierungsreagenzien wie z.B. N-alkoxycarbonyl- oder N-carboxamido-Oxaziridine (Y₄ = NR₂) oder entsprechenen elektrophilen Hydroxylierungsreagenzien wie z.B. Oxodiperoxymolybdän(pyridin)(hexamethylphosphortriamid)-Komplex (MoOPH (Y₄ = OH) erfolgen. Aldolartige Umsetzungen können auch im sauren Milieau erfolgen. Substiuenten können darüber hinaus mittels einer Mannichreaktion unter sauren Bedingungen (Camphersulfonsäure , p-TosOH etc.) eingeführt werden.

Die Synthesen der Cycloxexanonderivate mit der allgemeinen Formel A-3 sind in der Literatur bekannt (WO05066183, WO040043967, WO0290317, US 4065573, Lednicer et al., J. Med. Chem., 23, 1980, 424-430.)

### c) Synthese der Aminocyclohexanone

### (1) Aminonitril-/ Triazol-Route

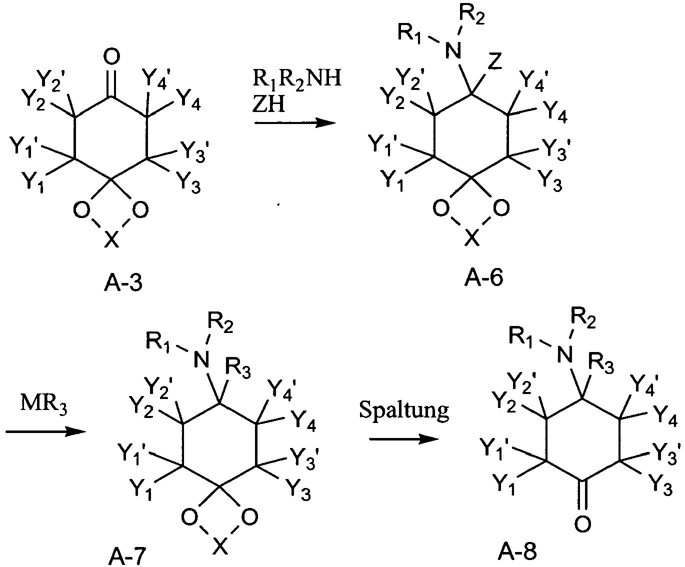

Strukturen der Formel A-6 lassen sich durch Reaktion von Ketonen A-3 mit Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu Verbindungen der Struktur A-6 ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.

Ein weiterer besonders bevorzugter Weg zu Verbindungen der Struktur A-6 ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich A-6 analoge Strukturen mit Benzotriazol- oder Pyrazol- statt Triazolgruppen einführen.

Allgemein können Ketale A-7 auch durch Substitution von geeigneten Abgangsgruppen Z in Strukturen der Formel A-6 erhalten werden. Geeignete Abgangsgruppen sind bevorzugt Cyanogruppen; 1,2,3-Triazol-1-yl-Gruppen. Weitere geeignete Abgangsgruppen sind 1*H-*Benzo[d][1,2,3]triazol-1-yl-Gruppen und Pyrazol-1-yl-Gruppen (Katritzky et al., Synthesis 1989, 66-69).

Ein besonders bevorzugter Weg zu Verbindungen der Struktur A-7 ist die Umsetzung von Aminonitrilen A-6 mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach bekannten Methoden hergestellt werden. Ein weiterer besonders bevorzugter Weg zu Verbindungen der Struktur A-7 ist die Umsetzung von Aminotriazolen A-6 mit entsprechenden Organometallverbindungen, vorzugsweise Grignardverbindungen, vorzugsweise in Ethern, vorzugsweise bei RT.

Die Organometallverbindungen sind entweder käuflich erhältlich oder können nach literaturbekannten Methoden hergestellt werden.

Verbindungen der Formel A-8 können aus entsprechenden Ketalen A-7, oder aus deren Salzen, nach dem Fachmann bekannten Methoden durch Entschützen mittels Säuren freigesetzt werden. Dabei ist X aus der Gruppe Alkyl, Alkyl/ Alkyliden/ mit Aryl oder Alkyl (gesättigt/ungesättigt) substituiertem Alkyliden ausgewählt.

### (2) Iminroute

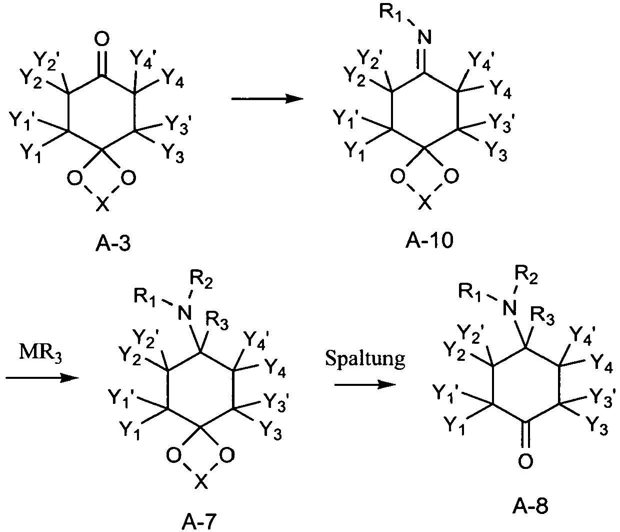

In der Imin-Route wird aus einem Ketonvorläufer A-3 das Imin A-10 synthetisiert, welches unter Verwendung eines Nukleophils MR₃ in den Baustein A-7 und weiter in A-8 überführt wird. Die benötigten Iminbausteine A-10 können nach einer dem Fachmann bekannten Methode hergestellt werden (Layer, Chem. Rev., 1963, 8, 489-510). Für Addition der Organometallspezies MR3 an das Imin A-10 wurde auf literaturbekannte Verfahren (z.B. Maddox et al., J. Med. Chem., 1965, 8, 230-235. Kudzma et al., J. Med. Chem., 1989, 32, 2534-2542.) zurückgegriffen.

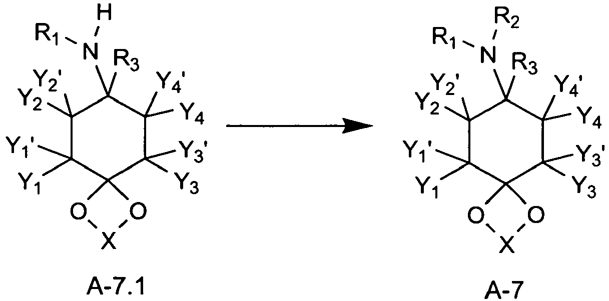

Aminoacetale A-7.1 mit maximal einem Substituenten am Stickstoffatom können nach dem Fachmann prinzipiell bekannte Verfahren, z.B. durch reduktive Aminierung, in entsprechende Amino-acetale A-7 mit einem oder zwei weiteren Substituenten (R2 ≠H) am Stickstoff übergeführt werden.

### d) Derivatisierung in 2-Position der Aminocyclohexanone

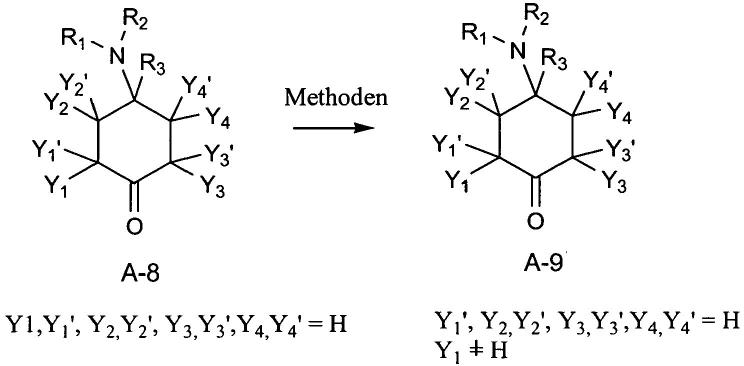

Substituierte Aminocyclohexanone des Typs A-9 lassen sich durch dem Fachmann bekannte Methoden aus den bekannten Edukten A-8 synthetisieren.

### Methode 1:

In der Literatur wird die α-Arylierung von Ketonen A-8 mit den entsprechenden Arylhalogeniden z. B. vom Typ Y₁-X (mit Y₁ = Aryl/Hetaryl und X= Br, I) durch Palladiumkatalyse in Anwesenheit von geeigneten Phosphinliganden wie z.B. Xantphos beschrieben (Elliott et al. Bioorg. Med. Chem. Lett.; EN; 16; 11; 2006; 2929; Dirat et al. Tetrahedron Lett.; EN; 47; 8; 2006; 1295.)

### Methode 2:

α-Substituierte Aminocyclohexanone des Typs A-9 können durch Umsatz der unsubstituierten Ketale A-8 mit einer Base, beispielsweise Lithiumdiisopropylamid (LDA), Lithiumhexamethyldisilazid (LHMDS), Kaliumhexamethyldisilazid (KHMDS), Natriumhydrid (NaH), Kaliumhydrid (KH), Natriummethanolat (NaOMe), Kaliumtertbutoxylat (K^{t}OBu), Aminbasen wie z.B. Diethylamin (HNEt₂), Diisopropylethylamin (Hünig's Base), Piperidin, Pyrrolidin, Prolin, und mit den entsprechenden Elektrophilen z. B. vom Typ Y₄-X (mit X= z. B. Br, I, OTos, OTf etc.) in organischen Lösungsmitteln oder Lösungsmittelgemischen, beispielsweise Dichlormethan (DCM), Dichlorethan (DCE), Diethylether (Et₂O), Tehtrahydrofuran (THF), Dimethoxyethan (DME), Methanol (MeOH), Ethanol (EtOH), Dimethylformamid (DMF), Dimethylsulfoxid (DMSO) bei Temperaturen zwischen -78°C und 150°C umgesetzt werden. Desweiteren kann das generierte Anion mit entsprechenden Michael-Akzeptorsystemen umgesetzt werden. Die Einführung von Heteroatomen kann durch Umsetzung mit Dischwefelverbindungen (Y₄ = S-alkyl oder S-aryl), entsprechenen elektrophilen Fluorierungsreagenzien wie z.B. Selectfluor™ (Y₄ = F), entsprechenen elektrophilen Aminierungsreagenzien wie z.B. N-alkoxycarbonyl- oder N-carboxamido-Oxaziridine (Y₄ = NR₂) oder entsprechenen elektrophilen Hydroxylierungsreagenzien wie z.B. Oxodiperoxymolybdän-(pyridin)(hexamethylphosphortriamid)-Komplex (MoOPH (Y₄ = OH) erfolgen. Aldolartige Umsetzungen können auch im sauren Milieau erfolgen. Substiuenten können darüber hinaus mittels einer Mannichreaktion unter sauren Bedingungen (Camphersulfonsäure , p-TosOH etc.) eingeführt werden.

### Synthese der Aminbausteine vom Typ B

### a) Aminbausteine B.1 für die Synthese von Verbindungen vom Typ 1-b

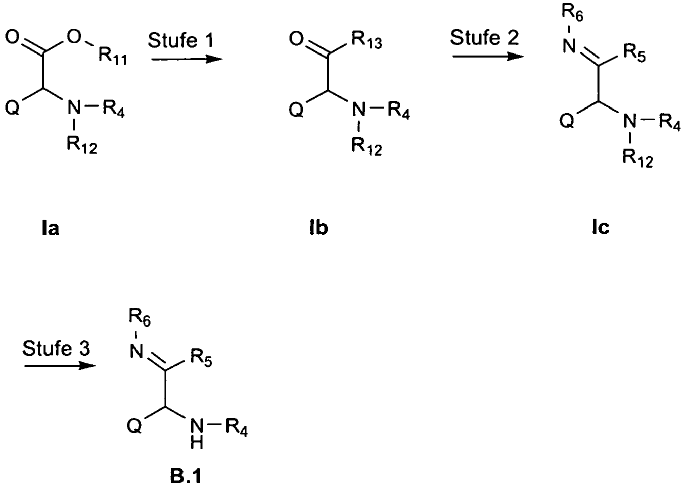

In Stufe 1 werden aus in der Literatur bekannten oder kommerziell erhältlichen alpha-Aminocarbonsäurederivaten der allgemeinen Formel la in der R₁₁ Wasserstoff, Alkyl, Aryl oder Reste, die üblicherweise zur Aktivierung von Carbonsäuren dienen (z. B. N-Succinimidyl oder Chlorid), und R₁₂ typische Aminschutzgruppen (z. B. tert-Butoxycarbonyl oder Benzyloxycarbonyl) darstellen, in alpha-Aminocarbonsäurederivate der Formel Ib überführt, in denen R₁₃ z. B. folgende Reste repräsentiert: -NH₂, -NH-NH₂, -NH-CH₂-C≡CH, -NH-CH₂-CH₂-OH, -O-N=C(NH₂)-CH₃.

In Stufe 2 werden aus Verbindungen der allgemeinen Formel Ib Verbindungen der allgemeinen Formel Ic gewonnen, in dem Verbindungen der allgemeinen Formel Ib in einer oder mehreren Stufen zu aromatischen und nichtaromatischen Heterocyclen der allgemeinen Formel Ic umgewandelt werden. So werden z. B. aus Verbindungen der allgemeinen Formel Ib mit R₁₃ gleich -NH₂ nach Dehydratisierung mit üblichen Reagenzien wie z. B. Trifluoracetanhydrid die entsprechenden Nitrile erhalten, welche durch Reaktion mit Aziden zu 1 H-Tetrazol-5-yl-Derivaten, durch Reaktion mit Hydroxylamin und Reaktion des Intermediates mit Essigsäureanhydrid zu 5-Methyl-[1,2,4]-oxadiazol-3-yl-Derivaten oder durch Reaktion mit einem C1-Baustein wie z. B. N,N-Dimethylformamiddimethylacetal und Weiterreaktion des Zwischenproduktes mit Hydroxylamin zu [1,2,4]Oxadiazol-5-yl-Derivaten umgesetzt werden. Aus Verbindungen der allgemeinen Formel Ib mit R₁₃ gleich -NH-NH₂ werden nach Reaktion mit C1-Bausteinen wie z. B. Phosgen oder N,N'-Carbonyldiimidazol 3H-[1,3,4]Oxadiazol-2-on-5-yl-Derivate erhalten. Aus Verbindungen der allgemeinen Formel Ib mit R₁₃ gleich -NH-CH₂-C≡CH werden durch Einwirkung katalytischer Mengen von Metallsalzen wie z. B. Gold(III)- oder Quecksilber(II)-salzen 5-Methyloxazol-2-yl-Derivate erhalten.

Aus Verbindungen der allgemeinen Formel Ib mit R₁₃ gleich NH-CH₂-CH₂-OH werden durch Dehydratisierung mit üblichen Reagenzien wie z. B. N,N-Diethylaminoschwefeltrifluorid oder Methoxycarbonylsulfamoyl-triethylammonium-hydroxid (Burgess-Reagenz) Oxazolin-2-yl-Derivate erhalten, die durch Oxidation zu Oxazolen reagieren. Aus Verbindungen der allgemeinen Formel Ib mit R¹³ gleich -O-N=C(NH₂)-CH₃ entstehen nach Dehydratisierung in Gegenwart von z. B. Molekularsieb 3-Methyl-[1,2,4]oxadiazol-5-yl-Derivate.

Die Synthesen von Heterocyclen auf den oben beschriebenen Wegen sind in der Literatur bekannt (V. Bavetsias et al., J. Med. Chem. 43, 2000, 1910-1926; A. Hamze et al., J. Org. Chem. 68, 2003, 7316-7321; S. Lee et al., Bull. Korean Chem. Soc. 25, 2004, 207-212; A. S. K. Hashmi et al., Org. Lett. 6, 2004, 4391-4394; T. Morwick et al., Org. Lett. 4, 2002, 2665-2668; K. Thompson et al., J. Med. Chem. 41, 1998, 3923-3927).

### Zur Erläuterung der Stufe 2

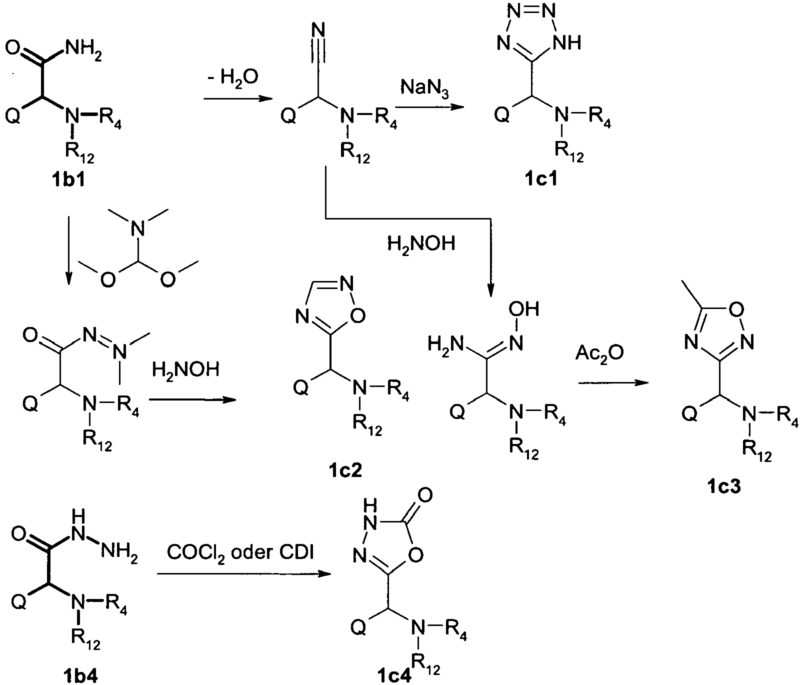

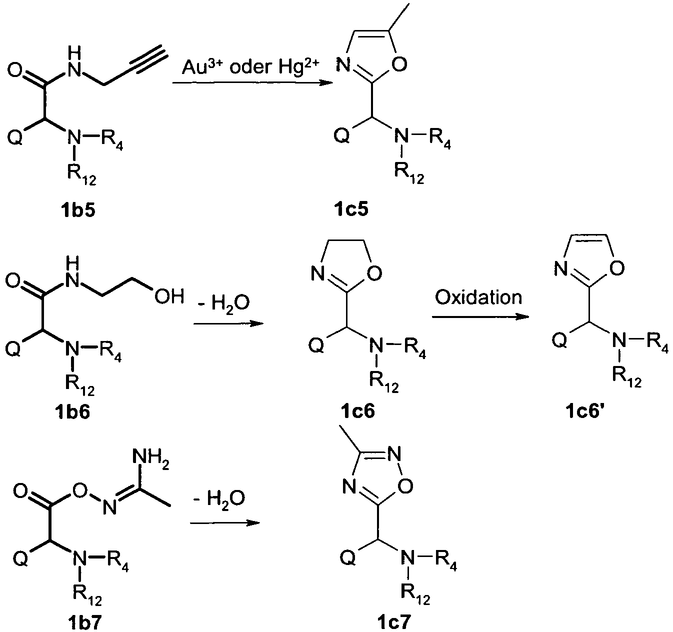

In Stufe 3 werden aus den Verbindungen der allgemeinen Formel 1c die Schutzgruppen auf übliche Weise abgespalten, wodurch die Verbindungen der allgemeinen Formel B.1 erhalten werden.

### b) Aminbausteine B.2 für die Synthese von Verbindungen vom Typ 1-a

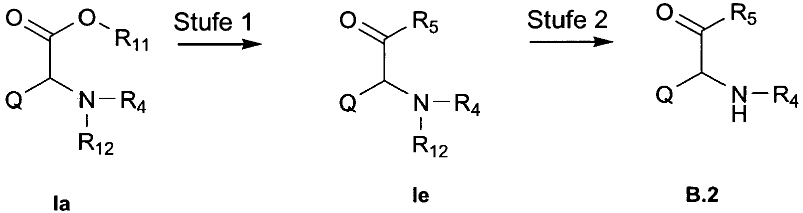

In Stufe 1 werden aus in der Literatur bekannten oder kommerziell erhältlichen alpha-Aminocarbonsäurederivaten der allgemeinen Formel la in der R₁₁ Wasserstoff, Alkyl, Aryl oder Reste, die üblicherweise zur Aktivierung von Carbonsäuren dienen (z. B. N-Succinimidyl oder Chlorid), und R₁₂ typische Aminschutzgruppen (z. B. tert-Butoxycarbonyl oder Benzyloxycarbonyl) darstellen, in alpha-Aminocarbonsäurederivate der Formel le überführt, in denen R₅ z. B. folgende Reste repräsentiert:
-NH₂, -NHMe, -NMe₂;

In Stufe 2 werden aus den Verbindungen der allgemeinen Formel 1 e die Schutzgruppen auf übliche Weise abgespalten, wodurch die Verbindungen der allgemeinen Formel B.2 erhalten werden.

Bezüglich weiterer Einzelheiten zur Synthese der erfindungsgemäßen Verbindungen kann vollumfänglich verweisen werden auf WO2002/090317, WO2002/90330, WO2003/008370, WO2003/008371 , WO2003/080557, WO2004/043899, WO2004/043900, WO02004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

### Beispiele

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### Beispiel 1 und Beispiel 2:

### Stufe 1:

### (S)-2-(4-(Dimethylamino)-4-phenylcyclohexylamino)-3-(1H-indol-3-yl)propanamid (polares und unpolares Diastereomer)

Das Hydrochlorid des L-Tryptophanamids (1,49 g, 6,3 mmol) wurde mit 1,2-Dichlorethan (30 ml), Tetrahydrofuran (20 ml) und gesättigter NaHC03-Lösung (40 ml) 15 min kräftig gerührt und die wäßrige Phase dann sofort mit einem Tetrahydrofuran/Ethylacetat-Gemisch (1 : 3, 5 x 40 ml) extrahiert. Nach dem Trocknen mit Na2S04 wurde die organische Phase eingeengt. Unter Argon wurde die freigesetzte Base (1,3 g, 6,3 mmol) und 4-(Dimethylamino)-4-phenylcyclohexanon (1,3 g, 6,3 mmol) in Tetrahydrofuran (40 ml) und 1,2-Dichlorethan (30 ml) gelöst. Zur klaren Lösung wurde Eisessig (0,37 ml, 6,3 mmol) und Na2S04 (3,2 g) hinzugefügt. Nach einer Reaktionszeit von 15 min wurde die Reaktionsmischung mit NaBH(OAc)3 (2 g, 9 mmol) versetzt und 2 d bei Raumtemperatur gerührt. Zur Aufarbeitung des Ansatzes wurde die Mischung mit gesättigter NaHC03-Lösung (60 ml) versetzt und 15 min gerührt. Die wäßrige Phase wurde mit Dichlormethan (2 x 40 ml) extrahiert. Die vereinigten organischen Phasen wurden nach dem Trocknen eingeengt, wobei ein hellbraunes Öl erhalten wurde. Die chromatographische Trennung des Substanzgemisches an Kieselgel 60 (50 g) erfolgte mit Ethylacetat/Methanol (1 : 1).
Ausbeute (unpolares Diastereomer): 25 % (631 mg), beigefarbener Feststoff
Ausbeute (polares Diastereomer): 298 mg (12 %), beigefarbener Feststoff

### Stufe 2:

### (S)-2-(4-(Dimethylamino)-4-phenylcyclohexylamino)-3-(1H-indol-3-yl)propanamid; Dihydrochlorid (Beispiel 1, unpolares Diastereomer)

Das unpolarere Diastereomer aus Stufe 1 (600 mg, 1,4 mmol) wurde in Ethylmethylketon (1000 ml) gelöst und mit Me3SiCl (0,5 ml, 3,7 mmol) versetzt. Nach 1 h wurde ein weißer, kristalliner Feststoff abgesaugt.
Ausbeute: 340 mg (46 %)
Schmelzpunkt: 181-214°C
13C NMR (101 MHz, DMSO-D6) δ ppm: 22.1, 23.8, 25.0, 26.2, 38.1, 51.2, 58.0, 67.7, 107.2, 111.3, 118.4, 118.5, 120.9, 124.2, 127.2, 128.8, 129.5, 132.6, 135.9, 169.0

### (S)-2-(4-(Dimethylamino)-4-phenylcyclohexylamino)-3-(1H-indol-3-yl)propanamid; Dihydrochlorid (Beispiel 2, polares Diastereomer)

Zu einer Lösung des polareren Diastereomers aus Stufe 1 (280 mg, 0,69 mmol) in Ethylmethylketon (20 ml) wurde Me3SiCl (0,23 ml, 1,7 mmol) gegeben. Nach einer Reaktionszeit von 1 h wurde der Festoff abgesaugt.
Ausbeute: 326 mg (93 %)
Schmelzpunkt: 201-210 °C 13C NMR (101 MHz, DMSO-D6) δ ppm: 23.9, 25.4, 26.2, 28.2, 28.3, 37.2, 54.4, 57.6, 67.6, 106.9, 111.3, 118.3, 118.4, 120.9, 124.3, 127.1, 129.1, 129.6, 135.9, 169.1

### Beispiel 3 und Beispiel 4:

### Stufe 1:

### 2-(4-(Dimethylamino)-4-phenylcyclohexylamino)-3-(1H-indol-3-yl)propanamid Dihydrochlorid (polares und unpolares Diastereomer)

D,L-Tryptophanamid Hydrochlorid (1,49 g, 6,25 mmol) wurde mit 1,2-Dichlorethan (30 ml), Tetrahydrofuran (20 ml) und gesättigter NaHC03-Lösung (40 ml) 15 min kräftig gerührt und die wäßrige Phase dann sofort mit einem Tetrahydrofuran/Ethylacetat-Gemisch (1 : 3, 5 x 40 ml) extrahiert. Nach dem Trocknen mit Na₂SO₄ wurde die organische Phase eingeengt. Unter Argon wurde die freigesetzte Base (1,03 g, 5,06 mmol) und 4-(Dimethylamino)-4-phenylcyclohexanon (1,09 g, 5,06 mmol) in Tetrahydrofuran (40 ml) und 1,2-Dichlorethan (30 ml) gelöst. Zur klaren Lösung wurde Eisessig (0,291 ml, 5,06 mmol) und Na2S04 (2,53 g) hinzugefügt. Nach einer Reaktionszeit von 15 min wurde die Reaktionsmischung mit NaBH(OAc)3 (1,52 g, 7,08 mmol) versetzt und 2 d bei Raumtemperatur gerührt. Zur Aufarbeitung des Ansatzes wurde die Mischung mit gesättigter NaHC03-Lösung (60 ml) versetzt und 15 min gerührt. Die wäßrige Phase wurde mit Dichlormethan (2 x 40 ml) extrahiert. Die vereinigten organischen Phasen wurden nach dem Trocknen eingeengt, wobei ein hellbraunes Öl erhalten wurde. Die chromatographische Trennung des Substanzgemisches an Kieselgel 60 (150 g) erfolgte mit Ethylacetat/Methanol (1 : 1). Ausbeute (unpolares Diastereomer): 821 mg (41 %), beigefarbener Feststoff Ausbeute (polares Diastereomer): 377 mg (19 %), beigefarbener Feststoff

### 2-(4-(Dimethylamino)-4-phenylcyclohexylamino)-3-(1H-indol-3-yl)propanamid Dihydrochlorid (Beispiel 3, unpolares Diastereomer)

Das unpolare Diastereomer aus Stufe 1 (870 mg, 2,1 mmol) wurde in Ethylmethylketon (50 ml) gelöst und mit Me3SiCl (0,73 ml, 5,3 mmol) versetzt. Nach 1 h wurde der ausfgefallene Festoff abgesaugt.
Ausbeute: 900 mg (93 %), weißer, kristalliner Feststoff Schmelzpunkt: 227-233 °C
13C NMR (101 MHz, DMSO-D6) δ ppm: 22.1, 23.8, 24.9, 26.2, 38.1, 51.2, 58.0, 67.7, 107.3, 111.3, 118.4, 118.5, 120.9, 124.2, 127.2, 128.8, 129.5, 132.6, 135.9, 169.0

### 2-(4-(Dimethylamino)-4-phenylcyclohexylamino)-3-(1H-indol-3-yl)propanamid; Dihydrochlorid (Beispiel 4, polares Diastereomer)

Zu einer Lösung des polareren Diastereomers aus Stufe 1 (360 mg, 0,89 mmol) in Ethylmethylketon (250 ml) wurde Me3SiCl (0,3 ml, 2,2 mmol) gegeben. Nach einer Reaktionszeit von 1 h wurde der ausfgefallene Festoff abgesaugt.
Ausbeute: 444 mg (100 %) Schmelzpunkt: 201-210 °C
13C NMR (101 MHz, DMSO-D6) δ ppm: 24.0, 25.4, 26.2, 28.2, 28.3, 37.2, 54.3, 57.6, 67.6, 107.0, 111.3, 118.3, 118.4, 120.9, 124.3, 127.1, 129.1, 129.6, 129.7, 135,9, 169,1

### Beispiel 5 und Beispiel 6:

### Stufe 1:

### (S)-Benzyl 1-(dimethylamino)-3-(1H-indol-3-yl)-1-oxopropan-2-ylcarbamat

Unter Argon wurde (S)-2,5-Dioxopyrrolidin-1-yl 2-(benzyloxycarbonylamino)-3-(1 H-indol-3-yl)propanoat (2 g, 4,6 mmol) in Tetrahydrofuran (60 ml) gelöst. Zu der klaren Lösung wurde Dimethylamin (2M in THF, 4,6 ml, 9,2 mmol) hinzugefügt. Sofort nach Zugabe fiel ein weißer Niederschlag aus. Die Reaktionsmischung wurde 24 h bei Raumtemperatur gerührt. - Zur Aufarbeitung wurde der Ansatz mit 2N HCl auf pH 1 eingestellt. Das wäßrige Gemisch wurde mit Ethylacetat (3 x 40 ml) extrahiert. Die vereinigten organischen Phasen wurden mit gesättigter Natriumhydrogencarbonatlösung (1 x 40 ml) gewaschen und nach dem Trocknen mit NaS04 eingeengt. Das Rohprodukt wurde ohne weitere Reinigung in der nächsten Reaktion weiterverarbeitet. Ausbeute :1,6 g (95 %)

### Stufe 2:

### (S)-2-Amino-3-(1H-indol-3-yl)-N,N-dimethylpropanamid

(S)-Benzyl 1-(dimethylamino)-3-(1H-indol-3-yl)-1-oxopropan-2-ylcarbamat (1,88 g, 5,14 mmol) wurde in abs. Methanol (60 ml) mit Palladium als Katalysator (Pd/C, 5 %, 800 mg) versetzt und 2 h bei RT hydriert (Wasserstoffdruck: 3 bar). Der Katalysator wurde über eine Fritte, versehen mit einer 1 cm hohen Schicht Celite, entfernt. Die Fritte wurde mit Methanol (400 ml) gründlich gewaschen. Das Lösungsmittel wurde im Vakuum abdestilliert.
Ausbeute: 1 g (84 %)

### Stufe 3:

### (S)-2-(4-(Dimethylamino)-4-phenylcyclohexylamino)-3-(1H-indol-3-yl)-N,N-dimethylpropanamid (polares und unpolares Diastereomer)

4-(Dimethylamino)-4-phenylcyclohexanon (1,1 g, 5,41 mmol) wurde unter Argon in einer Mischung aus 1,2-Dichlorethan (30 ml) und Tetrahydrofuran (40 ml) gelöst, mit (S)-2-Amino-3-(1H-indol-3-yl)-N,N-dimethylpropanamid (1 g, 4,3 mmol), Essigsäure (0,31 ml, 5,41 mmol) und Na2S04 (2,7 g) versetzt. Es wurde 15 min bei RT gerührt und dann mit Natriumtriacetoxyborhydrid (1,65 g, 7,57 mmol) versetzt und 48 Stunden gerührt. Zur Aufarbeitung des Ansatzes wurde die Mischung mit gesättigter NaHC03-Lösung (60 ml) versetzt und 15 min gerührt. Die wäßrige Phase wurde mit Dichlormethan (2 x 40 ml) extrahiert. Die vereinigten organischen Phasen wurden nach dem Trocknen eingeengt, wobei ein hellbraunes Öl erhalten wurde. Die chromatographische Trennung des Substanzgemisches an Kieselgel 60 (100 g) erfolgte mit Ethylacetat/Methanol (1 : 1).
Ausbeute (unpolares Diastereomer): 250 mg (11 %), beigefarbener Feststoff
Ausbeute (polares Diastereomer): 430 mg (18 %), beigefarbener Feststof

### Stufe 4:

### (S)-2-(4-(Dimethylamino)-4-phenylcyclohexylamino)-3-(1H-indol-3-yl)-N,N-dimethylpropanamid Dihydrochlorid (Beispiel 5, unpolares Diastereomer)

(S)-2-(4-(Dimethylamino)-4-phenylcyclohexylamino)-3-(1H-indol-3-yl)-N,N-dimethylpropanamid (240 mg, 0,55 mmol, unpolares Diastereomer aus Stufe 3) wurde in Ethylmethylketon (10 ml) gelöst und mit Me3SiCl (0,18 ml, 1,39 mmol) versetzt. Nach 1 h wurde der weiße, kristalline Festoff abgesaugt.
Ausbeute: 160 mg (54 %) Schmelzpunkt: 189-204 °C
13C NMR (101 MHz, DMSO-D6) δ ppm: 22.7, 24.0, 24.8, 25.0, 26.5, 35.4, 36.3, 38.8, 51.5, 55.1, 67.7, 106.6, 111.5, 118.1, 118.5, 121.2, 124.5, 127.0, 128.8, 128.9, 129.4, 132.7, 135.9, 167.6

### (S)-2-(4-(Dimethylamino)-4-phenylcyclohexylamino)-3-(1H-indol-3-yl)-N,N-dimethylpropanamid Dihydrochlorid (Beispiel 6, polares Diastereomer)

Zu einer Lösung von (S)-2-(4-(Dimethylamino)-4-phenylcyclohexylamino)-3-(1H-indol-3-yl)-N,N-dimethylpropanamid (420 mg, 0,9 mmol, polares Diastereomer aus Stufe 3) in Ethylmethylketon (10 ml) wurde Me3SiCl (0,3 ml, 2,4 mmol) gegeben. Nach einer Reaktionszeit von 1 h wurde der Feststoff abgesaugt.
Ausbeute: 460 mg (93 %)
Schmelzpunkt: 199-212 °C 13C NMR (101 MHz, DMSO-D6) δ ppm: 24.5, 25.2, 26.6, 28.3, 28.6, 35.3, 36.1, 37.2, 54.4, 54.5, 67.5, 106.3, 111.5, 117.9, 118.5, 121.1, 124.6, 127.0, 129.1, 129.5, 129.6, 135.9, 167.6

### Beispiel 7 und Beispiel 8:

### Stufe 1:

### (S)-Benzyl 3-(1H-indol-3-yl)-1-(methylamino)-1-oxopropan-2-ylcarbamat

Unter Argon wurde (S)-2,5-Dioxopyrrolidin-1-yl 2-(benzyloxycarbonylamino)-3-(1H-indol-3-yl)propanoat (1,5 g, 3,4 mmol) in Tetrahydrofuran (10 ml) gelöst. Zu dieser klaren Lösung wurde Methylamin (2M in THF, 3,4 ml, 6,88 mmol) hinzugefügt. Sofort nach Zugabe fiel ein weißer Niederschlag aus. Die Reaktionsmischung wurde 24 h bei Raumtemperatur gerührt. Zur Aufarbeitung wurde der Ansatz mit 2N HCl auf pH 1 eingestellt. Das wäßrige Gemisch wurde mit Ethylacetat (3 x 40 ml) extrahiert. Die vereinigten organischen Phasen wurden mit gesättigter Natriumhydrogencarbonatlösung (1 x 40 ml) gewaschen und nach dem Trocknen mit NaS04 eingeengt. Das Rohprodukt wurde ohne weitere Reinigung in der nächsten Reaktion weiterverarbeitet. Ausbeute :1,19 g (100 %)

### Stufe 2:

### (S)-2-Amino-3-(1H-indol-3-yl)-N-methylpropanamid

(S)-Benzyl 3-(1H-indol-3-yl)-1-(methylamino)-1-oxopropan-2-ylcarbamat (187 mg, 0,5 mmol) wurde in abs. Methanol (30 ml) mit Palladium als Katalysator (Pd/C, 5 %, 80 mg) versetzt und 2 h bei RT hydriert (Wasserstoffdruck: 3 bar). Der Katalysator wurde über eine Fritte, versehen mit einer 1 cm hohen Schicht Celite, entfernt. Die Fritte wurde mit Methanol (200 ml) gründlich gewaschen. Das Lösungsmittel wurde im Vakuum abdestilliert.
Ausbeute: 108 mg (99 %)

### Stufe 3:

### (S)-2-(4-(Dimethylamino)-4-phenylcyclohexylamino)-3-(1H-indol-3-yl)-N-methylpropanamid (polares und unpolares Diastereomer)

4-(Dimethylamino)-4-phenylcyclohexanon (781 mg, 3,6 mmol) wurde unter Argon in einer Mischung aus 1,2-Dichlorethan (20 ml) und Tetrahydrofuran (30 ml) gelöst, mit (S)-2-Amino-3-(1H-indol-3-yl)-N-methylpropanamid (790 mg, 3,6 mmol), Essigsäure (0,2 ml, 3,6 mmol) und Na2S04 (1,8 g) versetzt. Es wurde 15 min bei RT gerührt und dann mit

Natriumtriacetoxyborhydrid (1,1 g, 5,04 mmol) versetzt. Nach 48 h Rühren bei RT war dünnschichtchromatographisch kein Ausgangsprodukt mehr nachweisbar. Zur Aufarbeitung des Ansatzes wurde die Mischung mit gesättigter NaHC03-Lösung (60 ml) versetzt und 15 min gerührt. Die wäßrige Phase wurde mit Dichlormethan (2 x 40 ml) extrahiert. Die vereinigten organischen Phasen wurden nach dem Trocknen eingeengt, wobei ein hellbraunes Öl erhalten wurde. Die chromatographische Trennung des Substanzgemisches an Kieselgel 60 (100 g) erfolgte mit Ethylacetat/Methanol (1 : 1).
Ausbeute (unpolares Diastereomer): 500 mg (33 %), beigefarbener Feststoff Ausbeute (polares Diastereomer): 217 mg (14 %), beigefarbener Feststoff

### ((S)-2-(4-(Dimethylamino)-4-phenylcyclohexylamino)-3-(1H-indol-3-yl)-N-methylpropanamid Dihydrochlorid (Beispiel 7, unpolares Diastereomer)

(S)-2-(4-(Dimethylamino)-4-phenylcyclohexylamino)-3-(1H-indol-3-yl)-N-methylpropanamid (480 mg, 1,15 mmol, unpolares Diastereomer aus Stufe 3) wurde in Ethylmethylketon (50 ml) gelöst und mit Me3SiCl (0,39 ml, 2,8 mmol) versetzt. Nach 1 h wurde der Feststoff abgesaugt.
Ausbeute: 570 mg (96 %), weiß, kristallinSchmelzpunkt: 238-240 °C
13C NMR (101 MHz, DMSO-D6) δ ppm: 22.2, 24.0, 24.7, 25.5, 26.1, 38.1, 51.0, 58.6, 67.8, 107.4, 111.4, 118.4, 118.5, 121.0, 124.0, 127.1, 128.9, 129.5, 132.6, 135.9, 167.5

### ((S)-2-(4-(Dimethylamino)-4-phenylcyclohexylamino)-3-(1H-indol-3-yl)-N-methylpropanamid Dihydrochlorid (Beispiel 8, polares Diastereomer)

Zu einer Lösung von (S)-2-(4-(Dimethylamino)-4-phenylcyclohexylamino)-3-(1 H-indol-3-yl)-N-methylpropanamid (210 mg, 0,5 mmol, polares Diastereomer aus Stufe 3) in Ethylmethylketon (20 ml) wurde Me3SiCl (0,17 ml, 1,26 mmol) gegeben. Nach einer Reaktionszeit von 1 h wurde der Feststoff abgesaugt.
Ausbeute: 240 mg (97 %)
Schmelzpunkt: 199-212 °C
13C NMR (101 MHz, DMSO-D6) δ ppm: 24.1, 25.3, 25.6, 26.3, 28.2, 28.2, 37.2, 54.1, 57.8, 67.6, 107.0, 111.4, 118.2, 118.4, 120.9, 124.2, 127.0, 129.1, 129.6, 129.8, 135.9, 167.6

### Beispiel 9 und Beispiel 10:

### Stufe 1:

### (S)-Benzyl 1-hydrazinyl-3-(1H-indol-3-yl)-1-oxopropan-2-ylcarbamat

Eine Lösung von (S)-2-(Benzyloxycarbonylamino)-3-(1 H-indol-3-yl)propionsäure (5.00 g, 15 mmol) in wasserfreiem Tetrahydrofu¬ran (100 mL) wurde mit 1,1'-Carbonyldiimidazol (2.91 g, 18 mmol) versetzt und 2 h bei Raumtemperatur gerührt. Nach beendeter Gasentwicklung wurde eine 1 M Lösung von Hydrazin in Tetrahydrofuran (75 mL, 75 mmol) zugetropft und das Gemisch 2 Tage I bei Raumtemperatur gerührt. Der ausgefallene Feststoff wurde abfiltriert und getrocknet.
Ausbeute: 1.57 g (30 %), weißer Feststoff
Schmelzpunkt: 204-208 °C
1H-NMR (DMSO-d6): 2.80 (d, 1H, J = 9.4, 14.4 Hz); 3.04 (d, 1H, J = 14.7, 4.9 Hz); 4.21 (d, 2H, J = 3.0 Hz); 4.23-4.28 (m, 1 H); 4.93 (d, 2H, J = 1.9 Hz); 6.97 (t, 1 H, J = 7.4 Hz); 7.06 (dt, 1H, J = 7.0, 1.1 Hz); 7.14 (d, 1H, J = 2.1 Hz); 7.23-7.37 (m, 6H); 7.40 (d, 1H, J = 8.4 Hz); 7.62 (d, 1 H, J = 7.7 Hz); 9.24 (t, 1 H, J = 3.3 Hz); 10.79 (s, 1H).
13C-NMR (DMSO-d6): 28.0; 54.1; 65.2; 110.0; 111.2; 118.2; 118.4; 120.8; 123.7; 126.7; 127.2; 127.4; 127.6; 128.2; 136.0; 137.0; 155.6; 171.0.

### Stufe 2:

### (S)-Benzyl 2-(1H-indol-3-yl)-1-(5-oxo-4,5-dihydro-1,3,4-oxadiazol-2-yl)ethylcarbamat

Eine Lösung von (S)-Benzyl 1-hydrazinyl-3-(1H-indol-3-yl)-1-oxopropan-2-ylcarbamat (1.57 g, 4.4 mmol) in wasserfreiem Tetrahydrofuran (220 mL) wurde mit 1,1'-Carbonyldiimidazol (848 mg, 5.23 mmol) und Triethylamin (529 mg, 725 µL, 5.23 mmol) versetzt und bei Raumtemperatur über Nacht gerührt. Das Reaktionsgemisch wurde anschließend i. Vak. eingeengt und der Rückstand (3.20 g) durch Flashchromatographie (200 g, 20 × 5.7 cm) mit Chloroform / Methanol (95:5) gereinigt.
Ausbeute: 1.41 g (85 %), weißer Feststoff
Schmelzpunkt: 82-87 °C
1H-NMR (DMSO-d6): 3.12 (dd, 1H, J = 14.5, 8.4 Hz); 3.22 (dd, 1H, J = 14.6, 6.9 Hz); 4.73 (q, 1H, J = 8.1 Hz); 5.00 (s, 2H); 6.96 (t, 1H, J = 7.4 Hz); 7.08 (dt, 1H, J = 7.1, 0.9 Hz); 7.14 (d, 1 H, J = 2.3 Hz); 7.23-7.37 (m, 6H); 7.51 (d, 1 H, J = 7.8 Hz); 8.03 (d, 1 H, J = 8.0 Hz); 10.85 (s, 1H); 12.16 (br s, 1H).
13C-NMR (DMSO-d6): 27.1; 48.9; 65.5; 109.0; 111.4; 117.9; 118.4; 120.9; 123.8; 127.0; 127.6; 127.7; 128.3; 136.0; 136.7; 154.7; 155.6; 156.4.

### Stufe 3:

### (S)-5-(1-Amino-2-(1H-indol-3-yl)ethyl)-1,3,4-oxadiazol-2(3H)-on

Eine Lösung von (S)-Benzyl 2-(1H-indol-3-yl)-1-(5-oxo-4,5-dihydro-1,3,4-oxadiazol-2-yl)ethylcarbamat (1.41 g, 3.73 mmol) in wasserfreiem Tetrahydrofuran (100 mL) wurde mit 10 % Palladium auf Aktivkohle (160 mg) versetzt und 24 h bei Raumtemperatur und 3 bar hydriert. Es wurde eine weitere Portion 20 % Palladiumhydroxid auf Aktivkohle (160 mg) zugegeben und das Gemisch weitere 24 h bei 3 bar und 40 °C hydriert. Der Katalysator wurde anschließend abfiltriert, das Filtrat i. Vak. eingeengt und der Rückstand (1.24 g) durch Flashchro¬matographie (100 g, 20 × 4.0 cm) mit Chloroform / Methanol (95:5) gereinigt.
Ausbeute: 536 mg (59 %), weißer Feststoff
Schmelzpunkt: 79-85°C
1 H-NMR (DMSO-d6): 3.01 (dd, 1 H, J = 14.3, 6.6 Hz); 3.09 (dd, 1 H, J = 14.2, 7.5 Hz); 3.96 (t, 1 H, J = 7.0 Hz); 6.96 (dt, 1 H, J = 7.0, 1.0 Hz); 7.05 (dt, 1H, J = 8.1, 1.0 Hz); 7.11 (d, 1 H, J = 2.3 Hz); 7.32 (d, 1H, J = 8.0 Hz); 7.47 (d, 1H, J = 7.8 Hz); 10.85 (s, 1H). 13C-NMR (DMSO-d6): 30.4; 49.5; 109.6; 111.4; 117.9; 118.3; 120.8; 123.6; 127.2; 136.0; 155.0; 159.6.

### 5-((S)-1-(4-(Dimethylamino)-4-phenylcyclohexylamino)-2-(1H-indol-3-yl)ethyl)-1,3,4-oxadiazol-2(3H)-on Hydrochlorid (1:1) (Beispiel 9, unpolares Diastereomer) und 5-((S)-1-(4-(Dimethylamino)-4-phenylcyclohexylamino)-2-(1H-indol-3-yl)ethyl)-1,3,4-oxadiazol-2(3H)-on (Beispiel 10, polares Diastereomer)

Eine Lösung von (S)-5-(1-Amino-2-(1H-indol-3-yl)ethyl)-1,3,4-oxadiazol-2(3H)-on (485 mg, 1.98 mmol) und 4-Dimethylamino-4-phenyl¬cyclohexanon (430 mg, 1.98 mmol) in wasserfreiem Tetrahydrofuran (40 mL) wurde mit Natriumsulfat (1.00 g) versetzt und 2 h bei Raumtemperatur gerührt. Nach Zugabe von Essigsäure (297 mg, 283 µL, 4.95 mmol) wurde Natriumtriacetoxyborhydrid (633 mg, 2.97 mmol) zugegeben und die Mischung über Nacht bei Raumtemperatur gerührt. Das Lösungsmittel wurde anschließend i. Vak. entfernt, der Rückstand mit 1 M Kaliumcarbonatlösung (50 mL) versetzt und mit Ethylacetat (3 × 50 mL) extrahiert. Die organische Phase wurde mit Natriumsulfat getrocknet und i. Vak. eingeengt. Das Rohprodukt (780 mg) wurde durch Flashchroma-tographie (38 g, 20 × 2.5 cm) mit Ethylacetat / Methanol (9:1) und 1 % Triethylamin gereinigt.
Ausbeute (Beispiel 10, polares Diastereomer): 158 mg (18 %), weißer Feststoff Schmelzpunkt: 115-121 °C
1 H-NMR (DMSO-d6): 1.45-1.70 (m, 3H); 1.75-1.90 (m, 2H); 1.90 (s, 6H); 2.40-2.48 (m, 3H); 2.50-2.56 (m, 2H); 2.98-3.03 (m, 2H); 3.84 (t, 1 H, J = 7.3 Hz); 6.91-6.96 (m, 1 H); 7.01-7.06 (m, 2H); 7.24 (t, 1H, J = 7.1 Hz); 7.27-7.41 (m, 6H); 10.8 (s, 1H).
13C-NMR (DMSO-d6): 28.1; 28.9; 29.4; 30.5; 30.7; 38.0; 53.6; 54.8; 60.7; 109.5; 111.4; 117.7; 118.3; 120.8; 123.4; 126.1; 127.0; 127.5; 127.7; 136.0; 136.9; 154.9; 158.1.
[α]D24 = -7.1 (c 1.0, MeOH)

Die Fraktion mit dem unpolaren Diastereomer wurde nochmals mit Chloroform / Methanol (5:1) gereinigt. Dabei wurde das Hydrochlorid erhalten.

Ausbeute (Beispiel 9, unpolares Diastereomer): 426 mg (45 %), weißer Feststoff Schmelzpunkt: 152-161 °C
1H-NMR (DMSO-d6): 1.50-2.00 (m, 5H); 2.10-2.45 (br s, 10H); 2.50-2.70 (br s, 1H); 3.05-3.20 (m, 2H); 3.88 (br s, 1 H); 6.95-7.02 (m, 1 H); 7.03-7.10 (m, 1 H); 7.17 (br s, 1 H); 7.31-7.36 (m,1 H); 7.50 (br d, 4H, J = 7.7 Hz); 7.63 (br s, 2H); 9.80-10.20 (br s, 1 H); 10.89 (s, 1 H); 12.00 (s, 1H).
13C-NMR (DMSO-d6): 25.4; 27.7; 28.8; 37.1; 47.8; 53.4; 54.8; 109.5; 117.8; 118.4; 120.8; 123.7; 128.5 (sehr breit); 136.0; 154.8; 157.8.
[α]D24 = -12.3 (c 1.0, MeOH)

### Beispiel 11

Stufe 1:

### N'-Hydroxyacetimidamid

Eine Lösung von 50 % Hydroxylamin in Wasser (3.7 mL, 56 mmol) wurde mit Acetonitril (30 mL) versetzt und 24 h bei 90°C gerührt. Die Reaktionslösung wurde anschließend auf 4 °C abgekühlt, wobei das Produkt auskristallisierte, abfiltriert und i. Vak. getrocknet wurde.
Ausbeute: 2.66 g (64 %), weiße Nadeln
Schmelzpunkt: 137°C 1 H-NMR (400 MHz, CDCl3): 1.83 (s, 3H), 4.53 (br s, 3H).
13C-NMR (100 MHz, CDCl3): 16.9; 151.1.

### Stufe 2:

### (S)-tert-Butyl 1-(1-aminoethylidenaminooxy)-3-(1H-indol-3-yl)-1-oxopropan-2-ylcarbamat

Eine Lösung von N'-Hydroxyacetimidamid (500 mg, 6.74 mmol) und (S)-2-(tert-Butoxycarbonylamino)-3-(1H-indol-3-yl)propionsäure (1.7 g, 5.6 mmol) in Dichlormethan / N,N-Dimethylformamid (30 mL, 9:1) wurde bei -10°C mit 1-Hydroxybenzotriazol-Hydrat (910 mg, 6.74 mmol) und 1,3-Dicyclohexylcarbo¬diimid (1.39 g, 6.74 mmol) versetzt und 20 min bei dieser Temperatur gerührt. Anschließend wurde 2 h bei Raumtemperatur gerührt. Das Reaktionsgemisch wurde i. Vak. eingeengt und der Rückstand in Ethylacetat aufgenommen. Die organische Phase wurde mit Natriumhydrogencarbonat-Lösung (2 × 30 mL), Wasser (30 mL) und Ammoniumchlorid-Lösung (2 x 30 mL) gewaschen und mit Magnesiumsulfat getrocknet.

### Ausbeute: 849 mg (42 %), farbloser Feststoff

Schmelzpunkt: 75-78°C 1 H-NMR (300 MHz, DMSO-d6): 1.33 (s, 9H), 1.76 (s, 3H), 2.98 (dd, J = 14.5, 9.1 Hz, 1 H), 3.15 (dd, J = 14.5, 5.4 Hz, 1 H), 4.38 (dt, J = 8.9, 5.4 Hz, 1 H), 6.36 (br s, 2H), 6.98 (t, J = 6.9 Hz, 1 H), 7.06 (t, J = 6.9 Hz, 1 H), 7.13 (d, J = 2.2 Hz, 1 H), 7.24 (d, J = 8.3 Hz, 1 H), 7.33 (d, J = 7.9 Hz, 1 H), 7.58 (d, J = 7.8 Hz, 1 H), 10.84 (s, 1H).
13C-NMR (100 MHz, DMSO-d6): 16.4; 27.7; 28.7; 54.6; 79.5; 110.2; 112.0; 118.9; 119.3; 121.8; 124.1; 127.6; 136.5; 156.3; 157.6; 170.5.

### Stufe 3:

### (S)-tert-Butyl 2-(1H-indol-3-yl)-1-(3-methyl-1,2,4-oxadiazol-5-yl)ethylcarbamat

Eine Lösung von (S)-tert-Butyl 1-(1-aminoethylidenaminooxy)-3-(1H-indol-3-yl)-1-oxopropan-2-ylcarbamat (1.37 g, 4.0 mmol) in Acetonitril (40 mL) wurde mit Molsieb 4 Å (500 mg) versetzt und 16 h bei 120 °C in einem Teflondruckgefäß gerührt. Das Molsieb wurde anschließend abfiltriert und das Filtrat i. Vak. eingeengt. Der Rückstand wurde durch Flashchromatographie (200 g, 20 x 3.6 cm) mit Chloroform / Methanol / Triethylamin (10:0.2:0.01) gereinigt.
Ausbeute: 879 mg (63 %), farbloser Feststoff
Schmelzpunkt: 45 °C
1H-NMR (300 MHz, DMSO-d6): 1.33 (s, 9H), 2.31 (s, 3H), 3.22 (dd, J = 14.6, 8.4 Hz, 1H), 3.27 (dd, J = 15.0, 6.7 Hz, 1H), 4.99 (q, J = 7.7 Hz, 1 H), 6.98 (t, J = 6.8 Hz, 1H), 7.07 (t, J = 6.9 Hz, 1 H), 7.12 (d, J = 2.1 Hz, 1H), 7.33 (d, J = 7.9 Hz, 1H), 7.49 (d, J = 7.7 Hz, 1 H), 7.74 (d, J = 7.9 Hz, 1H), 10.87 (s, 1H).

### Stufe 4:

### (S)-2-(1H-Indol-3-yl)-1-(3-methyl-1,2,4-oxadiazol-5-yl)ethanamin

Eine Lösung von (S)-tert-Butyl 2-(1H-indol-3-yl)-1-(3-methyl-1,2,4-oxadiazol-5-yl)ethylcarbamat (879 mg, 2.6 mmol) in Dichlormethan (6 mL) wurde mit Trifluoressigsäure (3 mL) versetzt und 30 min bei Raumtemperatur gerührt. Das Reaktionsgemisch wurde anschließend i. Vak. eingeengt, wodurch die Titelverbin¬dung als Trifluoracetat erhalten wurde.
Ausbeute: 940 mg (100 %), braunes Öl
1H-NMR (300 MHz, DMSO-d6): 2.30 (s, 3H), 3.40 (dd, J = 14.6, 8.4 Hz, 1H), 3.47 (dd, J = 15.1, 5.8 Hz, 1 H), 5.06 (dd, J = 8.3, 5.8 Hz, 1 H), 6.98 (t, J = 6.9 Hz, 1 H), 7.09 (t, J = 7.0 Hz, 1 H), 7.13 (dd, J = 5.4, 3.0 Hz, 1 H), 7.36 (d, J = 8.0 Hz, 1 H), 7.41 (d, J = 7.8 Hz, 1 H), 9.01 (s, 3H), 11.05 (s, 1 H).

Zur Freisetzung der Base wurde das Trifluoracetat in Dichlormethan (30 mL) gelöst und mit einer gesättigten Kaliumcarbonat-Lösung (3 x 20 mL) gewaschen. Die organische Phase wurde mit Natriumsulfat getrocknet und i. Vak. eingeengt.
Ausbeute: 640 mg (100 %)

### N4-((S)-2-(1H-indol-3-yl)-1-(3-methyl-1,2,4-oxadiazol-5-yl)ethyl)-N1,N1-dimethyl-1-phenylcyclohexan-1,4-diamin (Beispiel 11, Diastereomerengemisch)

Eine Lösung von (S)-2-(1H-Indol-3-yl)-1-(3-methyl-1,2,4-oxadiazol-5-yl)ethanamin (180 mg, 0.74 mmol) und 4-Dimethylamino-4-phenylcyclohexanon (161 mg, 0.74 mmol) in wasserfreiem Tetrahydrofuran (20 mL) wurde mit Natriumsulfat (500 mg) versetzt und 2 h bei Raumtemperatur gerührt. Nach Zugabe von Essigsäure (111 mg, 1.85 mmol) wurde Natriumtriacetoxyborhydrid (235 mg, 1.11 mmol) zugegeben und das Reaktionsgemisch 72 h bei Raumtemperatur gerührt. Das Lösungsmittel wurde i. Vak. entfernt, der Rückstand mit 1 M Kalium¬carbonat-Lösung (50 mL) versetzt und mit Ethylacetat (3 x 50 mL) extrahiert. Die vereinigten organischen Phasen wurden mit Natriumsulfat getrocknet und i. Vak. eingeengt. Das Rohprodukt wurde durch Flashchromatographie (80 g, 20 x 1.6 cm) mit Chloroform / Methanol / Triethylamin (10:0.2:0.1□10:0.5:0.1) gereinigt. Da das Produkt als Hydrochlorid anfiel, wurde der Rückstand in Ethylacetat (30 mL) gelöst und mit gesättigter Kaliumcarbonat-Lösung (40 mL) gewaschen. Die wässrige Phase wurde mit Ethylacetat (3 x 40 mL) extrahiert und die vereinigten organischen Phasen wurden mit Natriumsulfat getrocknet.

Ausbeute (Diastereomerengemisch, ca. 2:1): 195 mg (59 %), farbloser Feststoff Schmelzpunkt: 47-52 °C
1 H-NMR (400 MHz, DMSO-d6): 1.29-1.64 (m, 9H), 1.87 (s, 2H), 1.88 (s, 4H), 2.24 (s, 1 H), 2.25 (s, 2H), 2.35 (d, J = 8.4 Hz, 0.33H), 2.42 (d, J = 12.4 Hz, 0.67H), 3.12 (d, J = 7.4 Hz, 0.67H), 3.22 (d, J = 6.2 Hz, 1.33H), 4.27 (q, J = 7.5 Hz, 0.33H), 4.38 (q, J = 7.5 Hz, 0.67H), 6.92 (t, J = 6.9 Hz, 0.33H), 6.94-6.99 (m, 0.67H), 6.99-7.03 (m, 0.33H), 7.04-7.08 (m, 1 H), 7.13-7.25 (m, 3H), 7.25-7.39 (m, 3.33H), 7.43 (d, J = 7.85 Hz, 1.33H), 10.77 (s, 0.33H), 10.83 (s, 0.67H).
13C-NMR (100 MHz, DMSO-d6): 11.1; 24.4; 25.3; 26.8; 28.0; 28.3; 29.4; 30.2Ä; 30.4; 30.5; 30.7; 37.4; 38.0; 53.0; 53.2; 53.3; 53.8; 58.6; 60.7; 109.3; 109.4; 111.3; 111.4; 117.8; 117.9; 118.3; 118.3; 120.8; 120.9; 123.4; 123.5; 126.1; 126.7; 126.9; 127.0; 127.0;127.2; 127.5; 127.6; 128.1; 128.8; 135.9; 136.0; 138.7; 166.3; 181.3; 181.4.

### Beispiel 12 und Beispiel 13:

### Stufe 1:

### (R)-Benzyl 1-hydrazinyl-3-(1H-indol-3-yl)-1-oxopropan-2-ylcarbamat

Eine Lösung von (R)-2-(Benzyloxycarbonylamino)-3-(1 H-indol-3-yl)propionsäure (6.76 g, 20 mmol) in wasserfreiem Tetrahydrofuran (100 mL) wurde mit 1,1'-Carbonyldiimidazol (3.89 g, 24 mmol) versetzt und 2 h bei Raumtemperatur gerührt. Nach beendeter Gasentwicklung wurde eine 1 M Lösung von Hydrazin in Tetrahydrofuran (100 mL, 100 mmol) zugetropft und 18 h bei Raumtemperatur gerührt. Der ausgefallene Feststoff wurde abfiltriert.
Ausbeute: 2.42 g (34 %), weißer Feststoff
Schmelzpunkt: 205-210°C
1H-NMR (DMSO-d6): 2.91 (dd, 1H, J = 14.2, 9.2 Hz); 3.04 (dd, 1H, J = 14.4, 5.5 Hz); 4.20-4.27 (m, 1H); 4.93 (d, 1H, J = 3.1 Hz); 6.97 (t, 2H, J = 7.6 Hz); 7.05 (t, 1H, J = 7.1 Hz); 7.14 (d, 1H, J = 2.1 Hz); 7.24-7.40 (m, 6H); 7.61 (d, 1H, J = 7.6 Hz); 9.22 (s, 1H); 10.79 (s, 1H). 3H unter einem breiten Signal von 3.5-5.0 ppm.

### Stufe 2:

### (R)-Benzyl 2-(1H-indol-3-yl)-1-(5-oxo-4,5-dihydro-1,3,4-oxadiazol-2-yl)ethylcarbamat

Eine Lösung von (R)-Benzyl 1-hydrazinyl-3-(1H-indol-3-yl)-1-oxopropan-2-ylcarbamat (2.42 g 6.86 mmol) in wasserfreiem Tetrahydrofuran (100 mL) wurde mit 1,1'-Carbonyldiimidazol (1.31 g, 8.08 mmol) und Triethylamin (817 mg, 1.12 mL, 8.08 mmol) versetzt und bei Raumtemperatur 2 Tage gerührt. Das Reaktionsgemisch wurde anschließend i. Vak. eingeengt und der Rückstand (4.50 g) durch Flashchromatographie (100 g, 20 × 4.0 cm) mit Chloro¬form / Methanol (9:1) gereinigt.
Ausbeute: 2.30 g (88 %), weißer Feststoff
Schmelzpunkt: 81-88 °C
1 H-NMR (DMSO-d6): 3.13 (dd, 1 H, J = 14.5, 8.3 Hz); 3.23 (dd, 1 H, J = 14.5, 6.9 Hz); 4.73 (q, 1 H, J = 8.2 Hz); 5.00 (s, 2H); 6.98 (t, 1 H, J = 7.6 Hz); 7.07 (dt, 1 H, J = 7.5, 1.0 Hz); 7.15 (d, 1H, J = 2.2 Hz); 7.25-7.40 (m, 6H); 7.52 (d, 1H, J = 7.8 Hz); 8.05 (d, 1H, J = 7.8 Hz); 10.88 (s, 1H); 12.18 (br s, 1H).
13C-NMR (DMSO-d6): 27.1; 48.9; 65.5; 109.0; 111.4; 117.9; 118.4; 120.9; 123.8; 127.0; 127.6; 127.7; 128.3; 136.0; 136.7; 154.7; 155.5; 156.4.

### Stufe 3:

### (R)-5-(1-Amino-2-(1H-indol-3-yl)ethyl)-1,3,4-oxadiazol-2(3H)-on

Eine Lösung von (R)-Benzyl 2-(1H-indol-3-yl)-1-(5-oxo-4,5-dihydro-1,3,4-oxadiazol-2-yl)ethylcarbamat (2.00 g, 5.3 mmol) in wasserfreiem Tetrahydrofuran (100 mL) wurde mit 20 % Palladiumhydroxid auf Aktivkohle (230 mg) versetzt und 5 h bei 40 °C und 3 bar hydriert. Es wurde erneut Katalysator (200 mg) zugegeben und weitere 18 h bei 40 °C und 6 bar hydriert. Nun wurde Methanol (50 mL) hinzugegeben und für weitere 24 h bei 40 °C und 6 bar hydriert. Der Katalysator wurde abfiltriert, das Filtrat i. Vak. eingeengt und der Rückstand (1.43 g) durch Flashchromatographie (100 g, 20 × 4.0 cm) mit Chloroform / Methanol (9:1) gereinigt.
Ausbeute: 684 mg (53 %), gelblicher Feststoff
Schmelzpunkt: 66-80 °C
1H-NMR (DMSO-d6): 3.01 (dd, 1H, J = 14.5, 6.5 Hz); 3.09 (dd, 1H, J = 14.3, 7.4 Hz); 3.97 (t, 1H, J = 7.0 Hz); 6.96 (ddd, 1H, J = 8.0, 7.0, 1.0 Hz); 7.06 (ddd, 1H, J = 8.1, 7.0, 1.2 Hz); 7.12 (d, 1H, J = 2.4 Hz); 7.33 (td, 1H, J = 8.1, 0.9 Hz); 7.47 (d,1H, J = 8.0 Hz); 10.84 (s, 1H). Drei austauschbare Protonen konnten nicht identifiziert wer¬den.
13C-NMR (DMSO-d6): 30.4; 49.5; 109.6; 111.4; 118.0; 118.3; 120.8; 123.6; 127.2; 136.0; 155.0; 159.6.

### 5-((R)-1-(4-(Dimethylamino)-4-phenylcyclohexylamino)-2-(1H-indol-3-yl)ethyl)-1,3,4-oxadiazol-2(3H)-on (Beispiel 12, unpolares Diastereomer) und (Beispiel 13, polares Diastereomer)

Eine Lösung von (R)-5-(1-Amino-2-(1H-indol-3-yl)ethyl)-1,3,4-oxadiazol-2(3H)-on (673 mg, 2.75 mmol) und 4-Dimethylamino-4-phenyl¬cyclohexanon (597 mg, 2.75 mmol) in wasserfreiem Tetrahydrofuran (100 mL) wurde mit Natriumsulfat (1.00 g) versetzt und 4 h bei Raumtemperatur gerührt. Nach Zugabe von Essigsäure (412 mg, 393 µL, 6.87 mmol) wurde Natriumtriacetoxy¬borhydrid (878 mg, 4.12 mmol) zugegeben und die Mischung 2 Tage bei Raumtemperatur gerührt. Das Lösungsmittel wurde anschließend i. Vak. einge-engt, der Rückstand mit 1 M Kaliumcarbonatlösung (50 mL) versetzt und mit Ethyl¬acetat (3 × 30 mL) extrahiert. Die organische Phase wurde mit Natriumsulfat getrocknet und i. Vak. eingeengt. Das Rohprodukt (1.15 g) wurde durch Flashchro¬matographie (100 g, 20 × 4.0 cm) mit Ethylacetat / Methanol (9:1) und 1 % Triethylamin gereinigt.

Das erhaltene unpolarere Produkt (635 mg) wurde nochmals durch Flashchromatographie (38 g, 20 × 2.5 cm) mit Chloroform / Methanol (5:1) gereinigt. Die isolierte Substanz (571 mg) wurde in 1 M Kaliumcarbonatlösung (20 mL) aufgenommen und mit Ethylacetat (6 × 10 mL) extrahiert. Die vereinigten organischen Phasen wurden mit Natriumsulfat getrocknet und i. Vak. eingeengt.

Ausbeute (Beispiel 12, unpolares Diastereomer): 423 mg (35 %), weißer Schaum Schmelzpunkt: 110-113°C
1H-NMR (DMSO-d6): 1.30-1.65 (m, 6H); 1.89 (s, 6H); 2.11 (br s, 1 H); 2.38-2.48 (m, 2H); 3.07-3.12 (m, 2H); 3.94 (br s, 1H); 6.97 (dd, 1 H, J = 7.9, 7.1, 1.1 Hz); 7.02-7.09 (m, 1 H); 7.12 (d, 1H, J = 2.3 Hz); 7.18-7.36 (m, 6H); 7.47 (s, 1H, J = 7.8 Hz); 10.85 (s, 1H); 11.90 (br s, 1H). 13C-NMR (DMSO-d6): 20.7; 26.8; 28.3; 29.1; 30.1; 31.1; 53.4; 109.6; 111.4; 117.8; 118.4; 120.9; 123.6; 126.9; 127.1; 127.3; 136.0; 154.9; 158.2.
[α]D24 = + 9.5 (c 1.0, MeOH)

Das erhaltene polarere Produkt (183 mg (15 %)) wurde in 1 M Kaliumcarbonatlösung (10 mL) aufgenommen wurde und die wässrige Phase mit Ethylacetat (6 × 5 mL) extrahiert. Die vereinigten organischen Phasen wurden mit Natriumsulfat getrocknet und i. Vak. eingeengt. Ausbeute (Beispiel 13, polares Diastereomer): 98 mg (8 %), weißer Feststoff Schmelzpunkt: 90-105 °C
1 H-NMR (DMSO-d6): 0.80-1.00 (m, 2H); 1.40-1.82 (m, 6H); 1.88 (s, 6H); 2.40-2.47 (m, 2H); 2.98 (d, 2H, J = 7.3 Hz); 3.82 (br s, 1 H); 6.93 (ddd, 1 H, J = 7.9, 7.1, 1.0 Hz); 6.95-7.06 (m, 2H); 7.21-7.41 (m, 7H); 10.78 (s, 1H, ), 11.95 (br s, 1 H).
13C-NMR (DMSO-d6): 20.7; 28.1; 28.9; 29.4; 30.5; 37.9; 53.5; 109.5; 111.4; 117.7; 118.3; 120.8; 123.4; 126.5; 127.0; 127.7; 127.9: 136.0; 154.9; 158.1.
[α]D24 = + 4.4 (c 1.0, MeOH)

### Beispiel 14 und Beispiel 15:

### Stufe 1:

### (R)-tert-Butyl 1-(1-aminoethylidenaminooxy)-3-(1H-indol-3-yl)-1-oxopropan-2-ylcarbamat

In Analogie zu Beispiel 11 (Stufe 2) wurden N'-Hydroxyacetimidamid (400 mg, 5.39 mmol, Herstellung vgl. Stufe 1 aus Bsp. 11) und (R)-2-(tert-Butoxycarbonylamino)-3-(1H-indol-3-yl)propionsäure (1.36 g, 4.48 mmol) umgesetzt.
Ausbeute: 1.91 g (98 %)
Schmelzpunkt: 70-75 °C
1H-NMR (300 MHz, DMSO-d6): 1.33 (s, 9H), 1.76 (s, 3H), 2.99 (dd, J = 14.2, 9.1 Hz, 1 H), 3.16 (dd, J = 14.5, 5.1 Hz, 1 H), 4.38 (dt, J = 8.7, 5.3 Hz, 1 H), 6.37 (br s, 2H), 6.98 (t, J = 6.9 Hz, 1 H), 7.07 (t, J = 6.9 Hz, 1 H), 7.14 (d, J = 2.2 Hz, 1 H), 7.24 (d, J = 8.4 Hz, 1 H), 7.33 (d, J = 7.9 Hz, 1 H), 7.58 (d, J = 7.7 Hz, 1 H), 10.84 (s, 1H).

### Stufe 2:

### (R)-tert-Butyl 2-(1H-indol-3-yl)-1-(3-methyl-1,2,4-oxadiazol-5-yl)ethylcarbamat

Eine Lösung von (R)-tert-Butyl 1-(1-aminoethylidenaminooxy)-3-(1H-indol-3-yl)-1-oxopropan-2-ylcarbamat (1.91 g, 5.3 mmol) in Acetonitril (40 mL) wurde mit Molsieb 4 A (1 g) versetzt und 48 h bei 120 °C in Teflondruckgefäß gerührt. Das Mol¬sieb wurde anschließend abfiltriert und das Filtrat i. Vak. eingeengt. Der Rückstand wurde durch Flashchromatographie (200 g, 20 x 3.6 cm) mit Chloroform / Methanol / Triethylamin (10:0.2:0.01) gereinigt.
Ausbeute: 1.81 g (100 %)

### Stufe 3:

### (R)-2-(1H-Indol-3-yl)-1-(3-methyl-1, 2,4-oxadiazol-5-yl)ethanamin

Eine Lösung von (R)-tert-Butyl 2-(1H-indol-3-yl)-1-(3-methyl-1,2,4-oxadiazol-5-yl)ethylcarbamat in Dichlormethan (10 mL) wurde mit Trifluoressigsäure (6 mL) versetzt und 5 h bei Raumtemperatur gerührt. Das Reaktionsgemisch wurde anschließend i. Vak. eingeengt. Der Rückstand wurde in Dichlormethan (30 mL) gelöst und mit gesättigter Kaliumcarbonat-Lösung (3 x 20 mL) gewaschen. Die organische Phase wurde mit Natriumsulfat getrocknet und i. Vak. eingeengt. Der Rückstand wurde durch Flashchromatographie (200 g, 20 x 3.6 cm) mit Chloroform / Methanol / Triethylamin (10:0.2:0.1) gereinigt.
Ausbeute: 727mg (100 %), bräunliches Öl
1H-NMR (300 MHz, DMSO-d6: 2.27 (s, 5H), 3.13 (dd, J = 14.3, 6.6 Hz, 1H), 3.21 (dd, J = 14.2, 7.2 Hz, 1 H), 4.36 (t, J = 6.8 Hz, 1 H), 6.95 (t, J = 6.9 Hz, 1 H), 7.05 (t, J = 6.9 Hz, 2H), 7.31 (d, J = 8.0 Hz, 1H), 7.44 (d, J = 7.6 Hz, 1H), 10.84 (s, 1H).

### N4-((R)-2-(1H-indol-3-yl)-1-(3-methyl-1,2,4-oxadiazol-5-yl)ethyl)-N1, N1-dimethyl-1-phenylcyclohexan-1,4-diamin (Beispiel 14, unpolares Diastereomer) und (Beispiel 15, polares Diastereomer)

Eine Lösung von (R)-2-(1H-Indol-3-yl)-1-(3-methyl-1,2,4-oxadiazol-5-yl)ethanamin (400 mg, 1.65 mmol) und 4-Dimethylamino-4-phenylcyclohexanon (358.7 g, 1.65 mmol) in wasserfreiem Tetrahydrofuran (50 mL) wurde mit Natriumsulfat (1.1 g) versetzt und 2 h bei Raumtemperatur gerührt. Nach Zugabe von Essigsäure (248 mg, 4.1 mmol) wurde Natriumtriacetoxyborhydrid (524 mg, 2.47 mmol) zugegeben und das Reaktionsgemisch 16 h bei Raumtemperatur gerührt. Das Lösungsmittel wurde i. Vak. entfernt, der Rückstand mit 1 M Kalium¬carbonat-Lösung (50 mL) versetzt und mit Ethylacetat (3 x 50 mL) extrahiert. Die vereinigten organischen Phasen wurden mit Natriumsulfat getrocknet und i. Vak. eingeengt. Das Rohprodukt wurde durch Flashchromatographie (400 g, 20 x 5.6 cm) mit Ethylacetat / Isopropanol / Triethylamin (10:0.3:0.01 → 5:1:0.1) gereinigt.
Ausbeute (Beispiel 14, unpolares Diastereomer): 315 mg (43 %), farbloser Feststoff Schmelzpunkt: 52-54°C

1 H-NMR (400 MHz, DMSO-d6): 1.39 (t, J = 14.5 Hz, 2H), 1.48 (t, J = 11.0, 2H), 1.58 (d, J = 12.0 Hz, 2H), 1.89 (s, 6H), 2.25 (s, 3H), 2.36 (dd, J = 9.2, 5.0 Hz, 2H), 2.42 (d, J = 13.0 Hz, 2H), 3.22 (d, J = 6.6 Hz, 2H), 4.38 (t, J = 7.0 Hz, 1 H), 6.97 (ddd, J = 7.9, 7.0, 1.0, Hz, 1 H), 7.05 (ddd, J = 8.2, 7.2, 1.1 Hz, 2H), 7.21 (t, J = 6.9 Hz, 1 H), 7.25-7.34 (m, 5H), 7.44 (d, J =
7.9 Hz, 1H), 10.81 (s, 1H).
13C-NMR(100 MHz, DMSO-d6): 11.0; 26.8; 28.3; 30.4; 37.4; 53.1; 53.2; 58.6; 109.4; 111.3;
117.8; 118.3; 120.8; 123.5; 126.1; 126.6; 127.1; 0; 127.2; 136.0; 138.8; 166.0; 181.4. Ausbeute (Beispiel 15, polares Diastereomer): 98 mg(13 %), farbloser Feststoff Schmelzpunkt: 62-64 °C
1 H-NMR (300 MHz, DMSO-d6): 0.90 (q, J = 9.8 Hz, 3H), 1.53 (m, 3H), 1.80 (d, J = 13.1 Hz, 2H), 1.92 (s, 6H), 2.24 (s, 3H), 2.32-2.47 (m, 2H), 3.12 (d, J = 7.2 Hz, 2H), 4.27 (t, J = 7.0 Hz, 1H), 6.92 (td, J = 6.8, 4.1 Hz, 2H), 7.02 (t, J = 6.9 Hz, 1H), 7.22-7.43 (m, 7H), 10.77 (s, 1H).
13C-NMR (100 MHz, DMSO-d6): 11.0; 28.0; 29.3; 30.2; 30.4; 30.5; 37.9; 53.2; 53.7; 54.8; 109.3; 111.3; 117.8; 118.3; 120.8; 123.4; 126.3; 126.9; 127.4; 127.6; 127.8; 135.9; 166.3; 181.3.

### Beispiel 16 und Beispiel 17:

### Stufe 1:

### (S)-tert-Butyl 2-(1H-indol-3-yl)-3-oxo-3-(prop-2-ynylamino)propylcarbamat

Eine Lösung von (S)-3-(tert-Butoxycarbonylamino)-2-(1H-indol-3-yl)propionsäure (1.0 g, 3.3 mmol) in THF (7 mL) wurde bei Raumtempe¬ratur portionsweise mit Carbonyldiimidazol (535 mg, 3.3 mmol) versetzt und 2 h bei Raumtempe¬ratur gerührt, bevor Propargylamin (270 mg, 5.0 mmol) tropfenweise hinzugegeben wurde. Nach 1 h bei Raumtemperatur wurde mit Diethylether (80 mL) verdünnt, mit gesättigter Natriumhydrogencarbonat-Lösung (2 x 20 mL) gewaschen und mit Natriumsulfat getrocknet. Das Lösemittel wurde i. Vak. entfernt und der Rückstand aus Diethylether umkristallisiert.
Ausbeute: 890 mg (79 %), weißer Feststoff 1H-NMR (CDCl3): 1.42 (9 H, s); 2.14 (1 H, t, J = 2.4 Hz); 3.15-3.33 (2 H, m); 3.93 (2 H, br s); 4.43 (1 H, br s); 5.10 (1 H, br s); 6.00 (1 H, br s); 7.06 (1 H, d, J = 2 Hz); 7.14 (1 H, m); 7.21 (1 H, m); 7.37 (1 H, d, J = 8 Hz); 7.65 (1 H, d, J = 8 Hz); 8.10 (1 H, br s).

### Stufe 2:

### (S)-tert-Butyl 2-(1H-indol-3-yl)-2-(5-methyloxazol-2-yl)ethylcarbamat

Eine Lösung von Gold(III)chlorid (45 mg, 0.15 mmol) in Acetonitril (2 mL) wurde bei Raumtemperatur mit einer Lösung von (S)-tert-Butyl 2-(1 H-indol-3-yl)-3-oxo-3-(prop-2-ynylamino)propylcarbamat (512 mg, 1.5 mmol) in Acetonitril (6 mL) versetzt und 18 h bei 50°C gerührt. Das Lösemittel wurde i. Vak. entfernt und der Rückstand durch Flashchromatographie (20 g, 16 x 2.5 cm) mit Ethylacetat / Cyclohexan (1:1) gereinigt. Ausbeute: 330 mg (64 %)
1 H-NMR (CDCl3): 1.41 (9 H, s); 2.23 (3 H, s); 3.38 (2 H, m); 5.20 (2 H, br s); 6.61 (1 H, s); 6.90 (1 H, s); 7.07 (1 H, t, J = 7.6 Hz); 7.16 (1 H, t, J = 7.6 Hz); 7.32 (1 H, d, J = 8 Hz); 7.43 (1 H, d, J = 8 Hz); 8.06 (1 H, br s).

### Stufe 3:

### (S)-2-(1H-Indol-3-yl)-2-(5-methyloxazol-2-yl)ethanamin

Eine Lösung von (S)-tert-Butyl 2-(1 H-indol-3-yl)-2-(5-methyloxazol-2-yl)ethylcarbamat (320 mg, 0.93 mmol) in Dichlormethan (10 mL) wurde bei 0°C tropfenweise mit Trifluoressigsäure (1.5 mL, 20 mmol) versetzt und 3 h bei Raumtempe¬ratur gerührt. Anschließend wurde mit Dichlormethan (80 mL) verdünnt, mit gesättigter Natriumhydrogencarbonat-Lösung (30 mL) gewaschen, mit Natrium-sulfat getrocknet und das Lösemittel i. Vak. entfernt.

Ausbeute: 220 mg (98 %), braunes Öl 1 H-NMR (CDCl3): 2.09 (2 H, br s); 2.29 (3 H, s); 3.18 (1 H, dd, J = 14.4 und 8.4 Hz); 3.42 (1 H, dd, J = 14.4 und 5 Hz); 4.38 (1 H, m); 6.61 (1 H, s); 7.05 (1 H, s); 7.10 (1 H, m); 7.19 (1 H, m); 7.35 (1 H, d, J = 8 Hz); 7.55 (1 H, d, J = 8 Hz); 8.09 (1 H, br s).

### N4-((S)-2-(1H-Indol-3-yl)-1-(5-methyloxazol-2-yl)ethyl)-N1,N1-dimethyl-1-phenylcyclohexan-1,4-diamin (Beispiel 16, unpolares Diastereomer) und (Beispiel 17, polares Diastereomer)

Eine Lösung von (S)-2-(1H-Indol-3-yl)-2-(5-methyloxazol-2-yl)ethanamin (220 mg, 0.91 mmol) und 4-Dimethylamino-4-phenylcyclo¬hexanon (218 mg, 1.0 mmol) in 1,2-Dichlorethan (15 mL) wurde mit gepulvertem Natriumtriacetoxyborhydrid (290 mg, 1.37 mmol) und Essigsäure (100 mg, 1.67 mmol) versetzt und 18 h bei Raumtemperatur gerührt.

Anschließend wurde mit Ethylacetat (80 mL) verdünnt, mit gesättigter Natriumhydrogencarbonat-Lösung (20 mL) gewaschen und mit Natrium-sulfat getrocknet. Das Lösemittel wurde i. Vak. entfernt und der Rückstand durch Flashchromatographie (15 g, 12 x 2.5 cm) mit Methanol / Dichlormethan (1:4) gereinigt.
Ausbeute (Beispiel 16, unpolares Diastereomer): 145 mg (36 %), weißer Feststoff Schmelzpunkt: 65-70°C.
αD20: -11° (c 0.2, MeOH).
1 H NMR (CDCl3): δ 1.40-1.60 (6 H, s); 1.90-2.20 (8 H, m); 2.25 (3 H, s); 2.35-2.47 (2 H, m); 3.22-3.33 (2 H, m); 4.27 (1 H, m); 6.60 (1 H, s); 7.10 (1 H, t, J = 7.6 Hz); 7.18 (1 H, t, J = 7.6 Hz); 7.26-7.37 (7 H, m); 7.57 (1 H, d, J = 8 Hz); 8.20 (1 H, br s).
13C NMR (CDCl3): δ 165.1; 148.5; 137.7; 136.2; 127.6; 127.5; 127.3; 126.8; 123.3; 122.3; 121.8; 119.2; 118.7; 111.6; 111.2; 60.9; 54.2; 52.2; 37.6; 31.1; 30.0; 29.5; 28.9; 26.6; 10.9. Ausbeute (Beispiel 17, polares Diastereomer): 100 mg (25 %), weißer Feststoff
Schmelzpunkt: 70-75°C.
αD20: -7° (c 0.2, MeOH).

1 H NMR (CDCl3): δ 1.00 (2 H, m); 1.63 (1 H, m); 1.82 (1 H, m); 2.08 (6 H, br s); 2.22 (3 H, s), 2.40-2.80 (6 H, m); 3.18 (2 H, m); 4.22 (1 H, t, J = 7 Hz); 6.60 (1 H, s); 6.90 (1 H, s); 7.06 (1 H, t, J = 7.6 Hz); 7.17 (1 H, t, J = 7.6 Hz); 7.26-7.36 (6 H, m); 7.45 (1 H, d, J = 8 Hz); 7.96 (1 H, br s).
13C NMR (CDCl3): δ 164.9; 148.4; 136.1; 135.6; 128.2; 128.0; 127.5; 126.8; 122.7; 122.4; 121.9; 119.3; 118.6; 111.5; 111.0; 62.3; 54.8; 54.6; 38.1; 31.5; 31.3; 31.0; 30.0; 28.9; 10.9.

### Beispiel 18 und Beispiel 19

### Stufe 1:

### (R)-tert.-Butyl-3-(1H-indol-3-yl)-1-oxo-1-(prop-2-ynylamino)propan-2-ylcarbamat

Eine Lösung von (R)-2-(tert.-Butoxycarbonylamino)-3-(1H-indol-3-yl)propionsäure (3.0 g, 9.9 mmol) in THF (20 mL) wurde bei Raumtemperatur portionsweise mit *N,N'-*Carbonyldiimidazol (1.62 g, 10 mmol) versetzt und 2 h bei Raumtemperatur gerührt, bevor Propargylamin (826 mg, 15 mmol) tropfenweise hinzu gegeben wurde. Nach 1 h bei Raumtemperatur wurde mit Diethylether (200 mL) verdünnt, mit gesättigter Natriumhydrogencarbonat-Lösung (2 x 50 mL) gewaschen und mit Natriumsulfat getrocknet. Das Lösemittel wurde i. Vak. entfernt und der Rückstand aus Diethylether umkristallisiert.
Ausbeute: 2.3 g (68 %), weißer Feststoff
Schmelzpunkt: 105-108°C
¹H-NMR (CDCl₃): 1.42 (9 H, s); 2.14 (1 H, t, *J =* 2.4 Hz); 3.15-3.33 (2 H, m); 3.93 (2 H, br s); 4.43 (1 H, br s); 5.10 (1 H, br s); 6.00 (1 H, br s); 7.06 (1 H, d, *J* = 2 Hz); 7.14 (1 H, m); 7.21 (1 H, m); 7.37 (1 H, d, *J* = 8 Hz); 7.65 (1 H, d, *J* = 8 Hz); 8.14 (1 H, br s).

### Stufe 2:

### (R)-tert-Butyl-2-(1H-indol-3-yl)-1-(5-methyloxazol-2-yl)ethylcarbamat

Eine Lösung von (R)-tert.-Butyl-3-(1 H-indol-3-yl)-1-oxo-1-(prop-2-ynylamino)propan-2-ylcarbamat (2.20 g, 6.44 mmol) in Acetonitril (25 mL) wurde bei Raumtemperatur mit Gold(III)-chlorid (190 mg, 0.63 mmol) versetzt und 18 h bei 50°C gerührt. Das Lösemittel wurde i. Vak. entfernt und der Rückstand durch Flashchromatographie (60 g, 15 x 4 cm) mit Ethylacetat / Cyclohexan (1:1) gereinigt.
Ausbeute: 1.30 g (59 %), bräunlicher Feststoff
Schmelzpunkt: 110-112°C
¹H-NMR (CDCl₃): 1.41 (9 H, s); 2.23 (3 H, s); 3.38 (2 H, m); 5.20 (2 H, br s); 6.61 (1 H, s); 6.90 (1 H, s); 7.07 (1 H, t, *J* = 7.6 Hz); 7.16 (1 H, t, *J* = 7.6 Hz); 7.32 (1 H, d, *J* = 8 Hz); 7.43 (1 H, d, *J* = 8 Hz); 8.00 (1 H, br s).

### Stufe 3:

### (R)-2-(1H-Indol-3-yl)-1-(5-methyloxazol-2-yl)ethylamin

Eine Lösung von (*R*)-tert-Butyl-2-(1*H*-indol-3-yl)-1-(5-methyloxazol-2-yl)ethylcarbamat (1.0 g, 2.9 mmol) in Dichlormethan (30 mL) wurde bei 0°C tropfenweise mit Trifluoressigsäure (6 mL, 81 mmol) versetzt und 1 h bei Raumtemperatur gerührt. Anschließend wurden die flüchtigen Bestandteile i. Vak. entfernt und der Rückstand mit gesättigter Natriumhydrogencarbonat-Lösung (20 mL) versetzt. Nach Extraktion mit Dichlormethan (3 × 20 mL) wurde die organische Phase mit Natriumsulfat getrocknet und das Lösemittel i. Vak. entfernt.
Ausbeute: 560 mg (80 %), braunes Öl
¹H-NMR (CDCl₃): 2.09 (2 H, br s); 2.29 (3 H, s); 3.18 (1 H, dd, *J* = 14.4 und 8.4 Hz); 3.42 (1 H, dd, *J* = 14.4 und 5 Hz); 4.38 (1 H, m); 6.61 (1 H, s); 7.05 (1 H, s); 7.10 (1 H, m); 7.19 (1 H, m); 7.35 (1 H, d, *J* = 8 Hz); 7.55 (1 H, d, *J* = 8 Hz); 8.09 (1 H, br s).

### Stufe 4:

### (R)-N⁴-(2-(1H-Indol-3-yl)-1-(5-methyloxazol-2-yl)ethyl)-N',N'-dimethyl-1-phenylcyclohexan-1,4-diamin (Beispiel 18, polares Diastereomer und Beispiel 19, unpolares Diastereomer)

Eine Lösung von (*R*)-2-(1*H*-Indol-3-yl)-1-(5-methyloxazol-2-yl)ethylamin (240 mg, 1.0 mmol) und 4-Dimethylamino-4-phenylcyclo¬hexanon (218 mg, 1.0 mmol) in 1,2-Dichlorethan (15 mL) wurde mit gepulvertem Natriumtriacetoxyborhydrid (317 mg, 1.5 mmol) und Essigsäure (120 mg, 2.0 mmol) versetzt und 18 h bei Raumtemperatur gerührt. Anschließend wurde mit Ethylacetat (80 mL) verdünnt, mit gesättigter Natriumhydrogencarbonat-Lösung (20 mL) gewaschen und mit Natriumsulfat getrocknet. Das Lösemittel wurde i. Vak. entfernt und der Rückstand durch Flashchromatographie (15 g, 12 x 2.5 cm) mit Methanol / Dichlormethan (1:4) gereinigt.
Beispiel 18: (polares Diastereoisomer)
Ausbeute: 120 mg (27 %), weißer Feststoff
Schmelzpunkt: 70-75 °C.
[α]D²⁰: +11° (*c* 0.2, MeOH).
¹H-NMR (CDCl₃): 1.00 (2 H, m); 1.63 (1 H, m); 1.82 (1 H, m); 2.08 (6 H, br s); 2.22 (3 H, s), 2.40-2.80 (6 H, m); 3.18 (2 H, m); 4.22 (1 H, t, J = 7 Hz); 6.60 (1 H, s); 6.90 (1 H, s); 7.06 (1 H, t, *J* = 7.6 Hz); 7.17 (1 H, t, J = 7.6 Hz); 7.26-7.36 (6 H, m); 7.45 (1 H, d, *J* = 8 Hz); 7.96 (1 H, br s).
¹³C-NMR(CDCl₃): 164.9; 148.4; 136.1; 135.6; 128.2; 128.0; 127.5; 126.8; 122.7; 122.4; 121.9; 119.3; 118.6; 111.5; 111.0; 62.3; 54.8; 54.6; 38.1; 31.5; 31.3; 31.0; 30.0; 28.9; 10.9. LC-MS (Methode 8): m/z: M+H]⁺ = 443.3, Rₜ = 1.5 min.
Beispiel 19: (unpolares Diastereoisomer)
Ausbeute: 170 mg (38 %), weißer Feststoff
Schmelzpunkt: 65-70°C.
[α]D²⁰: +14° (c 0.2, MeOH).
¹H-NMR (CDCl₃): 1.40-1.60 (6 H, s); 1.90-2.20 (8 H, m); 2.25 (3 H, s); 2.35-2.47 (2 H, m); 3.22-3.33 (2 H, m); 4.27 (1 H, m); 6.60 (1 H, s); 7.10 (1 H, t, *J* = 7.6 Hz); 7.18 (1 H, t, *J* = 7.6 Hz); 7.26-7.37 (7 H, m); 7.57 (1 H, d, *J* = 8 Hz); 8.20 (1 H, br s).
¹³C-NMR (CDCl₃): 165.1; 148.5; 137.7; 136.2; 127.6; 127.5; 127.3; 126.8; 123.3; 122.3; 121.8; 119.2; 118.7; 111.6; 111.2; 60.9; 54.2; 52.2; 37.6; 31.1; 30.0; 29.5; 28.9; 26.6; 10.9. LC-MS (Methode 8): m/z: [M+H]⁺ = 443.3, Rₜ = 2.35 min.

### Beispiel 20 und Beispiel 21

### Stufe 1:

### (S)-tert-Butyl-1-amino-3-(1H-indol-3-yl)-1-thioxopropan-2-ylcarbamat

Eine Lösung von (S)-tert-Butyl-1-amino-3-(1H-indol-3-yl)-1-oxopropan-2-ylcarbamat (500 mg, 1.6 mmol) in wasserfreiem 1,2-Dimethoxyethan (10 mL) wurde mit Natriumhydrogencarbonat (520 mg, 6.2 mmol) und Diphosphorpentasulfid (730 mg, 3.2 mmol) portionsweise versetzt und über Nacht bei Raumtemperatur gerührt. Das Reaktionsgemisch wurde anschließend i. Vak. eingeengt, der Rückstand in Ethylacetat (30 mL) aufgenommen und mit Wasser und gesättigter Natriumhydrogencarbonat-Lösung (je 3 × 20 mL) gewaschen. Die organische Phase wurde mit Natriumsulfat getrocknet und i. Vak. eingeengt. Das Rohprodukt (600 mg) wurde in Diethylether / Cyclohexan (je 10 mL) aufgenommen und wieder eingeengt. Dabei fiel ein weißer Feststoff aus.
Ausbeute: 510 mg (100 %), weißer Feststoff
Schmelzpunkt: 57-62 °C
¹H-NMR (DMSO-d₆): 1.30 (s, 9H); 2.88-3.00 (m, 1H); 3.10-3.20 (m, 1H); 4.40-4.56 (m, 1H); 6.72 (d, 1H, J = 8.4 Hz); 6.94-7.10 (m, 2H); 7.17 (s, 1H); 7.32 (d, 1H, J = 8.3 Hz); 7.65 (d, 1H, J = 7.4 Hz); 9.16 (s, 1H); 9.61 (s, 1H); 10.80 (s, 1H).

### Stufe 2:

### tert-Butyl-(1S)-1-(4-hydroxy-4-methyl-4,5-dihydrothiazol-2-yl)-2-(1H-indol-3-yl)ethyl]carbamat

Eine Lösung von (*S*)-tert-Butyl-1-amino-3-(1H-indol-3-yl)-1-thioxopropan-2-ylcarbamat (400 mg, 1.2 mmol) in wasserfreiem 1,2-Dimethoxyethan (20 mL) wurde mit gepulvertem Kaliumhydrogencarbonat (600 mg, 6 mmol) und Chloraceton (556 mg, 447 µL, 6 mmol) versetzt und 8 h bei 70 °C und über das Wochenende bei 45 °C gerührt. Das Reaktionsgemisch wurde anschließend filtriert und das Filtrat i. Vak. eingeengt.
Ausbeute: 500 mg (100 %), bernsteinfarbenes Öl

### Stufe 3:

### (S)-tert.-Butyl-2-(1H-indol-3-yl)-1-(4-methylthiazol-2-yl)ethylcarbamat

Eine Lösung von tert-Butyl-(1*S*)-1-(4-hydroxy-4-methyl-4,5-dihydrothiazol-2-yl)-2-(1*H*-indol-3-yl)ethyl]carbamat(280 mg, 0.74 mmol) in Toluol (20 mL) wurde mit Natriumsulfat (1.00 g) versetzt und 1 h unter Rückfluss gerührt. Das Gemisch wurde anschließend filtriert, das Filtrat i. Vak. eingeengt und der Rückstand (260 mg) durch Flashchromatographie (18 g, 20 x 2.0 cm) mit Ethylacetat / Cyclohexan (1:2) gereinigt.
Ausbeute: 185 mg (70 %), bräunliches Öl
¹H-NMR (DMSO-d₆): 1.31 (s, 9H); 2.36 (d, 3H, J = 1 Hz); 3.05-3.17 (m, 1 H); 3.37-3.42 (m, 1 H); 4.92-5.02 (m, 1 H); 6.95-7.15 (m, 4H); 7.34 (d, 1 H, J = 8.0 Hz); 7.54 (d, 1 H, J = 7.8 Hz); 7.63 (d, 1 H, J = 8.3 Hz); 10.82 (s, 1H).

### Stufe 4:

### (S)-2-(1H-Indol-3-yl)-1-(4-methylthiazol-2-yl)ethylamin

Eine Lösung von (*S*)-tert.-Butyl-2-(1*H*-indol-3-yl)-1-(4-methylthiazol-2-yl)ethylcarbamat (2.13 g, 5.95 mmol) in wasserfreiem Dichlormethan (15 mL) wurde mit Trifluoressigsäure (5 mL) versetzt und 1 h bei Raumtemperatur gerührt. Die Lösung wurde anschließend i. Vak. eingeengt, der Rückstand in Dichlormethan (100 mL) aufgenommen und mit 2 M Kaliumcarbonatlösung (3 × 20 mL) gewaschen. Die organische Phase wurde mit Natriumsulfat getrocknet und i. Vak. eingeengt.
Ausbeute: 1.49 g (97 %), braunes Öl
¹H-NMR (DMSO-d₆): 2.07-2.17 (br s, 2H); 2.34 und 2.35 (d, 3H, J = 0.8 Hz); 2.91 (dd, 1 H, J = 14.2, 8.5 Hz); 3.28 (dd, 1 H, J = 14.2, 4.5 Hz); 4.35 (dd, 1 H, J = 8.5, 4.4 Hz); 6.93-7.00 (m, 1H); 7.02-7.09 (m, 2H); 7.15 (d, 1H, J = 2.2 Hz); 7.33 (d, 1H, J = 8.0 Hz); 7.49 (d, 1H, J = 7.8 Hz); 10.85 (s, 1H).
¹³C-NMR (DMSO-d₆): 17.0; 29.3; 34.4; 54.0; 10.4; 111.3; 113.3; 118.3; 120.8; 123.8; 127.4; 136.2; 151.5; 177.7.

### Stufe 5:

### (S)-N⁴-(2-(1H-Indol-3-yl)-1-(4-methylthiazol-2-yl)ethyl)-N',N'-dimethyl-1-penylcyclohexan-1,4-diamin (Beispiel 20, unpolares Diastereomer, Beispiel 21, polares Diastereomer)

Eine Lösung von (*S*)-2-(1*H*-Indol-3-yl)-1-(4-methylthiazol-2-yl)ethylamin (500 mg, 1.94 mmol) und 4-Dimethylamino-4-phenylcyclohexanon (421 mg, 1.94 mmol) in wasserfreiem Tetrahydrofuran (40 mL) wurde mit Natriumsulfat (1.00 g) versetzt und 3 h bei Raumtemperatur gerührt. Nach Zugabe von Essigsäure (291 mg, 287 µL, 4.85 mmol) wurde Natriumtriacetoxyborhydrid (574 mg, 2.71 mmol) zugegeben und die Mischung über Nacht bei Raumtemperatur gerührt. Das Gemisch wurde anschließend filtriert, das Filtrat i. Vak. eingeengt, der Rückstand mit 1 *M* Kaliumcarbonatlösung (50 mL) versetzt und mit Ethylacetat (3 × 30 mL) extrahiert. Die vereinigten organischen Phasen wurden mit Natriumsulfat getrocknet und i. Vak. eingeengt. Das Rohprodukt (750 mg) wurde durch Flashchromatographie (100 g, 20 x 4.0 cm) mit Ethylacetat / Methanol [97:3→9:1 und jeweils 1 % NH₃ (33 % in Wasser)] gereinigt.
Beispiel 20: (unpolares Diastereoisomer)
Ausbeute: 400 mg (45 %), weißer Feststoff
Schmelzpunkt: 70-72 °C
¹H-NMR (DMSO-d₆): 1.20-1.48 (m, 6H); 1.82 (s, 6H); 2.10-2.24 (m, 1H); 2.28-2.38 (m, 2H); 2.33 (d, 3H, J = 1.0 Hz); 2.96 (dd, 1H, J = 14.2, 8.5 Hz); 3.21 (dd, 1H, J = 14.3, 4.5 Hz); 4.33 (dd, 1 H, J = 8.4, 4.7 Hz), 6.99 (ddd, 1 H, J = 8.0, 7.1, 1.1 Hz); 7.04-7.11 (m, 2H); 7.14-7.41 (m, 8H); 7.50 (d, 1H, J = 7.5 Hz); 10.88 (s, 1H).
¹³C-NMR (DMSO-d₆): 17.0; 27.0; 28.7; 30.2; 30.5; 33.4; 37.4; 53.6; 57.9; 58.6; 110.0; 111.4; 113.5; 118.0; 120.9; 124.0; 126.1; 126.7; 127.2; 127.3; 136.2; 138.8; 151.6; 177.9.
LC-MS (Methode 7): m/z: [M+H]⁺ = 459.3, Rₜ = 2.7 min.
Drehwert: [α][_{D}²⁴ = +1.4° (*c* 1.0, MeOH).
Beispiel 21: (polares Diastereoisomer)
Ausbeute: 186 mg (21 %), gelbliches Öl
¹H-NMR (CDCl₃): 0.66-0.77 (m, 1 H); 0.85-1.07 (m, 1 H); 1.43-1.76 (m, 4H); 1.97 (s, 6H); 2.23-2.33 (m, 1 H); 2.45 (d, 3H, J = 0.9 Hz); 2.39-2.53 (m, 2H); 2.98 (dd, 1 H, J = 14.5, 8.8 Hz); 3.30 (dd, 1H, J = 14.5, 4.8 Hz), 4,43 (dd, 1H, J = 8.8, 4.8 Hz); 6.75 (d, 1H, J = 1.0 Hz); 6.91 (d, 1 H, J = 2.3 Hz); 7.07 (ddd, 1 H, J = 8.0, 7.1, 1.0 Hz); 7.13-7.25 (m, 5H); 7.28-7.34 (m, 3H); 7.58 (d, 1H, J = 7.9 Hz); 7.99 (s, 1H).
¹³C-NMR (CDCl₃): 17.2; 28.7; 30.4; 31.3; 31.6, 34.0; 38.2; 55.0; 58.5, 61.5; 111.1; 111.7; 113.0; 118.8; 119.3; 122.0; 122.8; 126.4; 127.4; 127.7; 127.9; 136.2; 136.4; 152.3; 177.7. LC-MS (Methode 7): m/z: [M+H]⁺ = 459.3, Rₜ = 1.9 min.
Drehwert: [α]_{D}²⁴ = -0.15° (*c* 1.0, MeOH).

### Beispiel 22 und Beispiel 23

### Stufe 1:

### (R)-tert.-Butyl-1-amino-3-(1H-indol-3-yl)-1-oxopropan-2-ylcarbamat

Eine Lösung von (R)-2-(tert.-Butoxycarbonylamino)-3-(1 H-indol-3-yl)propansäure (7.00 g, 23 mmol) und 1,1'-Carbonyldiimidazol (4.44 g, 27.3 mmol) in wasserfreiem Tetrahydrofuran (200 mL) wurde 2 h bei Raumtemperatur gerührt. Diese Lösung wurde anschließend zu einer Lösung von 33 % wässrigem Ammoniak (14 mL, 230 mmol) in Tetrahydrofuran (50 mL) getropft und 20 h bei Raumtemperatur gerührt. Die Reaktionslösung wurde i. Vak. eingeengt und der Rückstand in Wasser (200 mL) aufgenommen. Nach 30 min fiel das Produkt als weißer Feststoff, der abfiltriert, mit Wasser gewaschen und über Kaliumhydroxid im Exsikkator getrocknet wurde, aus.
Ausbeute: 6.90 g (99 %), weißer Feststoff
Schmelzpunkt: 113-118°C
¹H-NMR(DMSO-d₆):1.31 (s, 9H); 2.89 (dd, 1 H, J = 14.5, 9.1 Hz); 3.07 (dd, 1H, J = 14.5, 4.7 Hz); 4.09-4.19 (m, 1 H); 6.62 (d, 1 H, J = 8.2 Hz); 6.93-7.14 (m, 4H); 7.29-7.38 (m, 2H); 7.60 (d, 1H, J=7.7 Hz); 10.77 (s, 1H).

### Stufe 2:

### (R)-tert.-Butyl-1-amino-3-(1H-Indol-3-yl)-1-thioxopropan-2-ylcarbamat

Eine Lösung von (*R*)-tert.-Butyl-1-amino-3-(1*H*-indol-3-yl)-1-oxopropan-2-ylcarbamat (6.80 g, 22.4 mmol) in wasserfreiem Dimethoxyethan (100 mL) wurde mit Natriumhydrogencarbonat (7.14 g, 85.1 mmol) und Diphosphorpentasulfid (10.2 g, 44.8 mmol) portionsweise versetzt und über Nacht bei Raumtemperatur gerührt. Das Reaktionsgemisch wurde i. Vak. eingeengt, der Rückstand in Ethylacetat (100 mL) aufgenommen und mit Wasser und gesättigter Natriumhydrogencarbonat-Lösung (je 3 × 50 mL) gewaschen. Die organische Phase wurde mit Natriumsulfat getrocknet und i. Vak. eingeengt. Das Rohprodukt wurde in Diethylether / Cyclohexan (je 50 mL) aufgenommen und wieder eingeengt. Dabei fiel das Produkt als weißer Feststoff, der abfiltriert wurde, aus.
Ausbeute: 6.49 g (91 %), weißer Feststoff
Schmelzpunkt: 55-65 °C
¹H-NMR (DMSO-d₆): 1.30 (s, 9H); 2.95 (dd, 1H, J = 9.3 Hz); 3.17 (dd, 1H, J = 14.3, 4.3 Hz); 4.44-4.54 (m, 1H); 6.70 (d, 1H, J = 8.4 Hz); 6.95-7.09 (m, 2H); 7.16 (s, 1H); 7.33 (d, 1H, J = 8.0 Hz); 7.65 (d, 1H, J = 7.7 Hz); 9.15 (s, 1H); 9.61 (s, 1H); 10.80 (s, 1H).

### Stufe 3:

### tert. -Butyl-(1R)-1-(4-hydroxy-4-methyl-4,5-dihydrothiazol-2-yl)-2-(1H-indol-3-yl)ethylcarbamat

Eine Lösung von (*R*)-tert.-Butyl-1-amino-3-(1*H*-Indol-3-yl)-1-thioxopropan-2-ylcarbamat (6.49 g, 20.3 mmol) in wasserfreiem Dimethoxyethan (100 mL) wurde mit gepulvertem Kaliumhydrogencarbonat (10.1 g, 101 mmol) und Chloraceton (9.69 g, 8.4 mL, 101 mmol) versetzt und 8 h bei 70 °C und über das Wochenende bei 45 °C gerührt. Anschließend wurde das Reaktionsgemisch filtriert und das Filtrat i. Vak. eingeengt.
Ausbeute: 7.12 g (93 %), bernsteinfarbenes Öl

### Stufe 4:

### (R)-tert.-Butyl-2-(1H-indol-3-yl)-1-(4-methylthiazol-2-yl)ethylcarbamat

Eine Lösung von tert.-Butyl-(1*R*)-1-(4-hydroxy-4-methyl-4,5-dihydrothiazol-2-yl)-2-(1*H*-indol-3-yl)ethylcarbamat (7.12 g, 18.9 mmol) in Toluol (150 mL) wurde mit Natriumsulfat (9.00 g) versetzt und 1 h unter Rückfluss gerührt. Das Gemisch wurde filtriert, das Filtrat i. Vak. eingeengt und der Rückstand (6.18 g) durch Flashchromatographie (400 g, 20 × 7.5 cm) mit Ethylacetat / Cyclohexan (1:2) gereinigt.
Ausbeute: 4.97 g (74 %), bräunliches Öl ¹H-NMR (DMSO-d₆): 1.31 (s, 9H); 2.36 (s, 3H); 3.03-3.20 (m, 1 H); 3.35-3.45 (m, 1 H); 4.97 (dt, 1H, J = 10.0, 4.6 Hz); 6.95-7.14 (m, 4H); 7.33 (d, 1H, J = 8.1 Hz); 7.53 (d, 1H, J = 7.7 Hz), 7.60 (d, 1 H, J = 8.4 Hz); 10.80 (s, 1 H).

### Stufe 5:

### (R)-2-(1H-Indol-3-yl)-1-(4-methylthiazol-2-yl)ethylamin

Eine Lösung von (*R*)-tert.-Butyl-2-(1*H*-indol-3-yl)-1-(4-methylthiazol-2-yl)ethylcarbamat (4.97 g, 13.9 mmol) in wasserfreiem Dichlormethan (40 mL) wurde mit Trifluoressigsäure (7 mL) versetzt und 24 h bei Raumtemperatur gerührt. Die Lösung wurde anschließend i. Vak. eingeengt, der Rückstand in Dichlormethan (100 mL) aufgenommen und mit 2 M Kaliumcarbonatlösung (3 × 30 mL) gewaschen. Die organische Phase wurde mit Natriumsulfat getrocknet und i. Vak. eingeengt.
Ausbeute: 3.47 g (97 %), braunes Öl
¹H-NMR (DMSO-d₆): 2.00-2.18 (br s, 2H); 2.34 (d, 3H, J = 1Hz); 2.92 (dd, 1H, J = 14.3, 8.4 Hz); 3.28 (dd, 1H, J = 14.4, 4.7 Hz); 4.36 (dd, 1H, J = 8.4, 4.4 Hz); 6.93-7.09 (m, 3H); 7.15 (d, 1H, J = 2.3 Hz); 7.31-7.36 (m, 1H); 7.50 (d, 1H, J = 7.8 Hz); 10.85 (s, 1H).

### Stufe 6:

### (R)-N⁴-(2-(1H-Indol-3-yl)-1-(4-methylthiazol-2-yl)ethyl)-N¹,N¹-dimethyl-1-penylcyclohexan-1,4-diamin (Beispiel 22, unpolares Diastereomer und Beispiel 23, polares Diastereomer)

Eine Lösung von (*R*)-2-(1*H*-Indol-3-yl)-1-(4-methylthiazol-2-yl)ethylamin (500 mg, 1.94 mmol) und 4-Dimethylamino-4-phenylcyclohexanon (421 mg, 1.94 mmol) in wasserfreiem Tetrahydrofuran (40 mL) wurde mit Natriumsulfat (1.00 g) versetzt und 3 h bei Raumtemperatur gerührt. Nach Zugabe von Essigsäure (291 mg, 287 µL, 4.85 mmol) wurde Natriumtriacetoxyborhydrid (574 mg, 2.71 mmol) zugegeben und die Mischung über Nacht bei Raumtemperatur gerührt. Das Gemisch wurde filtriert, das Filtrat i. Vak. eingeengt, der Rückstand mit 1 M Kaliumcarbonatlösung (50 mL) versetzt und mit Ethylacetat (3 × 30 mL) extrahiert. Die vereinigten organischen Phasen wurden mit Natriumsulfat getrocknet und i. Vak. eingeengt. Das Rohprodukt (876 mg) wurde durch Flashchromatographie (100 g, 20 × 4.0 cm) mit Ethylacetat / Methanol (95:5→9:1) und jeweils 1 % Ammoniak (33 % in Wasser) gereinigt. Das unpolare Diastereoisomer (450 mg) wurde nochmals durch Flashchromatographie (38 g, 20 × 2.5 cm) mit Dichlormethan / Methanol (300:9) und 1 % Ammoniak (33 % in Wasser) gereinigt. Das polare Diastereoisomer (262 mg) wurde ebenfalls erneut durch Flashchromatographie (18 g, 20 × 2.0 cm) mit Dichlormethan / Methanol (24:1) und 1 % Ammoniak (33 % in Wasser) gereinigt.
Beispiel 22: (unpolares Diastereoisomer)
Ausbeute: 252 mg (28 %), weißer Feststoff
Schmelzpunkt: 75-77 °C
¹H-NMR (DMSO-d₆): 1.12-1.47 (m, 6H); 1.81 (s, 6H); 2.10-2.24 (m, 1 H); 2.27-2.37 (m, 2H); 2.33 (d, 3H, J = 0.9 Hz); 2.96 (dd, 1 H, J = 14.3, 8.5 Hz); 3.21 (dd, 1H, J = 14.2, 4.7 Hz); 4.33 (dd, 1 H, J = 8.4, 4.7 Hz); 6.99 (ddd, 1 H, J = 7.9, 7.1, 1.0 Hz); 7.04-7.11 (m, 2H); 7.14-7.37 (m, 7H); 7.50 (d, 1 H, J = 7.6 Hz); 10.87 (s, 1 H). 1 H konnte nicht identifiziert werden. ¹³C-NMR (DMSO-d₆): 17.0; 27.1; 28.7; 30.2; 30.5; 33.5; 37.4, 53.6; 57.9; 58.7; 110.1; 111.4; 113.6; 118.1; 118.4; 121.0; 124.0; 126.1; 126.7; 127.2; 127.3; 136.2; 138.8; 151.6; 177.9. LC-MS (Methode 7): m/z: [M+H]⁺ = 459.3, Rₜ 2.0 min.
Drehwert: [α]_{D}²⁴ = +0.55° (*c* 1.0, CHCl₃).
Beispiel 23: (polares Diastereoisomer)
Ausbeute: 108 mg (12 %), weißer Feststoff
Schmelzpunkt: 71-74 °C
¹H-NMR (CDCl₃): 0.68-0.84 (m, 1 H); 0.98-1.14 (m, 1 H); 1.39-1.84 (m, 5H); 1.97 (s, 6H); 2.26-2.40 (m, 1 H); 2.42-2.58 (m, 2H); 2.44 (s, 3H); 3.03 (dd, 1 H, J = 14.4, 8.8 Hz); 3.35 (dd, 1H, J = 14.4, 4.7 Hz); 4.49 (dd, 1 H, J = 8.8, 4.7 Hz); 6.78-6.81 (m, 1 H); 6.91 (d, 1 H, J = 2.1 Hz); 7.05-7.40 (m, 8H); 7.61 (d, 1 H, J = 7.8 Hz); 8.48 (s, 1H).
¹³C-NMR (DMSO-d₆): 17.2; 28.7; 30.4; 31.3; 31.6; 34.0; 38.2; 55.0; 58.5; 61.3; 111.0; 111.5; 113.0; 118.8; 119.3; 121.9; 122.9; 126.3; 127.4; 127.7; 127.8; 136.2; 136.6; 152.2; 177.7. LC-MS (Methode 7): m/z: [M+H]⁺ = 459.3, Rₜ 2.0 min.
Drehwert: [α]_{D}²⁴ = +1.97 ° (*c* 1.0, CHCl₃).
Beispiel 24 und Beispiel 25

### (Referenzbeispiele) Stufe 1:

### 2-(1H-Indol-3-yl)-1-phenylethanon

NaCN (1,4 g, 29 mmol) wurde unter Argon in absolutem Dimethylformamid (10 ml) vorgelegt. Bei einer Badtemperatur von 35°C wurde Benzaldehyd (3 ml, 29 mmol), gelöst in absolutem Dimethylformamid (10 ml), innerhalb von 1.5 h zugetropft und noch weitere 0,5 h bei dieser Temperatur gerührt. Zu dieser Reaktionslösung wurde Gramin (10,1 g, 58 mmol), gelöst in absolutem Dimethylformamid (30 ml), innerhalb von 2 h bei 70 °C Innentemperatur zugetropft. Es wurde noch 1 h bei dieser Temperatur gerührt. - Zur Aufarbeitung wurde der Kolbeninhalt in Wasser (150 ml) gegeben. Die wäßrige Phase wurde mit 2N HCl angesäuert, um unumgesetztes Gramin als wasserlösliches Hydrochlorid abzutrennen. Danach wurde die wäßrige Phase mit Chloroform (3 × 50 ml) extrahiert. Die vereinigten Extrakte wurden mit gesättigter NaHCO₃-Lösung (50 ml) gewaschen und über Na₂SO₄ getrocknet. Anschließend wurden die flüchtigen Bestandteile vollständig im Vakuum entfernt. Der nach Abdestillieren des Lösungsmittels erhaltene Rückstand (9 g) wurde säulenchromatographisch gereinigt [Kieselgel 60 (300 g); Cyclohexan/Ethylacetat 6 : 1 (1500 ml)].Ausbeute: 1 g (14 %) gelbes Öl.

### Stufe 2:

### 2-(1H-Indol-3-yl)-1-phenylethanon-oxim

Das 2-(1*H*-Indol-3-yl)-1-phenylethanon (0,97 g, 4,12 mmol), gelöst in absolutem Ethanol (20 ml), wurde zu einer Lösung von Hydroxylamin-hydrochlorid (0,97 g, 13,95 mmol) und Kaliumacetat (1,54 g, 15,74 mmol) in absolutem Ethanol (20 ml) gegeben. Diese Reaktionsmischung wurde bei Raumtemperatur 24 h gerührt. Anschließend wurde der Ansatz 3 h unter Rückfluß gekocht. Danach wurde der Ansatz bei 70 °C in Wasser (20 ml) gelöst. Anschließend wurde der Ansatz 5 h bei 4 °C stehengelassen. Da kein Feststoff ausfiel, wurden die flüchtigen Bestandteile vollständig im Vakuum entfernt. Der Rückstand wurde mit Wasser (10 ml) versetzt und der pH-Wert der Lösung mit 5N NatriumhydroxidLösung auf pH 11 eingestellt. Dann wurde mit Ethylacetat (5 x 30 ml) extrahiert. Die vereinigten organischen Phasen wurden mit Natriumsulfat getrocknet, filtriert und im Vakuum von allen flüchtigen Bestandteilen befreit. Ausbeute: 90 % (990 mg) gelbbraunes Öl

### Stufe 3:

### 2-(1H-Indol-3-yl)-1-phenylethylamin

Das 2-(1*H*-lndol-3-yl)-1-phenylethanon-oxim (0,2 g, 0,75 mmol), gelöst in Ethanol (10 ml), wurde in der Siedehitze portionsweise mit Natriumstückchen (500 mg, 21 mmol) versetzt. Diese Reaktionsmischung wurde bis zur vollständigen Auflösung des Metalls unter Rückfluß gerührt. Nach Abkühlung im Eisbad wurde zu der Reaktionslösung vorsichtig Wasser (15 ml) gegeben. Anschließend wurden die flüchtigen Bestandteile im Vakuum entfernt. Der wäßrige Rückstand wurde mit Ethylacetat (3 x 30 ml) extrahiert. Die vereinigten organischen Phasen wurden mit Natriumsulfat getrocknet, filtriert und im Vakuum von allen flüchtigen Bestandteilen befreit. Der nach Abdestillieren des Lösungsmittels erhaltene Rückstand (163 mg) wurde säulenchromatographisch gereinigt [Kieselgel 60 (30 g); Methanol (500 ml)]. Ausbeute: 45 mg (25 %), hellgelbes Öl.

Da eine Vergrößerung des Ansatzes zu Ausbeuteverlusten führte, wurde dieser Ansatz dreimal wiederholt.

### Stufe 4:

### N⁴-(2-(1H-Indol-3-yl)-1-phenylethyl)-N¹,N¹-dimethyl-1-phenylcyclohexan-1,4-diamin (Beispiel 24, unpolares Diastereoisomer und Beispiel 25, polares Diastereoisomer)

Unter Argon wurden das 2-(1*H*-Indol-3-yl)-1-phenylethylamin (135 mg, 0,57 mmol) und das 4-Dimethylamino-4-phenylcyclohexanon (124 mg, 0,57 mmol) in Tetrahydrofuran (10 ml) und 1,2-Dichlorethan (5 ml) gelöst. Zur klaren Lösung wurde Eisessig (0,035 ml, 0,57 mmol) hinzugefügt. Nach einer Reaktionszeit von 15 min wurde die Reaktionsmischung mit NaBH(OAc)₃ (180 mg, 0,8 mmol) versetzt und 2 d bei Raumtemperatur gerührt. Zur Aufarbeitung des Ansatzes wurde die Mischung mit gesättigter NaHCO₃-Lösung (40 ml) versetzt und 15 min gerührt. Die wäßrige Phase wurde mit Dichlormethan (2 x 30 ml) extrahiert. Die vereinigten organischen Phasen wurden nach dem Trocknen über Na₂SO₄ eingeengt, wobei ein hellbraunes Öl erhalten wurde. Die chromatographische Trennung des Substanzgemisches an Kieselgel 60 (50 g) erfolgte mit Methanol (600 ml). Das unpolarere Amin wurde in einer Ausbeute von 67 % (168 mg, ) als beigefarbene Verbindung erhalten. Ein Schmelzpunkt konnte nicht bestimmt werden. Das polarere Amin wurde in einer Ausbeute von 30 % (75 mg,) als beigefarbene Verbindung erhalten. Ein Schmelzpunkt konnte nicht bestimmt werden.
Beispiel 24: (unpolares Diastereomer) ¹³C-NMR (101 MHz, DMSO-D₆) δ ppm: 27.0, 29.4, 30.5, 30.9, 35.4, 37.8, 52.4, 59.6, 60.0, 111.1, 113.2, 118.9, 119.2, 121.9, 122.8, 126.2, 126.4, 126.7, 127.1, 127.2, 127.3, 127.6, 128.2, 136.4, 139.0, 145.3
Beispiel 25: (polares Diastereomer) ¹³C-NMR (101 MHz, DMSO-D₆) δ ppm: 29.1, 30.8, 31.6, 318, 35.2, 38.354.4, 60.4, 61.4, 111.0, 113.0, 118.8, 119.2, 121.9, 122.5, 126.3, 126.7, 127.2, 127.6, 127.7, 128.0, 128.2, 136.2, 136.9, 145.2

### Stufe 5:

### N⁴-(2-(1H-Indol-3-yl)-1-phenylethyl)-N¹,N¹-dimethyl-1-phenylcyclohexan-1,4-diamin Dihydrochlorid (Beispiel 24,unpolares Diastereoisomer und Beispiel 25, polares Diastereoisomer)

### Beispiel 24: (unpolares Diastereomer)

Zur Herstellung des Hydrochlorids wurde das *N*⁴-(2-(1*H*-Indol-3-yl)-1-phenylethyl)-*N¹,N¹*-dimethyl-1-phenylcyclohexan-1,4-diamin (unpolareres Amin) (160 mg, 0,36 mmol) in Ethylmethylketon (15 ml) gelöst, mit Trimethylchlorsilan (115 µl, 0,9 mmol) versetzt und 3 h bei Raumtemperatur gerührt. Das ausgefallene farblose Hydrochloridwurde abgesaugt und getrocknet. Das Hydrochlorid wurde in einer Ausbeute von 100 mg (53 %) mit einem Schmelzpunkt von 198-202 °C erhalten.

### Beispiel 25: (polares Diastereomer)

Zur Herstellung des Hydrochlorids wurde das *N*⁴-(2-(1*H*-Indol-3-yl)-1-phenylethyl)-*N¹,N¹*-dimethyl-1-phenylcyclohexan-1,4-diamin (polareres Amin) (70 mg, 0,16 mmol) in Ethylmethylketon (7 ml) gelöst, mit Trimethylchlorsilan (51 µl, 0,4 mmol) versetzt und 3 h bei Raumtemperatur gerührt. Das ausgefallene farblose Hydrochlorid wurde abgesaugt und getrocknet. Das Hydrochlorid wurde in einer Ausbeute von 70 mg (85 %) mit einem Schmelzpunkt von 208-217 °C erhalten.

### Beispiel 26, Beispiel 27, Beispiel 28 und Beispiel 29

### Stufe 1:

### 2-(4-Dimethylamino-4-phenylcyclohexylamino)-3-(1H-indol-3-yl)propionamid (unpolares Diastereomer und polares Diastereomer)

DL-Tryptophanamid-Hydrochlorid (5.00 g, 20.8 mmol) wurde in 0.5 M Natronlauge (20 mL) aufgenommen, die Lösung anschließend mit 4 M Natronlauge auf pH 9 gestellt und danach mit Ethylacetat (5 x 30 mL) extrahiert. Die vereinigten organischen Phasen wurden mit Natriumsulfat getrocknet und i. Vak. eingeengt. Das so gewonnene DL-Tryptophanamid (3.87 g, 19.04 mmol) wurde in Tetrahydrofuran (105 mL) und 1,2-Dichlorethan (60 mL) gelöst und mit 4-Dimethylamino-4-phenylcyclohexanon (4.13 g, 19.0 mmol) versetzt. Die Lösung wurde mit Eisessig (1.09 mL, 19.04 mmol) und Natriumsulfat (9.52 g) versetzt und 15 min gerührt. Anschließend wurde Natriumtriacetoxyborhydrid (5.71 g, 26.6 mmol) zugesetzt, das Reaktionsgemisch über Nacht bei Raumtemperatur gerührt, dann mit gesättigter Natriumhydrogencarbonat-Lösung (225 mL) versetzt und 15 min gerührt. Nach Phasentrennung wurde die wässrige Phase mit Dichlormethan (2 x 40 mL) extrahiert. Die vereinigten organischen Phasen wurden mit Natriumsulfat getrocknet und i. Vak. eingeengt. Das Rohprodukt (7.6 g) wurde durch Flashchromatographie (500 g, 20 x 7.6 cm) mit Ethylacetat / Methanol (1:1) gereinigt.
Unpolares Diastereoisomer
Ausbeute: 3.88 g (50 %), weißer, amorpher Feststoff
¹H-NMR (DMSO-d₆): 1.22-1.36 (m, 2H); 1.36-1.56 (m, 4H); 1.85 (s, 6H); 2.24-2.46 (m, 3H); 2.81 (dd, 1H, J = 14.2, 7.9 Hz); 3.01 (dd, 1H, J = 14.3, 5.4 Hz); 3.39 (t, 1H, J = 6.5 Hz); 6.94-7.01 (m, 2H); 7.06 (ddd, 1H, J = 8.1, 7.0, 1.1 Hz); 7.16-7.38 (m, 9H); 7.56 (d, 1H, J = 7.8 Hz); 10.83 (s, 1H).
Polares Diastereoisomer
Ausbeute: 1.64 g (21 %), weißer, amorpher Feststoff ¹H-NMR (DMSO-d₆): 0.75 (m, 1 H); 0.92 (m, 1 H); 1.35-1.72 (m, 5H); 1.86 (s, 6H); 2.30-2.48 (m, 3H); 2.71 (dd, 1H, J = 14.3, 7.3 Hz); 2.91 (dd, 1H, J = 14.3, 5.9 Hz); 3.28 (t, 1H, J = 6.6 Hz); 6.87-6.96 (m, 2H); 7.03 (dt, 1H, J = 7.5, 1.0 Hz); 7.07 (d, 1H, J = 7.2 Hz); 7.18-7.39 (m, 7H); 7.49 (d, 1 H, J = 7.8 Hz); 10.74 (s, 1 H).

### Stufe 2:

### N-[1-Cyano-2-(1H-indol-3-yl)ethyl]-N-(4-dimethylamino-4-phenylcyclohexyl)-2,2,2-trifluoracetamid(unpolares Diastereomer)

Eine Lösung von 2-(4-Dimethylamino-4-phenylcyclohexylamino)-3-(1*H*-indol-3-yl)propionamid (unpolares Diastereomer) (500 mg, 1.23 mmol) in wasserfreiem Tetrahydrofuran (30 mL) und Triethylamin (2 mL) wurde auf -15 °C gekühlt und mit Trifluoressigsäureanhydrid (777 mg, 514 µL, 3.69 mmol) versetzt. Das Gemisch wurde 3 h bei -15 °C gerührt und dann i. Vak. eingeengt. Der Rückstand wurde mit 5 % Natriumhydrogencarbonat-Lösung (30 mL) versetzt und die wässrige Suspension mit Dichlormethan (3 × 20 mL) extrahiert. Die vereinigten organischen Phasen wurden mit Natriumhydrogencarbonat-Lösung (3 x 20 mL), mit Natriumsulfat getrocknet und i. Vak. eingeengt. Das Rohprodukt (539 mg) wurde durch Flashchromatographie (85 g, 20 x 3.7 cm) mit Dichlormethan / Methanol (95:5) gereinigt.
Ausbeute: 382 mg (64 %), gelber Feststoff
Schmelzpunkt: 230 °C
¹H-NMR(DMSO-d₆): 0.94-1-06 (m, 1H); 1.23-1.45 (m, 2H); 1.55-1.75 (m, 2H); 1.95-2.10 (m, 2H); 1.91 (s, 6H); 2.75 (br d, 1 H, J = 11.7 Hz); 3.42 (dd, 1 H, J = 7.2 und 14.2 Hz); 3.66 ( dd, 1H, J = 8.4 und 14.2 Hz); 3.70-3.85 (m, 1 H); 4.70 (t, 1 H, J = 7.8 Hz); 7.04 (t, 1H, J = 7.4 Hz); 7.12 (t, 1H, J = 7.3 Hz); 7.21-7.38 (m, 6H); 7.41 (d, 1H, J = 8.0 Hz); 7.63 (d, 1H, J = 7.7 Hz); 11.07 (s, 1H).
¹³C-NMR (DMSO-d₆): 24.2; 24.9; 25.1; 25.9; 31.2; 37.2; 37.5; 45.6; 45.9; 54.7; 57.2; 57.3; 66.8; 107.2; 111.8; 115.7 (q, J = 288 Hz); 116.9; 117.7; 118.6; 121.3; 125.1; 126.4; 126.6; 127.3; 128.1; 136.1; 138.1; 155.7 (q, J = 36 Hz).
LC-MS (Methode 8): [M+H]⁺: m/z = 483.3, Rₜ = 2.7 min.

### Stufe 3:

### N-(2-(1H-Indol-3-yl)-1-(1H-tetrazol-5-yl)ethyl)-N-(4-dimethylamino-4-phenylcyclohexyl)-2,2,2-trifluoracetamid (Beispiel 26, unpolares Diastereomer)

Eine Lösung von *N*-[1-Cyano-2-(1*H*-indol-3-yl)ethyl]-*N*-(4-dimethylamino-4-phenylcyclohexyl)-2,2,2-trifluoracetamid (unpolares Diastereomer)(241 mg, 0.5 mmol) in *N,N*-Dimethylformamid (5 mL) und Toluol (5 mL) wurde mit Natriumazid (325 mg, 5.0 mmol) und Triethylamin-Hydrochlorid (688 mg, 5.0 mmol) versetzt und 2 d bei 80 °C gerührt. Die Lösungsmittel wurden anschließend i. Vak. entfernt. Der Rückstand wurde wiederholt mit Toluol versetzt und jeweils wieder i. Vak. eingeengt. Das Rohprodukt wurde durch Flashchromatographie (37 g, 20 x 2.1 cm) mit Dichlormethan / Methanol [8:2 + 1 % NH₃ (32 % in H₂O)] gereinigt.
Beispiel 26: unpolares Diastereomer
Ausbeute: 219 mg (83 %), beigefarbener Feststoff
Schmelzpunkt: 216 °C
¹H-NMR (DMSO-d₆): 0.74 (d, 1 H, J = 10.8 Hz); 1.12-1.44 (m, 2H); 1.50-1.68 (m, 1 H); 1.70-1.80 (m, 1H); 1.86 (s, 5.4H); 2.00 (s, 0.6 H); 2.10-2.28 (m, 1H); 2.39 (d, 1H, J = 14.2 Hz); 2.78 (d, 1H); 3.68 (dd, 1H, J = 14.3, 8.1 Hz); 3.70-3.80 (m, 1H); 3.97 (dd, 1H, J = 6.8, 14.3 Hz); 5.20 (t, 0.9H, J = 7.2 Hz); 5.44 (m, 0.1 H); 7.02 (ddd, 1 H, J = 7.9, 7.0, 1.0 Hz); 7.09 (ddd, 1H, J = 7.9, 7.0, 1.0 Hz); 7.14 (d, 1 H, J = 2.3 Hz), 7.20-7.40 (m, 6H); 7.36 (d, 1H (J = 8.0 Hz); 7.67 (d, 1H, J = 7.4 Hz); 10.75 (s, 0.1 H); 10.91 (s, 0.9H).
LC-MS (Methode 8): [M+H]⁺: m/z = 526.3, Rₜ = 2.4 min.

### Stufe 4:

### N⁴-(2-(1H-Indol-3-yl)-1-(1H-tetrazol-5-yl)ethyl)-N¹,N¹-dimethyl-1-phenylcyclohexan-1,4-diamin (Beispiel 27, unpolares Diastereomer)

Eine Suspension von *N*-(2-(1*H*-Indol-3-yl)-1-(1*H*-tetrazol-5-yl)ethyl)-*N*-(4-dimethylamino-4-phenylcyclohexyl)-2,2,2-trifluoracetamid (unpolares Diastereomer) (200 mg, 0.38 mmol) in Methanol (10 mL) wurde mit 37 % Salzsäure (10 mL) versetzt und 7 h bei 100 °C im Teflon-Druckgefäß gerührt. Anschließend wurden erneut Methanol (10 mL) und 37 % Salzsäure (10 mL) zugesetzt und das Gemisch weitere 6 h bei 100 °C gerührt. Anschließend wurde die Reaktionslösung i. Vak. eingeengt und der Rückstand (250 mg) durch Flashchromatographie (37 g, 20 x 2.1 cm) mit Dichlormethan / Methanol [8:2 + 1 % NH₃ (32 % in H₂O)] gereinigt.
Beispiel 27: unpolares Diastereomer
Ausbeute: 98 mg (60 %), amorpher, beigefarbener Feststoff
¹H-NMR (DMSO-d₆): 1.35-2.00 (m, 8H); 2.11 (s, 6H); 2.55 (s, 1 H); 3.46 (d, 2H, J = 4.0 Hz); 4.61 (s, 1 H); 6.85 (s, 1 H); 6.95 (t, 1 H, J = 7.5 Hz); 7.03 (t, 1 H, J = 7.5 Hz); 7.10-7.80 (m, 9H); 10.82 (s, 1 H).
LC-MS (Methode 8): [M+H]⁺: m/z = 430.3, Rₜ = 1.6 min.

### Stufe 5:

### N-[1-Cyano-2-(1H-indol-3-yl)ethyl]-N-(4-dimethylamino-4-phenylcyclohexyl)-2,2,2-trifluoracetamid (polares Diastereomer)

2-(4-Dimethylamino-4-phenylcyclohexylamino)-3-(1 H-indol-3-yl)propionamid (polares Diastereomer) (1.58 g, 3.92 mmol) wurde mit wasserfreiem Pyridin (20 mL) versetzt und durch Rühren unter Rückfluss gelöst. Die Lösung wurde auf Raumtemperatur abgekühlt und mit wasserfreiem Dichlormethan (20 mL) und Triethylamin (6 mL) versetzt, dann auf -15 °C gekühlt und mit Trifluoressigsäureanhydrid (2.48 g, 1.64 mL, 11.8 mmol) versetzt. Das Gemisch wurde 3 h bei -15 °C gerührt. Anschließend wurden die Lösungsmittel i. Vak. entfernt. Der Rückstand wurde wiederholt mit Toluol versetzt und die Lösung jeweils wieder i. Vak. eingeengt. Der Rückstand wurde in Ethylacetat (20 mL) gelöst und mit Natriumhydrogencarbonat-Lösung (3 x 20 mL) gewaschen. Die organische Phase wurde mit Natriumsulfat getrocknet und i. Vak. eingeengt. Das Rohprodukt (1.6 g) wurde durch Flashchromatographie (120 g, 20 x 4.1 cm) mit Dichlormethan / Methanol [95:5 +1 % NH₃ (32 % in H₂O)] gereinigt.
Ausbeute: 693 mg (37 %), gelblicher Feststoff
Schmelzpunkt: 248 °C
¹H-NMR (DMSO-d₆): 0.98-1.07 (m, 2H); 1.22-1.72 (m, 4H); 1.86 (s, 6H); 2.43-2.50 (m, 1 H); 2.73 (br d, 1 H; J = 10.9 Hz); 3.20 (dd, 1 H, J = 14.0, 7.5 Hz); 3.45 (dd, 1 H, J = 14.0, 8.2 Hz); 3.73 (m, 1H); 3.99 (t, 1H; J = 7.8 Hz); 6.99 (t, 1H, J = 7.0 Hz); 7.06-7.15 (m, 3H); 7.24 (d, 2H, J = 7.8 Hz); 7.35 (t, 2H, J = 7.8 Hz); 7.43 (t, 2H, J = 7.6 Hz); 11.02 (s, 1H).
¹³C-NMR (DMSO-d₆): 25.4; 25.9; 26.5; 30.7; 31.6; 38.0; 45.0: 45.6; 57.8; 60.1; 106.9; 111.6; 115.7 (q, J = 287 Hz); 116.6; 117.6; 118.8; 121.2; 124.7; 126.5; 126.6; 127.7; 127.9; 135.3; 136.0; 155.6 (q, J = 36 Hz).
LC-MS (Methode 8): [M+H]⁺: m/z = 483.3, Rₜ = 2.3 min.

### Stufe 6:

### N-(2-(1H-Indol-3-yl)-1-(1H-tetrazol-5-yl)ethyl)-N-(4-dimethylamino-4-phenylcyclohexyl)-2,2,2-trifluoracetamid (Beispiel 28, polares Diastereomer)

Eine Lösung von N-[1-Cyano-2-(1H-indol-3-yl)ethyl]-N-(4-dimethylamino-4-phenylcyclohexyl)-2,2,2-trifluoracetamid (polares Diastereomer) (613 mg, 1.27 mmol) in *N,N-*Dimethylformamid (5 mL) und Toluol (5 mL) wurde mit Natriumazid (825 mg, 12.7 mmol) und Triethylamin-Hydrochlorid (1.74 g, 12.7 mmol) versetzt und 2 d bei 80 °C im Teflondruckgefäß gerührt. Anschließend wurden die Lösungsmittel i. Vak. eingeengt, der Rückstand wiederholt mit Toluol versetzt und die Lösung jeweils wieder i. Vak. eingeengt.

Das Rohprodukt (1.62 g) wurde durch Flashchromatographie (200 g, 20 x 4.1 cm) mit Dichlormethan / Methanol [2:1 + 1 % NH₃ (32 % in H₂O)] gereinigt.
Beispiel 28, polares Diastereomer
Ausbeute: 615 mg (92 %)
Schmelzpunkt: 195-228 °C
¹H-NMR (DMSO-d₆): 0.30-2.10 (m, 4H); 2.15 (s, 2H), 2.20-2.30 (m, 1H); 2.35 (4H, s); 2.64-3.00 (m, 2H); 3.24 (dd, 1H, J = 4.5 und 13.6 Hz); 3.50-3.75 (m, 1H); 3.85 (dd, 1H, J = 13.9, 10.2 Hz); 3.99 (m, 0.67 H); 4.25 (m, 0.33H); 5.32 (dd, 1H, J = 9.9, 4.2 Hz); 6.80 (d, 0.67 H, J = 2.0 Hz); 6.87 (d, 0.33H, J = 2.0 Hz); 6.92-7.62 (m, 10H); 10.77 (s, 0.66H); 10.92 (m, 0.33H).
LC-MS (Methode 8): [M+H]⁺: m/z = 526.3, Rₜ = 2.2 min.

### Stufe 7:

### N⁴-(2-(1H-Indol-3-yl)-1-(1H-tetrazol-5-yl)ethyl)-N¹,N¹-dimethyl-1-phenylcyclohexan-1,4-diamin (Beispiel 29, polares Diastereomer)

Eine Suspension von N-(2-(1H-Indol-3-yl)-1-(1H-tetrazol-5-yl)ethyl)-N-(4-dimethylamino-4-phenylcyclohexyl)-2,2,2-trifluoracetamid (100 mg, 0.19 mmol) in Methanol (10 mL) wurde mit 37 % Salzsäure (3 mL) versetzt und 7 h bei 100 °C im Teflon-Druckgefäß gerührt. Anschließend wurde erneut 37 % Salzsäure (3 mL) zugesetzt, das Gemisch weitere 24 h bei 100 °C gerührt und danach i. Vak. eingeengt. Das beiden Rohprodukt wurde durch Flashchromatographie (85 g, 20 x 3.6 cm) mit Dichlormethan / Methanol [2:1 + 1 % NH₃ (32 % in H₂O)] gereinigt.
Beispiel 29: polares Diastereomer
Ausbeute: 43 mg (53 %)
Schmelzpunkt: 190-195 °C
¹H-NMR (DMSO-d₆): 1.00-1.44 (m, 6H); 1.73 (br d, 2H, J = 10.6 Hz); 1.88 (s, 6H); 2.08 (br d, 1H, J = 12.1 Hz); 2.52-2.72 (m, 2H); 3.31 (dd, 1H, J = 13.7, 4.3 Hz); 3.36-3.48 (m, 1H); 4.63 (dd, 1H, J = 9.8, 4.4 Hz); 6.73 (d, 1H, J = 2.3 Hz); 6.93 (ddd, 1H, J = 7.9, 7.1, 1.1 H); 7.02 (ddd, 1H, J = 8.0, 7.1, 1.1 Hz); 7.24-7.55 (m, 7H); 10.75 (s, 1H).
¹³C-NMR (DMSO-d₆): 24.6; 26.3; 28.6; 30.4; 30.5; 37.8; 51.6; 53.8; 60.9; 108.8; 111.3; 117.7; 118.3; 120.7; 123.5; 126.5; 126.9; 127.7; 127.9; 135.6; 135.7; 157.3.
LC-MS (Methode 8): [M+H]⁺: m/z = 420.3, Rₜ = 1.0 min.

### Untersuchungen zur Wirksamkeit der erfindungsgemäßen Verbindungen

### Messung der ORL1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder% Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel IIC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### ³H]-BTX-Bindung am Natriumkanal

### (Natriumkanalbindungsstelle 2; BTX Assay)

Die [³H]-BTX-Verdrängung durch Prüfsubstanzen wird an Synaprosomen (aus dem Cortexgewebe von männlichen Sprague Dawley Ratten, 150-350g) in Gegenwart von [³H]-BTX, TTX und alpha-Scorpion Venom getestet.
- Batrachotoxin (BTX) bindet an Bindungsstelle 2 des Na⁺-Rezeptors und hemmt dort die Inaktivierung des Kanals
- Tetrodotoxin (TTX) bindet an Bindungsstelle 2 des Rezeptors und reduziert hier unspezifische Bindung von BTX
- Scorpion Venom verbessert die spezifische Bindung von BTX an Bindungsstelle 2 um das 20-30fache, indem es Bindungsstelle 3 spezifisch bindet
- Veratridin (VTD) bindet ebenso wie BTX Bindungsstelle 2 und hemmt somit kompetitiv die Bindung von BTX

Die Versuche werden bei einer fixen Kaliumkonzentration von 5,4 mM bei 37°C durchgeführt. Die Inkubationszeit beträgt 120 min. Unspezifische Bindungen werden in Gegenwart von VTD bestimmt. Nach Ablauf der Inkubationszeit wird die Testplatte über einer Filterplatte abgesaugt. Im Filter verbliebene und von [³H]-BTX gebundene Rezeptormoleküle können nun entsprechend über die Messung der Radioaktivität quantifiziert werden, woraus wiederum Aussagen über die Verdrängung von BTX durch Prüfsubstanzen resultieren. Weitere Details können im Methodenteil von Catterall et al. (1981) nachgelesen werden.

Literatur: Binding of batrachotoxinin A 20-alpha-benzoate to a receptor site associated with sodium channels in synaptic nerve ending particles. Catterall WA, Morrow CS, Daly JW, Brown GB. J Biol Chem. 1981 Sep 10;256(17):8922-7.

### Chung-Modell: Mononeuropathieschmerz nach spinaler Nervenligatur

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren ^{®}, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black, USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Datenpunkt. Die Tiere wurden 30 min vor und zu verschiedenen Zeiten nach Applikation von Testsubstanz-oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt.

Statistische Auswertung: ED₅₀ Werte und 95% Vertrauensbereiche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim, S.H. and Chung,J.M., An experimental model for peripheral neuropathy produced by segmental spinal nerve ligation in the rat, Pain, 50 (1992) 355-363.

### Nephelometrische Löslichkeitsuntersuchung (Phosphatpuffer pH 7.4):

Diese Methode untersucht die Löslichkeit einer Substanz bei festgelegten Konzentrationen (1 µM, 3 µM, 10 µM, 30 µM und 100 µM) in 10 mM Phosphatpufferlösung bei pH 7.4. Initial wird eine 10 mM Lösung der Substanzen in DMSO benötigt, aus der 100-fach Stammlösungen der oben genannten Konzentrationslevel wiederum in DMSO hergestellt werden, die finale DMSO Konzentration im Versuchsansatz beträgt 1 % (v/v). Das Experiment wird in mehrfach Bestimmung durchgeführt. Nach Zugabe der DMSO Stammlösungen zum Puffer, wird der Ansatz 2h bei 37°C inkubiert, bevor eine Absorptionsbestimmung bei 620 nm stattfindet. Steigt die Absorption der Proben über die der reinen Puffer/DMSO Lösung an, so gilt dies als Indikator für eine Präzipitatbildung. Die untere Löslichkeitsgrenze ("lower bound") ist die Konzentration, die derjenigen mit erster Präzipitatbildung vorangegangen ist (z.B. 3 µM, wenn Präzipitatbildung bei 10 µM detektiert wurde).

Ergebnistabelle:

| Nr. | % Hemmung (ORL1) [1 µM] | Ki (ORL1) Mittel [µM] | % Hemmung (µ)[1 µM] | Ki (µ) Mittel [µM] | SNL, Ratte, i.v. |
|---|---|---|---|---|---|
| 1 | 83 | 0,019 | 94 | 0,014 | nd |
| 2 | 47 | 0,395 | 65 | 0,730 | nd |
| 3 | 96 | 0,005 | 96 | 0,013 | 26% MPE bei 300 µg/kg |
| 4 | 42 | 0,165 | 69 | 0,103 | nd |
| 5 | nd | 0,060 | 72 | 0,195 | nd |
| 7 | nd | 0,052 | 95 | 0,011 | nd |
| 8 | nd | 0,630 | 45 | 1,010 | nd |
| 9 | 92 | 0,006 | 92 | 0,016 | nd |
| 10 | 71 | 0,135 | 91 | 0,480 | nd |
| 11 | 93 | nd | 93 | nd | nd |
| 12 | 91 | 0,008 | 97 | 0,007 | nd |
| 13 | 53 | nd | 80 | nd | nd |
| 14 | 93 | 0,010 | 97 | 0,005 | nd |
| 15 | 61 | 0,320 | 81 | 0,036 | nd |
| 17 | 97 | 0,004 | nd | 0,018 | nd |
| 18 | 60 | nd | 87 | nd | nd |
| 19 | 88 | 0,011 | 96 | 0,009 | nd |
| 20 | 86 | 0,02 | nd | 0,009 | nd |
| 22 | 78 | 0,015 | 98 | 0,01 | nd |
| 23 | 53 | 0,3 | 86 | 0,1 | nd |
| 24 | 92 | nd | 72 | nd | nd |
| 25 | 47 | nd | 46 | nd | nd |
| 26 | 39 | nd | 72 | nd | nd |
| 29 | 26 | 2,8 | nd | 0,34 | nd |

| | | | | | |
|---|---|---|---|---|---|
| nd = nicht bestimmt | | | | | |

Die erfindungsgemäßen Verbindungen vom Typ E mit Z = -NH, R # H (Bsp. 4, 6, 8, 13 und 15) wurden mit entsprechenden Verbindungen vom Typ E mit Z = O oder -NH, R = H und Me und(V-1 bis V-3) verglichen:

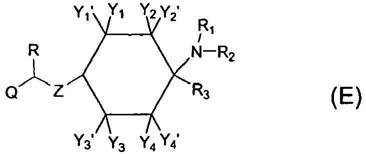

| Nr. | Z | R | Diastereomer | Inhibition(BTX) Mittel [% bei 10µM] |
|---|---|---|---|---|
| V-1 | -NH- | H | polar | 86 |
| V-2 | -NH- | Me | polar | 62 |
| V-3 | -O- | H | polar | 77 |
| Bsp.-4 | -NH- | 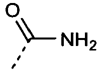 | polar | 29 |
| Bsp.-6 | -NH- | 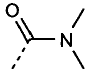 | polar | 15 |
| Bsp.-8 | -NH- | 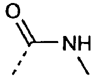 | polar | 27 |
| Bsp.-13 | -NH- | 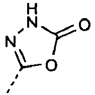 | polar | 43 |
| Bsp.-15 | -NH- | 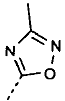 | polar | 41 |

Wie der vorstehende Vergleich belegt, weisen die erfindungsgemäßen Verbindungen, insbesondere das jeweils polarere Diastereomer, im Vergleich zu strukturell ähnlichen Verbindungen eine geringere Affinität zum BTX-Ionenkanal auf, was insbesondere Vorteile im Hinblick auf unerwünschte kardiovasuläre Nebenwirkungen mit sich bringen sollte.

Die erfindungsgemäßen Verbindungen vom Typ E mit Z = -NH, R # H (Bsp. 1, 9, 18 und 20) wurden mit Verbindungen vom Typ F mit Z = -NH oder -NMe, R₆ = H oder Me (R¹ bis R³, Y₁ bis Y₄ und Y₁' bis Y₄' jeweils identisch) (V-4 bis V-6) verglichen:

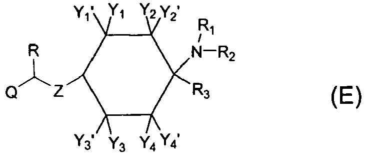

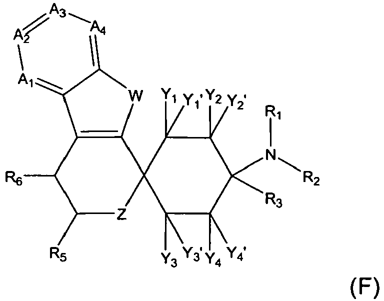

| Bsp. | R | Z | R₆ | Diastereomer | Nephelometrie (lower bound) µM |
|---|---|---|---|---|---|
| 1 | 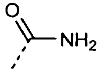 | NH | entfällt | unpolar | 100 |
| 9 | 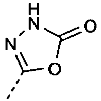 | NH | entfällt | unpolar | 100 |
| 18 | 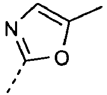 | NH | entfällt | polar | 100 |
| 20 | 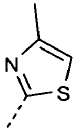 | NH | entfällt | unpolar | 100 |
| V-4: | entfällt | NMe | H | unpolar | 3 |
| V-5: | entfällt | NMe | H | polar | 3 |
| V-6: | entfällt | NH | Me | unpolar | 10 |

Wie der vorstehende Vergleich belegt, weisen die erfindungsgemäße Verbindung aus den Beispielen 1, 9, 18 und 20 im Vergleich zu strukturell ähnlichen Verbindungen (V-4 bis V-6) eine bessere Löslichkeit in wässrigen Medien auf, was insbesondere Vorteile im Hinblick auf die Resorptionseigenschaften und/oder die Bioverfügbarkeit mit sich bringen sollte.

## Patentansprüche

1. Verbindung der allgemeinen Formel (1), 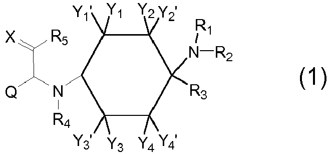 worin
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂; oder Y₁ und Y₁', oder Y₂ und Y₂', oder Y₃ und Y₃', oder Y₄ und Y₄' gemeinsam für =O stehen;
Q für -R₀ steht;
X für =O oder =N-R₆ steht;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -(CH₂)₃₋₆- oder -CH₂CH₂NR'CH₂CH₂- mit R' = -H, -R₀ oder -C(=O)R₀ stehen;
R₃ für -R₀ steht;
R₄ für -H, -C(=O)R₀ oder -R₀ steht;
R₅ für-NH₂, -NHR₀, oder -N(R₀)₂ steht;
R₆ für -H, -F, -Cl, -Br, -I, -CN, -NO₂, -CHO, -R₀, -C(=O)H, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O-, -NHC(=O)R₀, -NHC(=O)OR₀, -NHC(=O)NH₂, -NHC(=O)NHR₀ oder -NHC(=O)N(R₀)₂ steht;
oder R₅ und R₆ gemeinsam einen fünf- oder sechsgliedrigen Ring bilden, dessen übrige Ringatome jeweils unabhängig voneinander C, N, S oder O sind, wobei der Ring aromatisch oder nicht aromatisch, unsubstituiert oder ein- oder mehrfach substituiert ist durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀ und -NH-C(=O)N(R₀)₂;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt;
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O-, -NHC(=O)R₀, -NHC(=O)OR₀, -NHC(=O)NH₂, -NHC(=O)-NHR₀, -NH-C(=O)N(R₀)₂, -Si(R₀)₃ und -PO(OR₀)₂verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂ verstanden wird; wobei ggf. vorhandene N-Ringatome jeweils oxidiert sein können;
in Form eines einzelnen Stereoisomers oder deren Mischung, der freien Verbindungen und/oder ihrer physiologisch verträglichen Salze.

2. Verbindung nach Anspruch 1, welche die allgemeine Formel (1.1) oder (1.2) 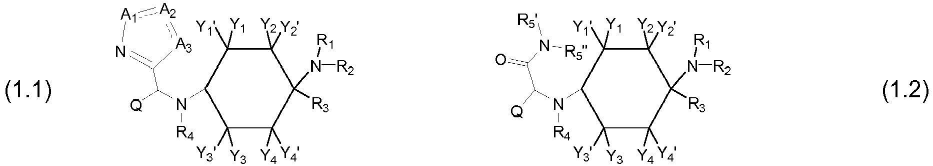 aufweist, worin, soweit vorhanden,
A₁ für -N=, -NH-, -NR₈- oder -CR₈= steht;
A₂ für =N-, -C(=O)- oder =CR₉- steht;
A₃ für -O-, -NH- oder -NR₁₀- steht; und
R₅', R₅", R₈, R₉ und R₁₀ jeweils unabhängig voneinander für -H, =O oder -C₁₋₈-Aliphat stehen.

3. Verbindung nach Anspruch 1, welche die allgemeine Formel (2), (3), (4), (5) oder (6) 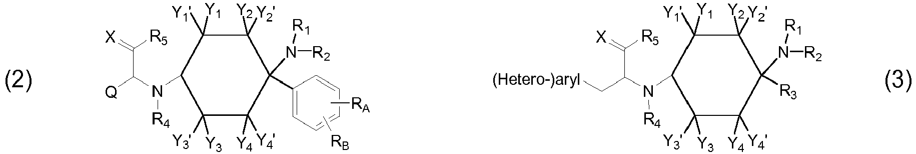 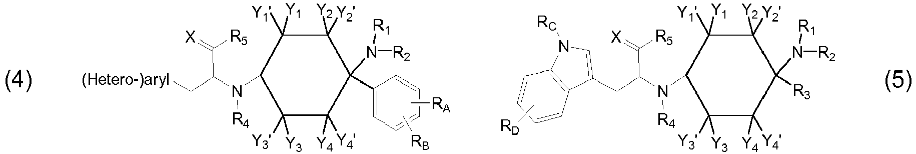 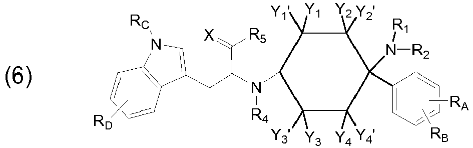 aufweist, worin, soweit vorhanden,
R_{A}, R_{B}, R_{C} und R_{D} jeweils unabhängig voneinander ausgewählt sind aus der Gruppe bestehend aus -H, -C₁₋₈-Aliphat, -OH, -OC₁₋₈-Aliphat, -CF₃, -F, -Cl, -Br, -NO₂, -CN, -Heteroaryl, -C₁₋₈-Aliphat-Aryl und -C₁₋₈-Aliphat-Heteroaryl; und
(Hetero-)aryl für -Heteroaryl oder -Aryl steht.

4. Verbindung nach Anspruch 1, ausgewählt aus der Gruppe bestehend aus
• (S)-2-(4-(Dimethylamino)-4-phenylcyclohexylamino)-3-(1H-indol-3-yl)propanamid;
• (±)-2-(4-(Dimethylamino)-4-phenylcyclohexylamino)-3-(1H-indol-3-yl)propanamid;
• (S)-2-(4-(Dimethylamino)-4-phenylcyclohexylamino)-3-(1H-indol-3-yl)-N,N-dimethyl-propanamid;
• (S)-2-(4-(Dimethylamino)-4-phenylcyclohexylamino)-3-(1H-indol-3-yl)-N-methyl-propanamid;
• 5-((*S*)-1-(4-(Dimethylamino)-4-phenylcyclohexylamino)-2-(1H-indol-3-yl)ethyl)-1,3,4-oxadiazol-2(3H)-on;
• N4-((S)-2-(1H-indol-3-yl)-1-(3-methyl-1,2,4-oxadiazol-5-yl)ethyl)-N1,N1-dimethyl-1-phenylcyclohexan-1,4-diamin;
• 5-((R)-1-(4-(Dimethylamino)-4-phenylcyclohexylamino)-2-(1H-indol-3-yl)ethyl)-1,3,4-oxadiazol-2(3H)-on;
• N4-((R)-2-(1H-indol-3-yl)-1-(3-methyl-1,2,4-oxadiazol-5-yl)ethyl)-N1,N1-diethyl-1-phenylcyclohexan-1,4-diamin;
• N4-((S)-2-(1H-indol-3-yl)-1-(5-methyloxazol-2-yl)ethyl)-N1,N1-dimethyl-1-phenyl-cyclohexan-1,4-diamin;
• N4-(2-(1H-indol-3-yl)-1-(1H-tetrazol-5-yl)ethyl)-N1,N1-dimethyl-1-phenylcyclohexan-1,4-diamin;
• N4-(2-(1H-Indol-3-yl)-1-(1H-tetrazol-5-yl)ethyl)-N1,N1,N4-trimethyl-1-phenylcyclo-hexan-1,4-diamin;
• N4-(2-(1H-indol-3-yl)-1-(3-methyl-1,2,4-oxadiazol-5-yl)ethyl)-N1,N1,N4-trimethyl-1-phenylcyclohexan-1,4-diamin;
• N4-(2-(1H-indol-3-yl)-1-(5-methyloxazol-2-yl)ethyl)-N1,N1,N4-trimethyl-1-phenyl-cyclohexan-1,4-diamin;
• 5-(1-((4-(dimethylamino)-4-phenylcyclohexyl)(methyl)amino)-2-(1H-indol-3-yl)ethyl)-1,3,4-oxadiazol-2(3H)-one;
• 2-((4-(dimethylamino)-4-phenylcyclohexyl)(methyl)amino)-3-(1H-indol-3-yl)-N,N-dimethylpropanamide;
• N4-(2-(1H-indol-3-yl)-1-(3-methyl-1,2,4-oxadiazol-5-yl)ethyl)-1-(3-fluorophenyl)-N1,N1-dimethylcyclohexan-1,4-diamin;
• N4-(2-(1H-indol-3-yl)-1-(5-methyloxazol-2-yl)ethyl)-1-(3-fluorophenyl)-N1,N1-dimethylcyclohexan-1,4-diamin;
• N-(2-(1H-indol-3-yl)-1-(5-methyloxazol-2-yl)ethyl)-N-(4-(dimethylamino)-4-(3-fluorophenyl)cyclohexyl)zimtsäureamid; und
• N-(2-(1H-indol-3-yl)-1-(3-methyl-1,2,4-oxadiazol-5-yl)ethyl)-N-(4-(dimethylamino)-4-(3-fluorophenyl)cyclohexyl)zimtsäureamid;
• (R)-N4-(2-(1H-Indol-3-yl)-1-(5-methyloxazol-2-yl)ethyl)-N1,N1-dimethyl-1-phenyl-cyclohexan-1,4-diamin
• (S)-N4-(2-(1H-Indol-3-yl)-1-(4-methylthiazol-2-yl)ethyl)-N1,N1-dimethyl-1-penylcyclo-hexan-1,4-diamin
• (R)-N4-(2-(1H-Indol-3-yl)-1-(4-methylthiazol-2-yl)ethyl)-N1,N1-dimethyl-1-penylcyclo-hexan-1,4-diamin
• N-(2-(1H-Indol-3-yl)-1-(1H-tetrazol-5-yl)ethyl)-N-(4-dimethylamino-4-phenyl-cyclohexyl)-2,2,2-trifluoracetamid
• N4-(2-(1H-Indol-3-yl)-1-(1H-tetrazol-5-yl)ethyl)-N1,N1-dimethyl-1-phenylcyclohexan-1,4-diamin
oder deren physiologisch verträgliche Salze.

5. Arzneimittel enthaltend wenigstens eine Verbindung nach einem der Ansprüche 1 bis 4 in Form eines einzelnen Stereoisomers oder deren Mischung, der freien Verbindungen und/oder ihrer physiologisch verträglichen Salze, sowie ggf. geeignete Zusatz- und/oder Hilfsstoffe und/oder gegebenenfalls weiterer Wirkstoffe.

6. Verwendung einer Verbindung nach einem der Ansprüche 1 bis 4 in Form eines einzelnen Stereoisomers oder deren Mischung, der freien Verbindung und/oder ihrer physiologisch verträglichen Salze, zur Herstellung eines Arzneimittels zur Behandlung von Schmerz.

7. Verwendung einer Verbindung nach einem der Ansprüche 1 bis 4 in Form eines einzelnen Stereoisomers oder deren Mischung, der freien Verbindungen und/oder ihrer physiologisch verträglichen Salze und/oder Solvate zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Compound of the general formula (1) 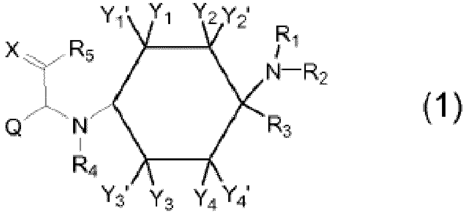 in which
Y₁, Y₁' , Y₂, Y₂' , Y₃, Y₃' , Y₄ and Y₄' are each independently of one another selected from the group consisting of -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, - S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ and -NHC(=O)N(R₀)₂, or Y₁ and Y₁' or Y₂ and Y₂' or Y₃ and Y₃' or Y₄ and Y₄' together represent =0,
Q represents -R₀,
X represents =0 or =N-R₆,
R₀ in each case independently represents -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl,
R₁ and R₂ independently of one another represent -H or -R₀, or R₁ and R₂ together represent -CH₂CH₂OCH₂CH₂-, - (CH₂)₃₋₆- or -CH₂CH₂NR'CH₂CH₂-, where R'= -H, -R₀ or -C(=O)R₀,
R₃ represents -R₀,
R₄ represents -H, -C(=O)R₀ or -R₀,
R₅ represents -NH₂, -NHR₀ or -N(R₀)₂,
R₆ represents -H, -F, -Cl, -Br, -I, -CN, -NO₂, -CHO, -R₀, -C(=O)H, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S (=0) ₁₋₂NH₂, -NH₂, -NHR₀, -N(R₀)₂, -N⁺(R₀)₃, -N⁺(R₀)₂O, -NHC(=O)R₀, -NHC(=O)OR₀, -NHC(=O)NH₂, -NHC(=O)NHR₀ or -NHC(=O)N=(R₀)₂,
or R₅ and R₆ together form a five- or six-membered ring whose other ring atoms are, in each case independently of one another, C, N, S or 0, where the ring is aromatic or non-aromatic, unsubstituted or mono- or polysubstituted by substituents independently of one another selected from the group consisting of -F, -Cl, -Br, -I, -CN, -NO₂, -CHO, =0, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC (=0) OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀ and -NH-C(=O)N(R₀)₂,
where
"aliphatic" is in each case a branched or straight-chain, saturated or mono- or polyunsaturated, unsubstituted or mono- or polysubstituted, aliphatic hydrocarbon radical, "cycloaliphatic" is in each case a saturated or mono- or polyunsaturated, unsubstituted or mono-or polysubstituted, alicyclic, mono- or multicyclic hydrocarbon radical whose number of ring carbon atoms is preferably in the stated range,
where, with respect to "aliphatic" and "cycloaliphatic" "mono- or polysubstituted" is to be understood as meaning mono- or polysubstitution of one or more hydrogen atoms, for example monosubstitution, disubstitution, trisubstitution or complete substitution, by substituents independently of one another selected from the group consisting of -F, -Cl, -Br, -I, -CN, -NO₂, -CHO, =0, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O, -NHC(=O)R₀, -NHC(=O)OR_{0,} -NHC(=O)NH₂, -NHC(=O)-NHR₀, -NH-C(=O)N(R₀)₂, -Si(R₀)₃ and -PO(OR₀)₂,
"aryl" in each case independently represents a carbocyclic ring system having at least one aromatic ring, but without heteroatoms in this ring, where the aryl radicals may optionally be condensed with further saturated, (partially) unsaturated or aromatic ring systems and each aryl radical may be present unsubstituted or mono- or polysubstituted, where the aryl substituents may be identical or different and may be in any possible position of the aryl,
"heteroaryl" represents a 5-, 6- or 7-membered cyclic aromatic radical which contains 1, 2, 3, 4 or 5 heteroatoms, where the heteroatoms are identical or different and are nitrogen, oxygen or sulphur and where the heterocycle may be unsubstituted or mono- or polysubstituted, where in the case of substitution at the heterocycle the substituents may be identical or different and may be in any possible position of the heteroaryl, and where the heterocycle may also be part of a bi- or polycyclic system,
where, with respect to "aryl" and "heteroaryl", "mono- or polysubstituted" is to be understood as meaning mono- or polysubstitution of one or more hydrogen atoms of the ring system by substituents selected from the group consisting of -F, -Cl, -Br, -I, -CN, -NO₂, -CHO, =0, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂, where any nitrogen ring atoms present may in each case be oxidized,
in the form of an individual stereoisomer or its mixture, of the free compounds and/or their physiologically acceptable salts.

2. Compound according to Claim 1 of the general formula (1.1) or (1.2) 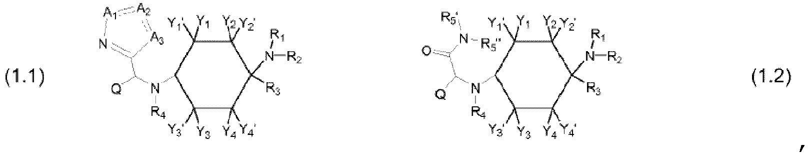 where, if present,
A₁ represents -N=, -NH-, -NR₈- or -CR₈=,
A₂ represents =N-, -C(=O)- or =CR₉-,
A₃ represents -O-, -NH- or -NR₁₀- and
R₅', R₅'', R₈, R₉ and R₁₀ in each case independently of one another represent -H, =0 or -C₁₋₈-aliphatic.

3. Compound according to Claim 1 of the general formula (2), (3), (4), (5) or (6) 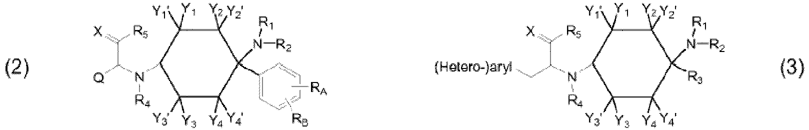 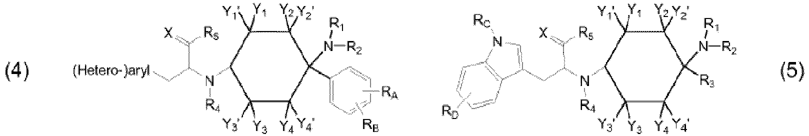 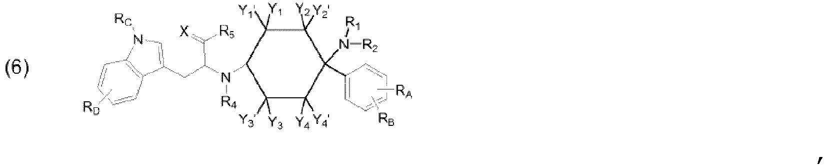 where, if present,
R_{A}, R_{B}, R_{C} and R_{D} are each independently of one another selected from the group consisting of -H, -C₁₋₈-aliphatic, -OH, -OC₁₋₈-aliphatic, -CF₃, -F, -Cl, -Br, -NO₂, -CN, -heteroaryl, -C₁₋₈-aliphatic-aryl and -C₁₋₈-aliphatic-heteroaryl and (hetero-)aryl represents -heteroaryl or -aryl.

4. Compound according to Claim 1, selected from the group consisting of
• (S)-2-(4-(dimethylamino)-4-phenylcyclohexylamino)-3-(1H-indol-3-yl)propaneamide,
• (±)-2-(4-(dimethylamino)-4-phenylcyclohexylamino)-3-(1H-indol-3-yl)propaneamide,
• (S)-2-(4-(dimethylamino)-4-phenylcyclohexylamino)-3-(1H-indol-3-yl)-N,N-dimethylpropaneamide,
• (S)-2-(4-(dimethylamino)-4-phenylcyclohexylamino)-3-(1H-indol-3-yl)-N-methylpropaneamide,
• 5-((S)-1-(4-(dimethylamino)-4-phenylcyclohexylamino)-2-(1H-indol-3-yl)ethyl)-1,3,4-oxadioazol-2(3H)-one,
• N4-((S)-2-(1H-indol-3-yl)-1-(3-methyl-1,2,4-oxadiazol-5-yl)ethyl)-N1,N1-dimethyl-1-phenylcyclohexane-1,4-diamine,
• 5-((R)-1-(4-(dimethylamino)-4-phenylcyclohexylamino)-2-(1H-indol-3-yl)ethyl)-1,3,4-oxadiazol-2(3H)-one,
• N4-((R)-2-(1H-indol-3-yl)-1-(3-methyl-1,2,4-oxadiazol-5-yl)ethyl)-N1,N1-dimethyl-1-phenylcyclohexane-1,4-diamine,
• N4-((S)-2-(1H-indol-3-yl)-1-(5-methyloxazol-2-yl)ethyl)-N1,N1-dimethyl-1-phenylcyclohexane-1,4-diamine,
• N4-(2-(1H-indol-3-yl)-1-(1H-tetrazol-5-yl)ethyl)-N1,N1-dimethyl-1-phenylcyclohexane-1,4-diamine,
• N4-(2-(1H-indol-3-yl)-1-(1H-tetrazol-5-yl)ethyl)-N1,N1,N4-trimethyl-1-phenylcyclohexane-1,4-diamine,
• N4-(2-(1H-indol-3-yl)-1-(3-methyl-1,2,4-oxadiazol-5-yl)ethyl)-N1,N1,N4-trimethyl-1-phenylcyclohexane-1,4-diamine,
• N4-(2-(1H-indol-3-yl)-1-(5-methyloxazol-2-yl)ethyl)-N1,N1,N4-trimethyl-1-phenylcyclohexane-1,4-diamine,
• 5-(1-(4-(dimethylamino)-4-phenylcyclohexyl)(methyl)amino)-2-(1H-indol-3-yl)ethyl)-1,3,4-oxadiazol-2(3H)-ones,
• 2-((4-(dimethylamino)-4-phenylcyclohexyl)(methyl)amino)-3-(3H-indol-3-yl)-N,N-dimethylpropanamide,
• N4-(2-(1H-indol-3-yl)-1-(3-methyl-1,2,4-oxadiazol-5-yl)-ethyl)-1-(3-fluorophenyl)-N1,N1-dimethylcyclohexane-1,4-diamine,
• N4-(2-(1H-indol-3-yl)-1-(5-methyloxazol-2-yl)ethyl)-1-(3-fluorophenyl)-N1,N1-dimethylcyclohexane-1,4-diamine,
• N-(2-(1H-indol-3-yl)-1-(5-methyloxazol-2-yl)ethyl)-N-(4-(dimethylamino)-4-(3-fluorophenyl)cyclohexyl)cinnamide and
• N-(2-(1H-indol-3-yl)-1-(3-methyl-1,2,4-oxadiazol-5-yl)ethyl)-N-(4-(dimethylamino)-4-(3-fluorophenyl)cyclohexyl)cinnamide,
• (R)-N4-(2-(1H-indol-3-yl)-1-(5-methyloxazol-2-yl)ethyl)-N1,N1-dimethyl-1-phenylcyclohexane-1,4-diamine,
• (S)-N4-(2-(1H-indol-3-yl)-1-(4-methylthiazol-2-yl)ethyl)-N1,N1-dimethyl-1-phenylcyclohexane-1,4-diamine,
• (R)-N4-(2-(1H-indol-3-yl)-1-(4-methylthiazol-2-yl)ethyl)-N1,N1-dimethyl-1-phenylcyclohexane-1,4-diamine,
• N-(2-(1H-indol-3-yl)-1-(1H-tetrazol-5-yl)ethyl)-N-(4-dimethylamino-4-phenylcyclohexyl)-2,2,2-trifluoroacetamide,
• N4-(2-(1H-Indol-3-yl)-1-(1H-tetrazol-5-yl)ethyl)-N1,N1-dimethyl-1-phenylcyclohexane-1,4-diamine
or their physiologically acceptable salts.

5. Medicaments, comprising at least one compound according to any of Claims 1 to 4 in the form of an individual stereoisomer or a mixture thereof, of the free compounds and/or their physiologically acceptable salts, and optionally suitable additives and/or auxiliaries and/or optionally further active compounds.

6. Use of a compound according to any of Claims 1 to 4 in the form of an individual stereoisomer or a mixture thereof, of the free compound and/or its physiologically acceptable salts, for preparing a medicament for the treatment of pain.

7. Use of a compound according to any of Claims 1 to 4 in the form of an individual stereoisomer or a mixture thereof, of the free compounds and/or their physiologically acceptable salts and/or solvates for preparing a medicament for the treatment of anxiety, of stress and stress-related syndromes, depression, epilepsy, Alzheimer's disease, senile dementia, general cognitive dysfunction, impaired learning and memory (as nootropic), withdrawal symptoms, alcohol and/or drug and/or prescription drug abuse and/or dependency, sexual dysfunction, cardiovascular disorders, hypotension, hypertension, tinnitus, pruritus, migraine, hearing difficulty, lack of intestinal motility, impaired food uptake, anorexia, obesitas, locomotive disorders, diarrhoea, cachexia, urine incontinence or as a muscle relaxant, anticonvulsive drug or anaesthetic or for coadministration in the treatment with an opioid analgetic or an anaesthetic, for diuresis or antinatriuresis, anxiolysis, for modulating movement activity, for modulating neurotransmitter release and for treating neurodegenerative disorders associated therewith, for treating withdrawal symptoms and/or for reducing the addictive potential of opioids.

## Revendications

1. Composé de formule générale (1) 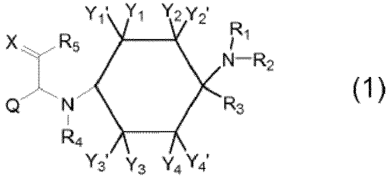 dans laquelle
Y₁, Y₁', Y₂, Y₂', Y₃, Y₃', Y₄ et Y₄' sont choisis chacun indépendamment les uns des autres dans le groupe constitué par -H, -F, -Cl, -Br, -I, -CN, -NO₂, -CHO,-R₀, -C(=O)R₀, -C(=O)H, -C(=O)-OH, -C(=O)OR₀, -C(=O)NH₂,-C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)3, -N⁺(R₀)₂O⁻, -NHC(=O)R₀, -NHC(=O)OR₀, -NHC(=O)NH₂,-NHC(=O)NHR₀ et -NHC(=O)N(R₀); ou Y₁ et Y₁' ou Y₂ et Y₂', ou Y₃ et Y₃' , ou Y₄ et Y₄' représentent ensemble =O ;
Q représente -R₀ ;
X représente =0 ou =N-R₆ ;
les R₀ représentent chacun indépendamment -aliphatique en C₁₋₈, -cycloaliphatique en C₃₋₁₂, -aryle, -hétéroaryle, -aliphatique en C₁₋₈-cycloaliphatique en C₃₋₁₂,-aliphatique en C₁₋₈-aryle, -aliphatique en C₁₋₈-hétéroaryle, -cycloaliphatique en C₃₋₈-aliphatique en C₁₋₈, -cycloaliphatique en C₃₋₈-aryle ou-cycloaliphatique en C₃₋₈-hétéroaryle ;
R₁ et R₂ représentent indépendamment l'un de l'autre -H ou -Ro ; ou R₁ et R₂ représentent ensemble-CH₂CH₂OCH₂CH₂-, - (CH₂)₃₋₆- ou -CH₂CH₂NR'CH₂CH₂- avec R' =-H, -R₀ ou -C(=O)R₀ ;
R₃ représente -R₀ ;
R₄ représente -H, -C(=O)R₀ ou -R₀ ;
R₅ représente -NH₂, -NHR₀ ou -N(R₀)₂ ;
R₆ représente -H, -F, -Cl, -Br, -I, -CN, -NO₂, -CHO,-R₀, -C(=O)H, -C(=O)R₀, -C(=O)OH, -C(=O)OR₀, -C(=O)NH₂,-C(=O)NHR₀, -C(=O)N(R₀)₂, -OH, -OR₀, -OC(=O)H, -OC(=O)R₀, -OC (=O) OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N+(R₀)₃, -N⁺(R₀)₂O⁻, -NHC(=O)R₀, -NHC(=O)OR₀, -NHC(=O)NH₂,-NHC(=O)NHR₀ ou -NHC(=O)N(R₀)₂ ;
ou R₅ et R₆ forment ensemble un cycle à cinq ou six éléments, dont les autres atomes de cycle sont chacun indépendamment les uns des autres C, N, S ou 0, le cycle étant aromatique ou non aromatique, non substitué ou substitué une ou plusieurs fois par des substituants choisis indépendamment les uns des autres dans le groupe constitué par -F, -Cl, -Br, -I, -CN, -NO₂, -CHO, =0, -R₀, -C(=O)R₀, -C(=O)H, -C(=O)OH, -C(=O)OR₀,-C(=O)NH₂, -C(=O)NHR₀, -C(=O)N(R₀)₂, -OH, -OR₀, -OC(=O)H, -OC(=O)R₀,-OC(=O)R₀,-OC(=O)NHR₀, -OC(=O)N(R₀)₂, -SH,-SR₀, -SO₃H, -S (=O)₁₋₂-R₀, -S (=0) ₁₋₂NH₂, -NH₂, -NHR₀,-N(R₀)₂, -N⁺(R₀)₃, -N⁺(R₀)₂O⁻, -NHC(=O)R₀, -NHC(=O)OR₀,-NHC(=O)NH₂, -NHC(=O)-NHR₀ et -NH-C (=0) N (R₀)₂ ; « aliphatique » étant à chaque fois un radical hydrocarboné aliphatique ramifié ou non ramifié, saturé ou mono- ou polyinsaturé, non substitué ou substitué une ou plusieurs fois ; « cycloaliphatique » étant à chaque fois un radical hydrocarboné saturé ou mono- ou polyinsaturé, non substitué ou substitué une ou plusieurs fois, alicyclique, mono- ou polycyclique, dont le nombre d'atomes de carbone de cycle se situe de préférence dans la plage indiquée ; au regard de « aliphatique » et « cycloaliphatique », « substitué une ou plusieurs fois » se rapportant au remplacement une ou plusieurs fois d'un ou de plusieurs atomes d'hydrogène, p. ex. le remplacement d'un, de deux, de trois ou de la totalité, par des substituants choisis indépendamment les uns des autres dans le groupe constitué par -F, -Cl, -Br, -I, -CN, -NO₂, -CHO, =0, -R₀, -C(=O)R₀, -C(=O)H, -C(=O)OH, -C(=O)OR₀,-C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH,-SR₀, -SO₃H, -S (=O)₁₋₂-R₀, -S (=O)₁₋₂NH₂, -NH₂, -NHR₀,-N(R₀)₂, -N⁺(R₀)₃, -N⁺(R₀)₂O⁻, -NHC(=O)R₀, -NHC(=O)OR₀, NHC(=O)NH₂, -NHC(=O)-NHR₀, -NH-C(=O)N(R₀)₂, -Si(R₀)₃ et-PO(OR₀)2 ;
« aryle » représentant à chaque fois indépendamment un système cyclique carbocyclique contenant au moins un cycle aromatique, mais sans hétéroatomes dans ce cycle, les radicaux aryle pouvant éventuellement être condensés avec d'autres systèmes cycliques saturés, (partiellement) insaturés ou aromatiques, et chaque radical aryle pouvant être non substitué ou substitué une ou plusieurs fois, les substituants de l'aryle pouvant être identiques ou différents, et pouvant se trouver à toute position quelconque et possible de l'aryle ;
« hétéroaryle » représentant un radical aromatique cyclique à 5, 6 ou 7 éléments, qui contient 1, 2, 3, 4 ou 5 hétéroatomes, les hétéroatomes pouvant être l'azote, l'oxygène ou le soufre de manière identique ou différente, et l'hétérocycle pouvant être non substitué ou substitué une ou plusieurs fois ; dans le cas de la substitution sur l'hétérocycle, les substituants pouvant être identiques ou différents, et pouvant se trouver à toute position quelconque et possible de l'hétéroaryle ; et l'hétérocycle pouvant également faire partie d'un système bi- ou polycyclique ;
au regard de « aryle » et « hétéroaryle », « substitué une ou plusieurs fois » se rapportant au remplacement une ou plusieurs fois d'un ou de plusieurs atomes d'hydrogène du système cyclique par des substituants choisis indépendamment dans le groupe constitué par -F, -Cl, -Br, -I, -CN, -NO₂, -CHO, =0, -R₀, -C(=O)R₀,-C(=O)H, -C(=O)OH, -C(=O)OR₀, -C(=O)NH₂, -C(=O)NHR₀,-C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻ , -NHC(=O)R₀, -NHC(=O)OR₀, -NHC(=)NH₂,-NHC(=O)NHR₀, -NH-C(=O)N(R₀)₂, -Si(R₀)₃ et -PO(OR₀)₂ ; les atomes de cycle N éventuellement présents pouvant à chaque fois être oxydés ;
sous la forme d'un stéréoisomère individuel ou son mélange, des composés libres et/ou leurs sels physiologiquement compatibles.

2. Composé selon la revendication 1, qui présente la formule générale (1.1) ou (1.2) 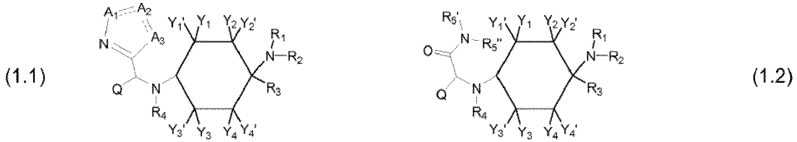 dans lesquelles, s'ils sont présents,
A₁ représente -N=, -NH-, -NR₈- ou -CR₈= ;
A₂ représente =N-, -C(=O)- ou =CR₉- ;
A₃ représente -0-, -NH- ou -NR₁₀- ; et
R₅' , R₅'', R₈, R₉ et R₁₀ représentent chacun indépendamment les uns des autres -H, =0, -aliphatique en C₁₋₈.

3. Composé selon la revendication 1, qui présente la formule générale (2), (3), (4), (5) ou (6) 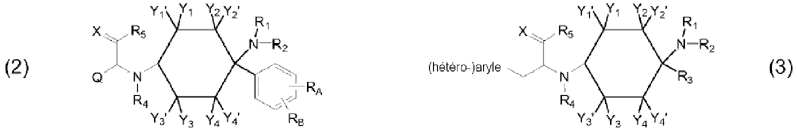 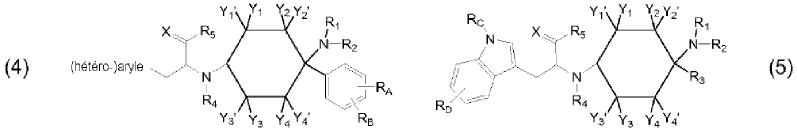 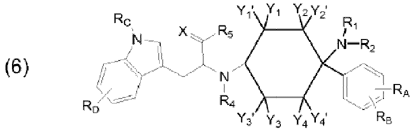 dans lesquelles, s'ils sont présents,
R_{A}, R_{B}, R_{C} et R_{D} sont chacun choisis indépendamment les uns des autres dans le groupe constitué par -H,-aliphatique en C₁₋₈, -OH, -0-aliphatique en C₁₋₈, -CF₃,-F, -Cl, -Br, -NO₂, -CN, -hétéroaryle, -aliphatique en C₁₋₈-aryle et -aliphatique en C₁₋₈-hétéroaryle ; et (hétéro-)aryle représente -hétéroaryle ou -aryle.

4. Composé selon la revendication 1, choisi dans le groupe constitué par
- (S)-2-(4-(diméthylamino)-4-phénylcyclohexylamino)-3-(1H-indol-3-yl)propanamide ;
- (±)-2-(4-(diméthylamino)-4-phénylcyclohexylamino)-3-(1H-indol-3-yl)propanamide ;
- (S)-2-(4-(diméthylamino)-4-phénylcyclohexylamino)-3-(1H-indol-3-yl)-N,N-diméthyl-propanamide ;
- (S)-2-(4-(diméthylamino)-4-phénylcyclohexylamino)-3-(1H-indol-3-yl)-N-méthyl-propanamide ;
- 5-((S)-1-(4-(diméthylamino)-4-phénylcyclohexylamino)-2-(1H-indol-3-yl)éthyl)-1,3,4-oxadiazole-2(3H)-one ;
- N4-((S)-2-(1H-indol-3-yl)-1-(3-méthyl-1,2,4-oxadiazol-5-yl)éthyl)-N1,N1-diméthyl-1-phénylcyclohexane-1,4-diamine ;
- 5-((R)-1-(4-(diméthylamino)-4-phénylcyclohexylamino)-2-(1H-indol-3-yl)éthyl)-1,3,4-oxadiazole-2(3H)-one ;
- N4-((R)-2-(1H-indol-3-yl)-1-(3-méthyl-1,2,4-oxadiazol-5-yl)éthyl)-N1,N1-diméthyl-1-phénylcyclohexane-1,4-diamine ;
- N4-((S)-2-(1H-indol-3-yl)-1-(5-méthyloxazol-2-yl)éthyl)-N1,N1-diméthyl-1-phényl-cÿclohexane-1,4-diamine ;
- N4-(2-(1H-indol-3-yl)-1-(1H-tétrazol-5-yl)éthyl)-N1,N1-diméthyl-1-phénylcyclohexane-1,4-diamine ;
- N4-(2-(1H-indol-3-yl)-1-(1H-tétrazol-5-yl)éthyl)-N1,N1,N4-triméthyl-1-phénylcyclohexane-1,4-diamine ;
- N4-(2-(1H-indol-3-yl)-1-(3-méthyl-1,2,4-oxadiazol-5-yl)éthyl)-N1,N1,N4-triméthyl-1-phénylcyclohexane-1,4-diamine ;
- N4-(2-(1H-indol-3-yl)-1-(5-méthyloxazol-2-yl)éthyl)-N1,N1,N4-triméthyl-1-phényl-cyclohexane-1,4-diamine ;
- 5-(1-((4-(diméthylamino)-4-phénylcyclohexyl)(méthyl)amino)-2-(1H-indol-3-yl)éthyl)-1,3,4-oxadiazole-2(3H)-one ;
- 2-((4-(diméthylamino)-4-phénylcyclohexyl)(méthyl)amino)-3-(1H-indol-3-yl)-N,N-diméthylpropanamide ;
- N4-(2-(1H-indol-3-yl)-1-(3-méthyl-1,2,4-oxadiazol-5-yl)éthyl)-1-(3-fluorophényl)-N1,N1-diméthylcyclohexane-1,4-diamine ;
- N4-(2-(1H-indol-3-yl)-1-(5-méthyloxazol-2-yl)éthyl)-1-(3-fluorophényl)-N1,N1-diméthylcyclohexane-1,4-diamine ;
- amide de l'acide N-(2-(1H-indol-3-yl)-1-(5-méthyloxazol-2-yl)éthyl)-N-(4-(diméthylamino)-4-(3-fluoro-phényl)cyclohexyl)cinnamique ; et
- amide de l'acide N-(2-(1H-indol-3-yl)-1-(3-méthyl-1,2,4-oxadiazol-5-yl)éthyl)-N-(4-(diméthylamino)-4-(3-fluorophényl)cyclohexyl)cinnamique ;
- (R)-N4-(2-(1H-indol-3-yl)-1-(5-méthyloxazol-2-yl)éthyl)-N1,N1-diméthyl-1-phényl-cyclohexane-1,4-diamine,
- (S)-N4-(2-(1H-indol-3-yl)-1-(4-méthylthiazol-2-yl)éthyl)-N1,N1-diméthyl-1-phénylcyclohexane-1,4-diamine,
- (R)-N4-(2-(1H-indol-3-yl)-1-(4-méthylthiazol-2-yl)éthyl)-N1,N1-diméthyl-1-phénylcyclohexane-1,4-diamine,
- N-(2-(1H-indol-3-yl)-1-(1H-tétrazol-5-yl)éthyl)-N-(4-diméthylamino-4-phényl-cyclohexyl)-2,2,2-trifluoroacétamide,
- N4-(2-(1H-indol-3-yl)-1-(1H-tétrazol-5-yl)éthyl)-N1,N1-diméthyl-1-phénylcyclohexane-1,4-diamine,
ou leurs sels physiologiquement compatibles.

5. Médicament contenant au moins un composé selon l'une quelconque des revendications 1 à 4, sous la forme d'un stéréoisomère individuel ou son mélange, des composés libres et/ou leurs sels physiologiquement compatibles, ainsi qu'éventuellement des additifs et/ou adjuvants appropriés et/ou éventuellement d'autres agents actifs.

6. Utilisation d'un composé selon l'une quelconque des revendications 1 à 4, sous la forme d'un stéréoisomère individuel ou son mélange, du composé libre et/ou ses sels physiologiquement compatibles, pour la fabrication d'un médicment pour le traitement de la douleur.

7. Utilisation d'un composé selon l'une quelconque des revendications 1 à 4 sous la forme d'un stéréoisomère individuel ou son mélange, des composés libres et/ou leurs sels et/ou solvates physiologiquement compatibles pour la fabrication d'un médicament pour le traitement des états d'anxiété, du stress et des syndromes associés au stress, des dépressions, de l'épilepsie, de la maladie d'Alzheimer, de la démence sénile, des dysfonctionnements cognitifs généraux, des troubles de l'apprentissage et de la mémoire (en tant que nootropique), des phénomènes de sevrage, de l'abus et/ou de la dépendance à l'alcool et/ou à des drogues et/ou à des médicaments, des dysfonctionnements sexuels, des maladies cardiovasculaires, de l'hypotension, de l'hypertension, de l'acouphène, du prurit, de la migraine, de la déficience de l'ouïe, du manque de motilité de l'intestin, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthétique ou pour la coadministration lors du traitement avec un analgésique opioïde ou avec un anesthétique, pour la diurèse ou l'antinatriurèse, l'anxiolyse, pour la modulation de l'activité motrice, pour la modulation de la distribution des neurotransmetteurs et le traitement de maladies neurodégénératrices associées, pour le traitement de phénomènes de sevrage et/ou pour la réduction de l'effet de dépendance des opioïdes.
